(19) Europäisches Patentamt
European Patent Office
Office européen des brevets

(11) Veröffentlichungsnummer: **0 080 154**
**B1**

(12) # EUROPÄISCHE PATENTSCHRIFT

(45) Veröffentlichungstag der Patentschrift: **28.08.85**

(21) Anmeldenummer: **82110582.2**

(22) Anmeldetag: **16.11.82**

(51) Int. Cl.[4]: **C 07 D401/04**, C 07 D401/14, C 07 D491/04, A 61 K 31/435 // (C07D491/04, 317:00, 209:00)

(54) **N-Substituierte 2-Pyridylindole, Verfahren zu ihrer Herstellung, pharmazeutische Präparate enthaltend diese Verbindungen, sowie ihre therapeutische Verwendung.**

(30) Priorität: **19.11.81 US 323018**

(43) Veröffentlichungstag der Anmeldung: **01.06.83 Patentblatt 83/22**

(45) Bekanntmachung des Hinweises auf die Patenterteilung: **28.08.85 Patentblatt 85/35**

(84) Benannte Vertragsstaaten: **AT BE CH DE FR IT LI LU NL SE**

(56) Entgegenhaltungen:
**EP-A- 0 015 523**
**US-A- 3 468 894**
**DIE PHARMAZIE, Band 23, Nr. 10, 1968, Seiten 557-560 G. BUCHMANN et al.: "Zur Synthese von 2-Pyridylindolen"**

(73) Patentinhaber: **CIBA-GEIGY AG**
**Postfach**
**CH-4002 Basel (CH)**

(72) Erfinder: **Renfroe, Harris Burt, Dr.**
**12 Stonehedge Drive**
**West Nyack New York 10994 (US)**

(74) Vertreter: **Zumstein, Fritz, Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 080 154 B1

**Beschreibung**

Im US-Patent 3,468,894 sind 1-unsubstituierte 3-Methyl-2-(3- oder 4-pyridyl)-indole als diuretische Mittel offenbart. Ueber 2-(2-Pyridyl)-indol-3-(essig- und propion)-säuren wird z. B. in Pharm. Bull. *4*, 16 (1956) bzw. in Chemical Abstracts *64*, 19540d (1966) berichtet. Verschiedene gegebenenfalls substituierte 2-(3-Pyridyl)-indol-3-essigsäuren sind als chemische Zwischenprodukte in Bull. Soc. Chim. France *1966*, 771-2 und Bull. Soc. Chim. France *1969*, 4154-9 beschrieben worden. Die Hestellung des 1-Cyanäthyl-2-(2-pyridyl)-indols ist in Pharmazie *23* (10) 557-60 (1968) angegeben worden.

Ferner werden in der EP-A-15523 Indolderivate mit lipidsenkenden und antiatherosklerotischen Wirkungen offenbart, die in 5-, 6-, 7- oder 8-Stellung eine substituierte Alkoxygruppe aufweisen, in 1-Stellung gegebenenfalls durch Alkyl oder Alkenyl substituiert sind, in 2-Stellung einen Alkyl oder Alkenyl substituiert sind, in 2-Stellung einen Alkyl-, Phenylalkyl-, Phenyl- oder Pyridyl-Substituierten tragen und auch in den verbleibenden freien Positionen gegebenenfalls noch weiter substituiert sind.

Es wurde nun überraschend gefunden, dass N- (oder 1)-substituierte 2-Pyridylindole der Formel I eine neue Klasse von ausserordentlich wirksamen und hochspezifischen Thromboxan-Synthetase-Inhibitoren bilden.

Die vorher genannten Eigenschaften tragen dazu bei, dass die N-substituierten 2-pyridylindole dieser Erfindung, bei Verabreichung, allein oder in Kombination, an Säugern, z. B. für die Behandlung oder Vorbeugung von Krankheiten, welche auf die Hemmung von Thromboxan-Synthetase ansprechen, besonders nützlich sind. Diese Krankheiten umfassen kardiovaskuläre Störungen wie Thrombose, Atherosklerose, Koronarkrämpfe, Arrthythmien, cerebrale ischämische Anfälle, Migräne und andere vaskuläre Kopfschmerzen, Myokardinfarkt, Angina pectoris, Hypertension ; Atmungsstörungen wie Asthma und Apnoe ; und Entzündungskrankheiten. Man hatte auch festgestellt, dass die Hemmung der Thromboxan-Synthetase die Metastase bei gewissen Tumorklassen vermindert. Die Verbindungen der Erfindung können daher in der Behandlung gewisser Krebsarten nützlich sein.

Die vorliegende Erfindung betrifft daher 1-substituierte 2-Pyridylindole der Formel I

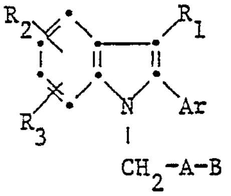

(I)

worin $R_1$ Wasserstoff oder Niederalkyl bedeutet, Ar für unsubstituiertes oder durch Niederalkyl, Carboxy, Niederalkoxycarbonyl oder Carbamoyl substituiertes Pyridyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy, Niederalkoxycarbonyl oder Niederalkyl-(thio, sulfinyl oder sulfonyl) bedeutet, oder $R_2$ und $R_3$ zusammen, an benachbarten Kohlenstoffatomen, für Niederalkylendioxy stehen ; A für Alkylen mit 1 bis 12 Kohlenstoffatomen, Alkenylen mit 2 bis 12 Kohlenstoffatomen, Alkynylen mit 2 bis 12 Kohlenstoffatomen, Niederalkyl-phenylen-niederalkylen, Niedearlkylen-phenylen, Phenylen-niederalkylen, Phenylen, eine direkte Bindung, Niederalkylen -(thio oder oxy)-niederalkylen, (Thio oder Oxy)-phenylen, Niederalkylen-(thio oder oxy)-phenylen, Phenylen-(thio oder oxy)-niederalkylen oder Phenylen-niederalkenylen steht, B Carboxy, Niederalkoxycarbonyl, Carbamoyl, Mono- oder Di-niederalkyl-carbamoyl, Hydroxymethyl, Hydroxycarbamoyl, 5-Tetrazolyl oder Formyl bedeutet ; ihre N-Oxide ; und ihre Salze, wobei die mit « nieder » bezeichneten Reste höchstens 7 Kohlenstoffatome aufweisen, insbesondere ihre therapeutisch verwendbaren Salze, sowie Verfahren zu ihrer Herstellung, pharmazeutische Präparate enthaltend diese Verbindungen, sowie ihre therapeutische Verwendung.

Bevorzugt sind Verbindungen der Formel I, worin $R_1$ Wasserstoff oder Niederalkyl bedeutet, Ar für unsubstituiertes oder durch Niederalkyl, Carboxy, Niederalkoxycarbonyl oder Carbamoyl substituiertes Pyridyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy oder Niederalkoxycarbonyl bedeutet, A für Alkylen mit 1 bis 12 Kohlenstoffatomen, Alkynylen mit 2 bis 12 Kohlenstoffatomen, Alkenylen mit 2 bis 12 Kohlenstoffatomen, Niederalkylen-phenylen-niederalkylen, Niederalkylen-phenylen, Phenylen-niederalkylen, Phenylen oder eine direkte Bindung steht, B Carboxy, Niederalkoxycarbonyl, Carbamoyl, Mono- oder Di-niederalkyl-carbamoyl oder Hydroxymethyl bedeutet ; ihre N-Oxide ; und ihre Salze, wobei die mit « nieder » bezeichneten Reste höchstens 7 Kohlenstoffatome aufweisen.

Bevorzugt sind Verbindungen der Formel I, worin $R_1$ Wasserstoff oder Niederalkyl bedeutet, Ar für gegebenenfalls durch Niederalkyl substituiertes 2-, 3- oder 4-Pyridyl steht, $R_2$ Wasserstoff, Niederalkyl, Halogen, Trifluormethyl, Hydroxy, Niederalkoxy, Niederalkylthio, Carboxy-niederalkyl oder Niederalkoxycarbonyl-niederalkyl bedeutet, $R_3$ für Wasserstoff steht, oder $R_2$ und $R_3$ zusammen, an benachbarten Kohlenstoffatomen Niederalkylendioxy bedeuten ; A Alkylen mit 1 bis 12 Kohlenstoffatomen, Phenylen, Nieder(alkylen-phenylen, alkylen-thio-phenylen oder alkylen-oxy-phenylen) mit 7 bis 10 Kohlenstoffatomen oder eine direkte Bindung bedeutet, B für Carboxy, Niederalkoxycarbonyl, Carbamoyl, Hydroxycarbamoyl, 5-Tetrazolyl oder Hydroxymethyl steht ; ihre N-Oxide ; und Salze, insbesondere ihre therapeutisch verwendbaren Salze.

Weitere bevorzugte Verbindungen sind die vorher genanten der Formel I, in welchen $R_2$ an die 5-Stellung des Indolkernes gebunden ist.

Besonders bevorzugt sind die vorher genannten Verbindungen der Formel I, worin B Carboxy, Niederalkoxycarbonyl, Carbamoyl, 5-Tetrazolyl oder Hydroxycarbamoyl bedeutet.

Sehr bevorzugt sind Verbindungen der Formel I, worin A Alkylen mit 3-10 Kohlenstoffatomen, Phenylen, Niederalkylen-thio-phenylen oder Niederalkylen-oxy-phenylen, jedes mit 7 bis 10 Kohlenstoffatomen bedeutet.

Ueberdies sind die Verbindungen der Formel I, worin A Alkylen mit 1 bis 12 Kohlenstoffatomen oder Phenylen bedeutet, wichtig.

Besonders hervorzuheben sind die Verbindungen der Formel II

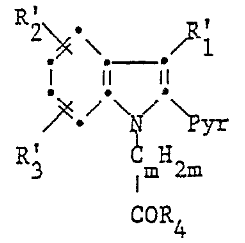

(II)

worin $R'_1$ Wasserstoff oder Niederalkyl bedeutet, $R'_2$ und $R'_3$ unabhängig voneinander Wasserstoff, Niederalkyl, Halogen, Trifluormethyl, Hydroxy, Niederalkylthio oder Niederalkoxy steht, oder $R'_2$ und $R'_3$ zusammen, an benachbarten Kohlenstoffatomen, Methylendioxy bedeuten, Pyr 2-, 3- oder 4-Pyridyl bedeutet, m für eine ganze Zahl von 1 bis 13 steht, $R_4$ Hydroxy, Niederalkoxy oder Amino bedeutet ; und ihre Salze, insbesondere ihre therapeutisch verwendbaren Salze, wobei die mit « nieder » bezeichneten Reste höchstens 7 Kohlenstoffatome aufweisen.

Bevorzugt sind Verbindungen der Formel II, worin $R'_3$ Wasserstoff bedeutet.

Besonders wichtig sind auch Verbindungen der Formel III

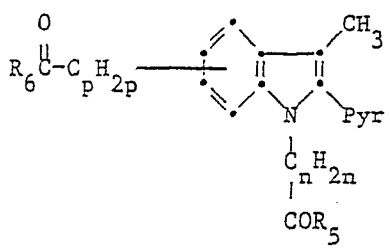

(III)

worin n eine ganze Zahl von 3 b is 10 bedeutet, p für eine ganze Zahl von 0 bis 4 steht, Pyr 2-, 3- oder 4-Pyridyl bedeutet, $R_5$ und $R_6$ unabhängig voneinander für Hydroxy oder Niederalkoxy stehen ; und Salze, insbesondere ihre therapeutisch verwendbaren Salze, wobei die mit « nieder » bezeichneten Reste höchstens 7 Kohlenstoffatome aufweisen.

Bevorzugt sind Verbindungen der Formel III, worin n 4 bis 8 ist, p 1 bis 4 bedeutet, Pyr für 3- oder 4-Pyridyl steht, und $R_5$ und $R_6$ Hydroxy bedeuten.

Wertvoll sind weitere Verbindungen der Formel IV

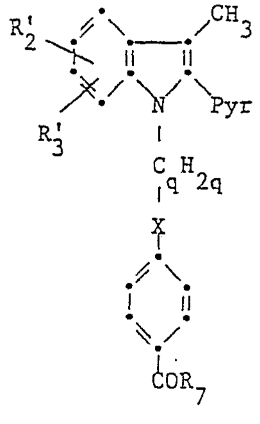

(IV)

worin $R'_2$ und $R'_3$ unabhängig voneinander Wasserstoff, Niederalkyl, Halogen, Niederalkoxy, Niederalkylthio oder Hydroxy bedeuten, oder $R'_2$ und $R'_3$ zusammen, an benachbarten Kohlenstoffatomen für Methylendioxy stehen ; X Sauerstoff, Schwefel oder eine direkte Bindung bedeutet, q für eine ganze Zahl von 1 bis 4 steht, $R_7$ Hydroxy oder Niederalkoxy bedeutet ; Pyr für 2-, 3- oder 4-Pyridyl steht, und Salze, insbesondere ihre therapeutisch verwendbaren Salze, wobei die mit « nieder » bezeichneten Reste höchstens 7 Kohlenstoffatome aufweisen.

Bevorzugt sind Verbindungen der Formel IV, worin X eine direkte Bindung bedeutet. Auch Verbindungen der Formel IV, worin q eine Zahl von 2 bis 4 steht und X Sauerstoff oder Schwefel bedeutet, sind hervorzuheben.

Die allgemeinen Definitionen, welche hier verwendet werden, haben innerhalb des Umfangs der vorliegenden Erfindung die folgenden Bedeutungen.

Ein Alkylenrest bedeutet Alkylen mit 1 bis 12 Kohlenstoffatomen, der geradkettig oder verzweigt sein kan, und vorzugsweise für Propylen, Butylen, Pentylen, Hexylen oder Heptylen steht, wobei die genannten Reste unsubstituiert oder durch eine oder mehrere Niederalkylgruppen substituiert sind, mit der Massgabe, dass die Summe der Kohlenstoffatome nicht mehr als 12 ist.

Der Ausdruck Alkenylen bedeutet einen Alkylenrest mit 2 bis 12 Kohlenstoffatomen, welche Rest geradkettig oder verzweigt sein kann, und vorzugsweise für Propenylen, 1-, oder 2-Butenylen, 1- oder 2-Pentenylen, 1-, 2- oder 3-Hexenylen, 1-, 2-, 3- oder 4-Heptenylen steht. Die genannten Reste sind unsubstituiert oder durch eine oder mehrere Niederalkylgruppen substituiert, mit der Massgabe, dass die Summe der Kohlenstoffatome 12 nicht übersteigt.

Der Begriff Alkynylen bezeichnet einen Alkynylenrest mit 2 bis 12 Kohlenstoffatomen, der geradkettig oder Verzweigt ist, und vorzugsweise für Propynylen, 1- oder 2-Butynylen, 1- oder 2-Pentynylen, 1-, 2- oder Hexynylen, 1- , 2-, 3- oder 4-Heptynylen steht. Diese Reste sind unsubstituiert oder durch eine oder mehrere Niederalkylgruppen substituiert, wobei die Summe der Kohlenstoffatome 12 nicht übersteigt.

Der Ausdruck Phenylen bedeutet 1,2-, 1,3- und vorzugsweise 1,4-Phenylen.

Der Ausdruck Pyridyl bedeutet 2-, 3- oder 4-Pyridyl, insbesondere 3-Pyridyl.

Der Ausdruck « nieder » definiert in den oben oder nachfolgend genannten organischen Gruppen, Reste oder Verbindungen solche mit höchstens 7, vorzugsweise 4, insbesondere 1, 2 oder 3 Kohlenstoffatomen.

Eine Niederalkylen-phenylengruppe, eine Phenylen-niederalkylengruppe, eine Niederalkylen-phenylen-niederalkylengruppe, eine Niederalkylen-(thio oder oxy)-phenylengruppe, eine Phenylen-(thio oder oxy)-niederalkylengruppe, oder eine Phenylen-niederalkenylengruppe enthält in jedem Alkylen- oder Alkenylenrest vorzugsweise 1 bis 4, insbesondere 1 oder 2 Kohlenstoffatome. Die Niederalkylen- oder Niederalkenylenreste können geradkettig oder verzweigt sein.

Eine Niederalkylen-(thio oder oxy)-niederalkylengruppe kann geradkettig oder verzweigt sein und kann insgesamt 2 bis 12, vorzugsweise 2 bis 8 Kohlenstoffatome haben.

Eine Niederalkylgruppe enthält vorzugsweise 1-4 Kohlenstoffatome und steht z. B. für Aethyl, Propyl oder Butyl, insbesondere für Methyl.

Eine Niederalkylendioxygruppe bedeutet vorzugsweise Aethylendioxy oder Methylendioxy.

Eine Niederalkoxygruppe enthält vorzugsweise 1 bis 4 Kohlenstoffatome und ist z. B. Aethoxy, Propoxy oder insbesondere Methoxy. Eine Niederalkyl-(thio, sulfinyl oder sulfonyl)-gruppe ist vorzugsweise Methylthio, Methylsulfinyl bzw. Methylsulfonyl.

Eine Niederalkoxycarbonylgruppe enthält vorzugsweise 1 bis 4 Kohlenstoffatome im Alkoxyteil und bedeutet z. B. Methoxycarbonyl, Propoxycarbonyl oder Isopropoxycarbonyl, insbesondere Aethoxycarbonyl. Eine Mono-(niederalkyl)-carbamoylgruppe hat vorzugsweise 1 bis 4 Kohlenstoffatome im Alkylteil und ist z. B. N-Methylcarbamoyl, N-Propylcarbamoyl oder insbesondere N-Aethylcarbamoyl. Eine Di-(niederalkyl)-carbamoylgruppe enthält vorzugsweise 1 bis 4 Kohlenstoffatome in jeder Niederalkylgruppe und steht z. B. für N,N-Dimethylcarbamoyl, N-Methyl-N-äthylcarbamoyl und insbesondere für N,N-Diäthylcarbamoyl.

Halogen ist vorzugsweise Fluor oder Chlor, es kann aber auch Brom oder Jod sein.

Salze sind vorzugsweise therapeutisch verwendbare Salze, z. B. Metall- oder Ammoniumsalze der genannten Verbindungen der Formel I, welche freies Carboxy haben, insbesondere Alkalimetall- oder Erdalkalimetallsalze, z. B. Natrium-, Kalium-, Magnesium- oder Calciumsalze ; in erster Linie leicht kristallisierende Ammoniumsalze. Diese werden abgeleitet von Ammoniak oder organischen Aminen, z. B. Mono-, Di- oder Tri-nieder-(alkyl, cycloalkyl oder hydroxyalkyl)-aminen, Niederalkylendiaminen oder (Hydroxy-niederalkyl oder Aryl-niederalkyl)-niederalkylammonium Basen, z. B. Methylamin, Diäthylamin, Triäthylamin, Dicyclohexylamin, Triäthanolamin, Aethylendiamin, Tris-(hydroxymethyl)-aminomethan oder Benzyl-trimethylammoniumhydroxid. Die genannten Verbindungen der Formel I bilden Säureadditionssalze. Diese werden vorzugsweise mit solchen Säuren, welche therapeutisch verwendbare Säureadditionssalze ergeben, hergestellt. Säuren, die therapeutisch verwendbare Säureadditionssalze ergeben, sind z. B. starke Mineralsäuren, wie Halogenwasserstoffsäuren, z. B. Chlorwasserstoff- oder Bromwasserstoffsäure, Schwefel-, Phosphor-, Salpeter- oder Perchlorsäure ; oder organische Säuren, wie aliphatische oder aromatische Carbon- oder Sulfonsäure, z. B. Ameisen-, Essig-, Propion-, Bernstein-, Glykol-, Milch-, Aepfel-, Wein-, Glucon-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminoslicyl-, Pamoe-, Nikotin-, Methansulfon-

, Aethansulfon-, Hydroxyäthansulfon-, Benzolsulfon-, p-Toluolsulfon-, Naphthalinsulfon-, Sulfanil oder Cyclohexyl-sulfaminsäure ; oder die Ascorbinsäure.

Die Verbindungen der Erfindung zeigen wertvolle pharmakologische Eingenschaften, z. B. kardiovaskuläre Effekte, indem sie die Thromboxan-Ausschüttung in Säugern selektiv hemmen. Diese Hemmung kommt durch selektive Herabsetzung des Thromboxan-Synthetase-Spiegels zustande. Die Verbindugnen sind daher nützlich in der Behandlung von Krankheiten, welche auf Thromboxan-Synthetase-Hemmung in Säugern, ansprechen. Solche Krankheiten sind in erster Linie kardiovaskuläre Störungen wie Thrombose, Arteriosklerose, Koronarkrämpfe, cerebrale ischämische Anfälle, Migräne und andere vaskuläre Kopfschmerzen, Myokardinfarkt, Angina pectoris und Hypertension.

Diese Wirkungen können durch in vitro Versuche oder in vivo-Tierversuche, vorzugsweise an Säugetieren, z. B. Meerschweinchen, Mäusen, Ratten, Katzen, Hunden oder Affen nachgewiesen werden. Die genannten Verbindungen können ihnen enteral oder parenteral, vorzugsweise oral oder subkutan, intravenös oder intraperitoneal, z. B. durch Gelatine-Kapseln oder in Form von Stärke enthaltenden Suspensionen oder wässerigen Lösungen, verabreicht werden. Die verwendete Dosis kann in einem Bereich von ungefähr zwischen 0,01 und 100 mg/kg/Tag, vorzugsweise ungefähr 0,05 und 50 mg/kg/Tag, insbesondere ungefähr 0,1 und 25 mg/kg/Tag liegen.

Die in vitro Hemmung des Thromboxan-Synthetase-Enzyms kann analog zu der Methode von Sun, Biochem. Biophys, Res. Comm. *74,* 1432 (1977) nachgewiesen werden. Das Testverfahren wird wie folgt durchgeführt :

$^{14}C$-Arachidonsäure wird mit einem Enzymgemisch-Präparat, bestehend aus solubilisierter und partiell gereinigter Prostaglandin-cyclooxygenase von Schaf-Samenblasen und aus einem rohen Mikrosomen-Präparat von Thromboxan-Synthetase von lysierten menschliechen Bluplättchen, inkubiert. Die Testverbindung (gelöst in einem Puffer, oder falls nötig, in wenig Aethanol) wird zu dem Inkubationsmedium gegeben. Am Ende der Inkubationsperiode (30 Minuten) wird das Prostaglandin $E_2$ ($PGE_2$) durch Hinzufügen von Natriumborhydrid zu einem Gemisch von Prostaglandin $F_2\alpha$ und $F_2\beta$ [$PGF_2$ ($\alpha + \beta$)] reduziert. Die radioaktiven Produkte und das überschüssige Substrat werden mit Essigsäureäthylester extrahiert und der Extrakt wird zur Trockene eingedampft. Der Rückstand wird in Aceton gelöst, auf Dünnschichtplatten tropfenweise aufgetragen und mit einem Lösungsmittelsystem von Toluol : Aceton : Eisessig [100 : 100 : 3 (Volumen)] chromatographiert. Die radioaktiven Zonen werden lokalisiert. Die Zonen von Thromboxan $B_2$ ($T \times B_2$) und $PGF_2\alpha + \beta$ werden in Szintillationsröhrchen für Flüssigkeiten übertragen und gezählt. Der Quotient der Zahlenwerte von $T \times B_2/PGF_2\alpha + \beta$ wird für jede Konzentration der Testverbindung berechnet und die $IC_{50}$-Werte werden graphisch ermittelt. Dieser Wert ist die Konzentration der Testverbindung, bei welcher der Quotient von $T \times B_2/PGF_2\alpha + \beta$ auf 50 % des Kontrollwertes reduziert wird.

Die in vitro Wirkung auf Prostaglandin-cyclo-oxygenase wird gemäss einer Modifikation der Methode von Takeguchi et al, welche in Biochemistry *10,* 2372 (1971) beschrieben ist, gemessen. Das Testverfahren ist wie folgt :

Lyophilisierte Samenblasen-Mikrosomen von Schafen werden als Prostaglandin synthetisierendes Enzympräparat verwendet. Es wird die Umwandlung von $^{14}C$-Arachidonsäure in $PGE_2$ gemessen. Die Testverbindungen (gelöst in einem Puffer, oder wenn nötig in wenig Aethanol) werden zu dem Inkubationsgemisch gegeben. Die Prostaglandine werden extrahiert und durch Dünnschichtchromatographie aufgetrennt. Die Platten werden überprüft, die dem $PGE_2$ entsprechenden radioaktiven Zonen werden in Szintillationsröhrchen für Flüssigkeiten übertragen und ihre Radioaktivität gezählt. Die $IC_{50}$-Werte der Hemmung werden graphisch ermittelt. Dieser Wert bedeutet die Konzentration der Testverbindung, welche die Menge des synthetisierten $PGE_2$ um 50 % reduziert.

Die in vitro Wirkung auf Prostacyclin-($PGI_2$)-Synthetase wird analog zu der Methode von Sun et al., Prostaglandins *14*, 1055 (1977) gemessen. Das Testverfahren ist wie folgt :

$^{14}C$-Arachidonsäure wird mit einem Enzymgemisch, bestehend aus solubilisierter und partiell gereinigter Prostaglandin-cyclo-oxygenase von Schaf-Samenblasen und aus rohem $PGI_2$-Synthetase in der Form einer Mikrosomen-Fraktion von Aorten von Rindern, inkubiert.

Die Testverbindung (gelöst in einem Puffer, oder wenn nötig, in wenig Aethanol) wird in das Inkubationsmedium gegeben. Das Reaktionsgemisch wird in 100 mMolarem Tris HCl (pH 7,5) 30 Minuten bei 37° inkubiert, auf den pH-Wert 3 angesäuert und mit Essigsäureäthylester extrahiert. Der Extrakt wird zur Trockene eingedampft, der Rückstand in Aceton gelöst auf Dünnschicht-Platten aufgetragen und mit einem von Sun et Al. beschriebenen Lösungsmittelsystem chromatographiert. Die radioaktiven Zonen werden mit einem Detektor lokalisiert. Die dem 6-Keto-$PGF_1\alpha$ (ein stabiles Endprodukt der Prostacyclin Biotransformation) und $PGE_2$ entsprechenden Zonen werden in Szintillationsröhrchen für Flüssigkeiten übertragen und gezählt. Der Quotient der Zahlenwerte von 6-Keto-$PGF_1\alpha/PGE_2$ wird für jede Konzentration der verwendeten Testverbindung berechnet. Die $IC_{50}$-Werte der Hemmung werden graphisch ermittelt. Dieser Wert ist die Konzentration der Testverbindung, bei welcher der Quotient von 6-Keto-$PGF_1\alpha/PGE_2$ auf 50 % des Kontrollwertes reduziert wird.

Die Hemmung der Synthese und die Vermidnerung des Throboxan-Plasmaspiegels wird in vivo durch Verabreichung der Testverbindung an Ratten (analog zu den von Tai et al. in Anual. Biochem. *87,* 343, 1978 und von Salmon in Prostaglandins *15,* 383, 1978 beschriebenen Verfahren) wie folgt bestimmt :

Ratten werden mit der Testsubstanz oder dem Trägermaterial behandelt und 2 Stunden später wird

ihnen Ionophor A 23187 (0,5 mg/kg) intravenös injiziert. Zwei Minuten nach der Ionophor-Verabreichung wird für die Analyse den Tieren Blut entnommen. In einer bestimmten Einzelmenge jeder Plasmaprobe wird das Thromboxan $B_2$, und aus weiteren Einzelmengen werden das 6-Keto-$PGF_1\alpha$, die stabilen Metaboliten des Thromboxans $A_2$ bzw. das Prostacyclin ($PGI_2$) durch Radioimmunoessay bestimmt.

Die Verbindungen der Formel I sind sehr wirksame und selektive Thromboxan-Synthetase Inhibitoren. Bei wirksamen Dosis-Spiegeln und darüber, wird weder das vorteilhafte Prostacyclin-Synthetasenoch das Prostaglandin-cyclooxygenase-Enzymsystem wesentlich gehemmt. Ueberraschenderweise werden die Prostacyclin-Spiegel signifikant erhöht.

Der $IC_{50}$-Wert für eine Verbindung der Erfindung, z. B. für das 1-(7-Carboxyheptyl)-3-methyl-2-(3-pyridyl)-indol beträgt $1{,}2 \times 10^{-8}$ Mol für die Thromboxan-Synthetase-Hemmung, während für die Hemmung der Prostacyclin-Synthetase und der Prostacyclin-cyclo-oxygenase der $IC_{50}$-Wert jeweils mehrere Potenzen höher, nämlich ungefähr $1 \times 10^{-4}$ Mol ist.

Weiter ist der $IC_{50}$-Wert für die Thromboxan-Synthetase-Hemmung $2 \times 10^{-8}$ Mol für 1-(5-Carboxypentyl)-5-(2-carboxyäthyl)-3-methyl-2-(3-pyridyl)-indol und $5 \times 10^{-8}$ Mol für 1-(4-Carboxybenzyl)-3-methyl-2-(3-pyridyl)-indol, $1 \times 10^{-9}$ Mol für 1-(5-Carboxypentyl)-5-chlor-3-methyl-2-(3-pyridyl)-indol und $1 \times 10^{-8}$ Mol für 1-(5-Carbamoylpentyl)-5-chlor-3-methyl-2-(3-pyridyl)-indol, $2{,}6 \times 10^{-8}$ Mol für 1-[2-(4-Carboxyphenoxy)-äthyl]-3-methyl-2-(3-pyridyl)-indol und $5{,}8 \times 10^{-8}$ Mol für 1-[2-(4-Carboxyphenylthio)-äthyl]-3-methyl-2-(3-pyridyl)-indol-hydrochlorid.

Das 1-(7-Carboxyheptyl)-3-methyl-2-(3-pyridyl)-indol und 1-(5-Carboxypentyl)-5-chlor-3-methyl-2-(3-pyridyl)-indol als repräsentative Verbindungen der Erfindung, vermindern den Plasmaspiegel von Thromboxan $B_2$ in der Ratte, bei einer so niedrigen oralen Dosis wie 0,10 mg/kg, um mehr als 50 %. Bei dieser oder höheren oralen Dosen wird eine überraschende Zunahme des Prostacyclin-Plasmaspiegels festgestellt.

Aufgrund der vorhergenannten vorteilhaften Eigenschaften sind die Verbindungen der Erfindung für Säuger, einschliesslich Menschen, als spezifische therapeutische Mittel sehr wertvoll.

Die Hemmung von der verschiedenartig hervorgerufenen Aggregation von Bluttplättchen und Thrombozytopenie durch die Verbindungen der Erfindung, z. B. durch 1-(7-Carboxyheptyl)-3-methyl-2-(3-pyridyl)-indol, zeigt die Nützlichkeit bei der Thromboembolie. Experimentell wird die Verlängerung der Blutungszeit an der Ratte als Zeichen für die günstige antithrombotische Wirkung angesehen. So weist z. B. das 1-(7-Carboxyheptyl)-3-methyl-2-(3-pyridyl)-indol diese Wirkung bei oraler Verabreichung an Ratten in einer Dosis von ungefähr 30 mg/kg auf.

Auf die günstigen Wirkungen bei Atmunsgsströrungen ist die Tatsache hinweisend, dass die Verbindungen der vorliegenden Erfindung Schutz gegen den plötzlichen Tod, der aufgrund der durch die Arachindonsäure hervorgerufene Lungenobstruktion eintrifft, gewähren. So schützt z. B. das 1-(7-Carboxyheptyl)-3-methyl-2-(3-pyridyl)-indol die Maus gegen den plötizlichen Tod bei einer oralen Verabreichung einer Dosis von 100 mg/kg.

Ausser den oben genannten therapeutisch verwendbaren Salzen bilden jene pharmakologischen Vorstufen der erfindungsgemässen Carbonsäuren, z. B. ihre Ester und Amide, welche durch Solvolyse oder unter physiologischen Bedingungen in die genannten Carbonsäuren umgewandelt werden können, einen weiteren Gegenstand dieser Erfindung.

Solche Ester sind vorzugsweise z. B. die unsubstituierten oder geeignet substituierten Niederalkylester, wie Pivaloyloxymethyl-, 2-Diäthylaminoäthyl-, Bornyloxycarbonylmethyl, $\alpha$-Carboxyäthyl- oder in geeigneter Weise veresterten $\alpha$-Carboxyäthylester, welche in an sich bekannter Weise hergestellt werden können.

Die genannten Amide sind vorzugsweise z. B. einfache primäre oder sekundäre Amide und solche, welche von Aminsäuren oder ihren Derivaten, z. B. von Alanin oder Phenylalanin, abgeleitet sind.

Die Verbindungen der vorliegenden Erfindung werden nach an sich bekannten Methoden, vorzugsweise dadurch hergestellt, dass man

1) eine Verbindung der Formel V

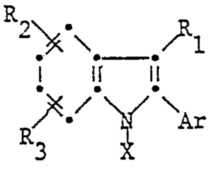

(V)

worin X Wasserstoff, Alkalimetall oder Tri-niederalkylsilyl bedeutet, $R_1$, $R_2$, $R_3$ und Ar die angegebene Bedeutung haben, mit einem reaktionsfähigen funktionellen Derivat einer Verbindung der Formel VI

$$HO{-}CH_2{-}A{-}B \qquad (VI)$$

worin A und B die oben angegebene Bedeutung haben, kondensiert, oder

2) eine Verbindung der Formel VII

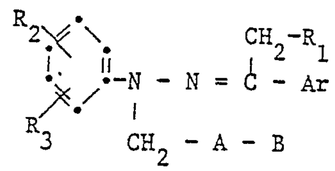

(VII)

worin Ar, $R_1$, $R_2$, $R_3$, A und B die oben angegebene Bedeutung haben, ringschliesst, oder

3) eine Verbindung der Formel VIII

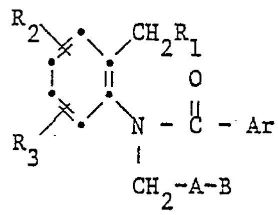

(VIII)

worin Ar, $R_1$, $R_2$, $R_3$, A und B die angegebene Bedeutung haben, cyclisiert, oder

4) in einer Verbindung der Formel Ia

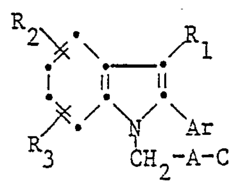

(Ia)

worin A, Ar, $R_1$, $R_2$ und $R_3$ die oben angegebene Bedeutung haben, und C eine von einer Gruppe B verschiedene, in die Gruppe B überführbare Gruppe bedeutet, die Gruppe C in B umwandelt, gegebenenfalls unter Verlängerung der Kette A innerhalb ihrer Definition, oder

5) eine Verbindung der Formel IX

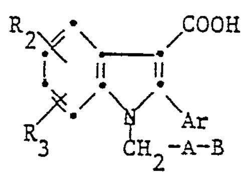

(IX)

worin A, B, Ar, $R_2$ und $R_3$ die oben angegebene Bedeutung haben, decarboxyliert, und, wenn erwünscht oder notwendig, eine störende reaktionsfähige Gruppe in allen diesen Verfahren vorübergehend schützt, und, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

Die Kondensation gemäss dem Verfahren 1) wird vorzugsweise unter basischen Bedingungen, z. B. mit einem basischen Alkalimetallsalz oder einem quaternären Ammoniumsalz, z. B. Tetrabutyl-ammoniumhydroxid, durchgeführt. So werden z. B. insbesondere Verbindungen der Formel V, worin X Wasserstoff bedeutet, vorzugsweise in situ, mit einem reaktionsfähigen metallisierenden Mittel in reaktionsfähige organometall Zwischenprodukte umgewandelt. Man arbeitet vorzugsweise mit ungefähr einem Moläquivalent z. B. einer starken Alaklimetallbase, wie Lithium-diisopropylamid, Natriumhydrid oder Kalium-tert.-butoxid, in einem inerten Lösungsmittel, z. B. Dimethylformamid oder Tetrahydrofuran, in einem Temperaturbereich zwischen − 50° bis + 75 °C, vorzugsweise − 25° und + 50 °C.

Die Kondensation der erhaltenen reaktionsfähigen Organometallverbindung der Formel V mit einem reaktionsfähigen funktionellen Derivat einer Verbindung der Formel VI wird in einem Temperaturbereich zwischen ungefähr − 25° bis + 50 °C, vorzugsweise bei einer Temperatur zwischen 0° und 30 °C

vorgenommen. Wenn B Carboxy, Carbamoyl, Hydroxycarbamoyl, oder Mono-niederalkyl-carbamoyl bedeutet, verwendet man zusätzlich, z. B. ein Moläquivalent des Metallisierungsmittels.

Beispielsweise die Ausgangsstoffe der Formel V, worin X Wasserstoff bedeutet, sind entweder bekannt (z. B. USP 3,468,894 ; J. Chem. Soc. *1955*, 2865 ; Bull. Soc. Chim. France *1969*, 4154) oder sie werden analog, ausgehend von entsprechenden, gegebenenfalls substituierten Phenylhydrazinen und Ketonen der Formel $ArCOCH_2R_1$ in Gegenwart von Kondensationsmitteln, z. B. äthanolischem Chlorwasserstoff oder Polyphosphorsäure gemäss der wohlbekannten Indolsynthese nach Fischer hergestellt.

Die Ausgangsstoffe der Formel VI oder die weiter unten beschriebenen der Formel VIa sind bekannt oder wenn neu, können sie nach an sich bekannten, z. B. im US-Patent 4,256,757 oder in der britischen Patentanmeldung 2,016,452A illustrierten Methoden oder wie hier in den Beispielen beschrieben, hergestellt werden.

Die Verbindungen der Formel I werden vorzugsweise gemäss dem Verfahren 1) dadurch hergestellt ; dass man eine Verbindung der Formel V

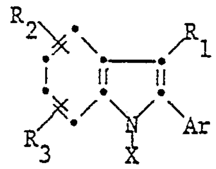

(V)

worin X Wasserstoff bedeutet, $R_1$ Wasserstoff oder Niederalkyl ist, Ar für gegebenenfalls durch Niederalkyl, Carboxy, Niederalkoxycarbonyl oder Carbamoyl substituiertes Pyridyl steht, jedes der Symbole $R_2$ und $R_3$ Wasserstoff, Niederalkyl, Halogen, Trifluormethyl, Hydroxy, Niederalkoxy, Carboxy-niederalkyl, Niederalkoxycarbonyl-niederalkyl, Carboxy oder Niederalkoxycarbonyl bedeutet, mit einem reaktionsfähigen funktionellen Derivat einer Verbindung der Formel VIa

$$HO—CH_2—A—B' \qquad (VIa)$$

worin A Alkylen mit 1 bis 12 Kohlenstoffatomen, Alkenylen mit 2 bis 12 Kohlenstoffatomen, Alkynylen mit 2 bis 12 Kohlenstoffatomen, Niederalkylen-phenylenniederalkylen, Niederalkylen-phenylen, Phenylenniederalkylen, Phenylen oder eine direkte Bindung bedeutet, und B' für Carboxy, Niederalkoxycarbonyl, Carbamoyl, Mono- oder Diniederalkyl-carbamoyl, Hydroxymethyl, veräthertes Hydroxymethyl, Halogenmethyl, Trialkoxymethyl oder Cyan steht, vorzugsweise unter basischen Bedingungen kondensiert, und in einer Verbindung der Formel Ib

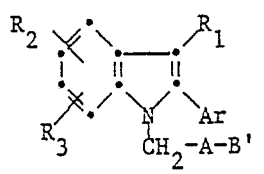

(Ib)

worin sich B' von B unterscheidet, den Rest B', gegebenenfalls unter Verlängerung der Kette A innerhalb ihrer Definition, in die Gruppe B unwandelt, und, wenn erwünscht, eine erhaltene Verbindung der Formel I in eine andere Verbindung überführt.

Die Umwandlung des Vorprodukts, in welchem sich B' von B unterscheidet und die Ueberführung eines erhaltenen Produktes in eine andere Verbindung der Erfindung werden nach an sich bekannten chemischen Methoden vorgenommen.

Der Ringschluss des Ausgangsstoffes der Formel VII gemäss dem Verfahren 2) wird gemäss der wohlbekannten Indolsynthese nach Fischer [wie in « Heterocyclic Compounds, Indoles Part I » herausgegeben von W.J. Houlihan pp. 232-317, beschrieben], thermisch oder vorzugsweise in Gegenwart eines sauren Kondensationsmittels durchgeführt. Man arbeitet in Gegenwart von vorzugsweise Halogenwasserstoffen, z. B. äthanolischem Chlorwasserstoff oder Polyphosporsäure, gegebenenfalls in einem inerten Lösungsmittel, bei Temperaturen zwischen ungefähr 50 und 100 °C.

Die Hydrazon-Ausgangsstoffe der Formel VII werden entweder isoliert oder vorzugsweise in situ durch Kondensation eines Ketons der Formel $ArCOCH_2R_1$, worin Ar und $R_1$ die oben angegebene Bedeutung haben, mit einem substituierten Hydrazin der Formel X

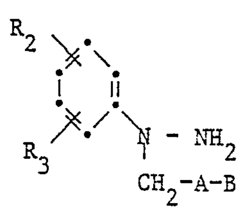

(X)

worin A, B, $R_2$ und $R_3$ die oben angegebenen Bedeutungen haben, vorzugsweise in Gegenwart eines saueren Katalysators, hergestellt.

Die Hydrazin-Ausgangsstoffe der Formel X werden ihrerseits vorzugsweise z. B. durch Nitrosierung von entsprechend substituierten Anilinen der Formel XI

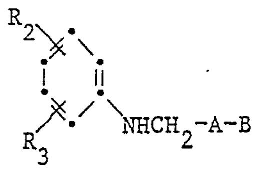

(XI)

worin die Symbole A, B, $R_2$ und $R_3$ die vorher angegebenen Bedeutungen haben, und nachfolgende Reduktion der N-Nitroso-Derivate, z. B. mit Zink in Essigsäure oder nach anderen an sich bekannten Methoden, hergestellt.

Wenn die genannten Zwischenprodukte störende reaktionsfähige Gruppen, z. B. Hydroxy- oder Aminogruppen enthalten, können solche vorzugsweise vorübergehend, an jeder Stufe, durch leicht abspaltbare Schutzgruppen, z. B. in der Form von Estern bzw. Amiden, gemäss wohlbekannten Methoden geschützt werden.

Die Cyclisierung gemäss dem Verfahren 3) wird unter den Bedingungen der Indolsynthese nach Madelung, wie in « Heterocyclic Compounds, Indoles Part I », Herausgeber W.J. Houlihan, pp. 385-396, beschrieben, vorgenommen. Die intramolekulare Cyclisierung wird vorzugsweise in Gegenwart einer starken Base, z. B. Natrium-äthoxid, Natriumamid, oder Kalium-tert.-butoxid, vorteilhafterveise bei erhöhter Temperatur, z. B. ungefähr 300° oder in einem inerten hochsiedenden Lösungsmittel, z. B. Tetrahydronaphthalin, durchgeführt.

Die Ausgangsstoffe der Formel VIII werden durch Acylierung von substituierten Anilinen der oben genannten Formel XI mit einer Verbindung der Formel ArCOOH oder einem ihrer reaktionsfähigen funktionellen Derivate, erhalten.

Die Decarboxylierung gemäss dem Verfahren 5) wird nach an sich bekannten Methoden, z. B. durch Erhitzen in einem hochsiedenden Lösungsmittel oder in Gegenwart eienr starken Säure, z. B. einer Mineralsäure wie Chlorwasserstoffsäure, durchgeführt.

Die als Ausgangstoffe verwendeten 3-Carboxy-substituierten Indole werden nach an sich bekannten Methoden hergestellt. So können z. B. Verbindungen der Formel IX, in welchen der Substituent in 3-Stellung Carboxy ist und einer der Reste $R_2$ und $R_3$ für 5-Hydroxy steht, gemäss der Nenitzescu-Synthese, wie in « Heterocyclic Compounds, Indoles Part I », p. 413 beschrieben, hergestellt werden. Man kondensiert z. B. p-Benzochinon mit einem β-Pyridyl-β-($CH_2$-A-B-substituiertes amino)-acrylsäure-niederalkylester, z. B. β-(3-Pyridyl)-β-(5-äthoxycarbonyl-pentylamino)-acrylsäure-niederalkylester und hydrolysiert den erhaltenen Niederalkylester der entsprechenden substituierten 5-Hydroxy-2-(3-pyridyl)-indol-3-carbonsäure (eine Verbindung der Formel I, worin $R_1$ Niederalkoxycarbonyl ist).

Die Umwandlung einer Verbindung der Formel Ia gemäss dem Verfahren 4), worin sich C von B unterscheidet, in eine Verbindung der Formel I, und die fakultative Umwandlung eines erhaltenen Produkts der Formel I in ein andere Verbindung dieser Erfindung, werden gemäss an sich bekannten chemischen Methoden und/oder wie hier beschrieben vorgenommen.

Umwandelbare Gruppen C sind vorzugsweise Trialkoxymethyl, verestertes Hydroxymethyl, veräthertes Hydroxymethyl, Halogenmethyl, Cyan, 2-Oxazolinyl, Dihydro-2-oxazolinyl, Niederalkanoyloxymethyl, Acetyl, Methyl, Carboxycarbonyl, Trihaloacetyl, Di-niederalkoxymethyl, Alkylendioxymethyl, Vinyl, Alkynyl, verestertes Carboxy und amidiertes Carboxy.

Die Ausgangstoffe der Formel Ia werden gemäss den Verfahren 1) bis 3) oder wie hier beschrieben, unter Verwendung von an sich bekannten chemischen Methoden hergestellt.

Die einzelnen Definitionen in den vorher beschriebenen Verfahren haben die folgenden Bedeutungen :

In einem reaktionsfähigen funktionellen Derivat eines Alkohols der Formel VI oder VIa ist die Hydroxygruppe z. B. mit einer starken anorganischen oder organischen Säure, vor allem mit einer Halogenwasserstoffsäure, z. B. Chlorwasserstoff-, Bromwasserstoff- oder Jodwasserstoffsäure, einer aliphatischen oder aromatischen Sulfonsäure, z. B. Methansulfon- oder p-Toluolsulfonsäure verestert. Diese Verbindungen werden in an sich bekannter Weise hergestellt.

Trialkoxymethyl bedeutet vorzugsweise Tri(niederalkoxy)-methyl, insbesondere Triäthoxy- oder Trimethoxy-methyl.

Veräthertes Hydroxymethyl bedeutet vorzugsweise tertiäres Niederalkoxymethyl, niederes Alkoxyalkoxymethyl, z. B. Methoxymethoxymethyl, 2-Oxa- oder 2-Thiacycloalkoxymethyl, insbesondere 2-Tetrahydropyranyloxymethyl.

Verestertes Hydroxymethyl bedeutet vorzugsweise Niederalkanoyloxymethyl, vorteilhafterweise Halogenmethyl, insbesondere Chlormethyl, aber auch Brommethyl oder Jodmethyl.

Ein Alkalimetall bedeutet vorzugsweise Lithium, es kann aber auch Kalium oder Natrium sein.

Zwischenprodukte der Formeln Ia oder Ib, in welchen C oder B' Halogenmethyl bedeutet, können vorzugsweise mit einem Alkalimetallcyanid, z. B. Kaliumcyanid, in an sich bekannter Weise umgesetzt

werden. Man erhält dabei Verbindungen der Formeln Ia oder Ib, in welchen die Kette um ein Kohlenstoffatom verlängert ist und C oder B' für Cyan steht. Diese können ihrerseits nach an sich bekannten Methoden in Verbindungen der Formel I, worin B Carboxy, Alkoxycarbonyl oder Carbamoyl bedeutet, übergeführt werden.

So können Verbindungen der Formeln Ia oder Ib, in welchen C oder B' Cyan bedeutet (Nitrile) in die Verbindungen der Formel I, worin B für Carboxy steht, durch Hydrolyse mit anorganischen Säuren, z. B. mit Halogenwasserstoffsäuren wie Chlorwasserstoffsäure oder Schwefelsäure in wässeriger Lösung, oder vorzugsweise durch Hydrolyse mit wässerigen Alkalimetallhydroxiden, z. B. Kaliumhydroxid bei Rückflusstemperatur, umgewandelt werden.

Die Ueberführung der genannten Nitrile in Verbindungen der Formel I, worin B Niederalkoxycarbonyl bedeutet, wird vorzugsweise durch Behandlung mit einem Niederalkanol, z. B. wasserfreiem Aethanol, in Gegenwart einer starken Säure, z. B. Chlorwasserstoffsäure, vorzugsweise unter Rückfluss, und nachfolgende vorsichtige Hydrolyse mit Wasser, vorgenommen.

Die Ueberführung der genannten Nitrile in Verbindungen der Formel I, worin B für Carbamoyl steht, wird vorzugsweise durch Behandlung mit einem Alkalimetallhydroxid, z. B. verdünntem Natriumhydroxid, und Wasserstoffsuperoxid, vorzugsweise bei Raumtemperatur, durchgeführt.

Zwischenprodukte der Formel Ia oder Ib, in welchen C oder B' halogenmethyl, z. B. Chlormethyl bedeutet, können in die Verbindungen der Formel I, worin B für Carboxy steht und die Kette um 2 Kohlenstoffatome verlängert ist, zuerst durch Behandlung z. B. mit einem Diniederalkyl-malonat, z. B. Diäthylmalonat, in Gegenwart einer Base, z. B. Kaliumcarbonat oder Natriumäthoxid, in einem Lösungsmittel wie Dimethylformamid, vorzugsweise bei einer Temperatur zwischen 50 und 100°, in substituierte Di-niederalkylmalonate umgewandelt werden. Diese werden dann mit einer wässerigen Base, z. B. verdünnter Natriumhydroxydlösung zu der entsprechenden Malonsäure hydrolysiert, welche unter Standardbedingungen, z. B. durch Erhitzen in Xylollösung, zu der Verbindung der Formel I, worin B Carboxy bedeutet, decarboxyliert wird. Ersetzt man den Malonsäure-di-niederalkylester durch einen Cyanessigsäure-niederalkylester, so erhält man die entsprechenden Verbindungen der Formel Ia oder Ib, in welchen C oder B' Cyan bedeutet.

Verbindungen der Erfindung, in welchen A ein geradkettiger oder verzweigter Alkenylenrest mit einer terminalen Doppelbindung bedeutet, können auch aus den Zwischenprodukten der Formel Ia oder Ib, worin C bzw. B' Halogenmethyl bedeutet, hergestellt werden. So können z. B. diese Zwischenprodukte zuerst mit einem Niederalkylester einer α-(Aryl- oder Alkyl)-thioessigsäure, z. B. α-(Phenyl-thio)-essigsäure-äthylester, in Gegenwart einer starken Base, z. B. Natriumhydroxid, behandelt werden. Die nachfolgende Oxidation des erhaltenen α-Arylthio oder α-Alkylthio substituierten Esters zu dem α-Arylsulfinyl- oder α-Alkylsulfinyl-ester mit z. B. Natriumperjodat und dann durch Hitze ausgelöste Eliminierung, z. B. durch Erhitzen in Xylol, ergibt eine Verbindung der allgemeinen Formel I (einen α,β-ungesättigten Ester) worin A Alkenylen bedeutet und B z. B. Niederalkoxycarbonyl ist. Die Kette wird gleichzeitig um zwei Kohlenstoffatome verlängert. Die gleiche Umwandlung wird auch unter Verwendung von z. B. α-(Phenylseleno)-essigsäure-äthylester, wie in J. Am. Chem. Soc. *95,* 6137 (1973) beschrieben, durchgeführt. Auch können die Verbindungen der Formel Ia, worin C Halogenmethyl bedeutet, zuerst in die entsprechenden Carboxaldehyde, z. B. mit Dimethylsulfoxid in Gegenwart von Triäthylamin und Silbertetrafluoroborat, oder mit Chromtrioxid und Pyridin in Methylenchlorid, umgewandelt werden. Die nachfolgende Wittig-Kondensation z. B. mit Trimethylphosphonoacetat oder (Triphenylphosphoranyliden)-essigsäure-äthylester ergibt die oben genannten α,β-ungesättigten Ester.

Verbindungen der Formel I, worin B Niederalkoxycarbonyl bedeutet, können mit Ammoniak, Mono- oder Di-niederalkylaminen, z. B. Methylamin oder Dimethylamin, in einem inerten Lösungsmittel, z. B. Niederalkanol wie Butanol, gegebenenfalls bei erhöhten Temperaturen, zu Verbindungen der Formel I, in welchem B unsubstituiertes Carbamoyl, Mono- oder Di-niederalkyl-carbamoyl bedeutet, amidiert werden.

Verbindungen der Formel I, worin A einen geradkettigen oder verzweigten Alkenylenrest mit einer terminalen Doppelbindung bedeutet, z. B. α,β-ungesättigte Ester können auch aus den entsprechenden α,β-gesättigten Estern durch Behandlung mit z. B. Phenylselenylchlorid in Gegenwart einer starken Base, gemäss der in J. Am. Chem. Soc. *95,* 6137 (1973) beschriebenen Methode, erhalten werden.

Die Umwandlung von Verbindungen der Formel I bzw. Ib, worin B bzw. B' Niederalkoxycarbonyl, Cyan, unsubstituiertes, Mono- oder Diniederalkylcarbamoyl bedeutet, in die Verbindungen der Formel I, in welchen B Carboxy ist, wird vorzugsweise durch Hydrolyse mit anorganischen Säuren, z. B. mit Halogenwasserstoffsäuren oder Schwefelsäure, oder mit wässerigen Alkalien, vorzugsweise mit Alkalimetallhydroxiden, z. B. Lithium- oder Natriumhydroxid, durchgeführt.

Verbindungen der Formel I, in welchen B Carboxy oder Niederalkoxycarbonyl bedeutet, können mit einfachen oder komplexen Leichmetallhydriden, z. B. mit Lithiumaluminiumhydrid, Alan oder Diboran, zu Verbindungen der Formel I, in welchen B für Hydroxymethyl steht, reduziert werden. Die Alkohole können auch durch geeignete Solvolyse von Zwischenprodukten der Formel Ia, worin C Halogenmethyl bedeutet, durch Behandlung z. B. mit einem Alkalimetallhydroxid, z. B. Lithium oder Natriumhydroxid, erhalten werden.

Die vorher genannten Alkohole können ihrerseits in Verbindungen der Formel I, worin B Carboxy bedeutet, mit konventionellen Oxidationsmitteln, vorzugsweise mit Pyridin-dichromat in Dimethylformamid bei Raumtemperatur, umgewandelt werden.

Freie Carbonsäuren können mit Niederalkanolen, z. B. Aethanol in Gegenwart einer starken Säure, z. B. Schwefelsäure, vorzugsweise bei erhöhten Temperaturen, oder mit Diazo-niederalkanen, z. B. Diazomethan in einem Lösungsmittel, z. B. Aethyläther, vorzugsweise bei Raumtemperatur, zu den entsprechenden Estern, nämlich zu solchen Verbindungen der Formel I, worin B Niederalkoxycarbonyl bedeutet, verestert werden.

Weiter können die freien Carbonsäuren durch Behandung ihrer reaktionsfähigen Zwischenprodukte, z. B. eines Acrylhalogenids, wie eines Säurechlorids, oder gemischten Anhydrids, z. B. eines das von einem Halogen-kohlensäure-niederalkylester, z. B. Chlorameisensäure-äthylester abgeleitet ist, mit Ammoniak, Mono- oder di-niederalkylaminen, in einem inerten Lösungsmittel, z. B. Methylenchlorid, vorzugsweise in Gegenwart eines basischen Katalysators, z. B. Pyridin, in die Verbindungen der Formel I, in welchen B für unsubstituiertes Carbamoyl, Mono- oder Di-(niederalkyl)-carbamoyl steht, umgewandelt werden.

Verbindungen der Formel I, worin B Mononiederalkyl-carbamoyl bedeutet, können in die Verbindungen der Formel I, in welchen B für Di-nieder-alkyl-carbamoyl steht, durch Behandlung mit einer starken Base, z. B. Natriumhydrid und dann mit einem Alkylierungsmittel, z. B. einem Niederalkylhalogenid, in einem inerten Lösungsmittel, z. B. Dimethylformamid, übergeführt werden.

Weiter können Verbindungen der Formel I, in welchen A einen geradkettigen oder verzweigten Alkynylen- oder Alkenylenrest bedeutet, durch katalytische Hydrierung, vorzugsweise unter neutralen Bedingungen, z. B. mit einem Palladium-Katalysator bei atmosphärischem Druck, in einem inerten Lösungsmittel, z. B. Aethanol, in die Verbindungen der Formel I, worin A für geradkettiges oder verzweigtes Alkylen steht, umgewandelt werden.

Die Carboxaldehyde, nämlich die Verbindungen der Formel I, in welchen B Formyl bedeutet, können durch Oxidation von Verbindungen der Formel Ia, worin C Hydroxymethyl bzw. Halogenmethyl bedeutet, mit z. B. Dimethylsulfoxid und einem Katalysator, z. B. einem Gemisch von Triäthylamin und Silbertetrafluoroborat, oder mit Chromtrioxid und Pyridin, oder mit anderen an sich bekannten geeigneten Oxidationsmitteln, hergestellt werden. Die genannten Carboxaldehyde können in die entsprechenden Acetale, d. h. Verbindungen der Formel Ia, worin C Di-(niederalkoxy)-methyl oder Alkylendioxymethyl bedeutet, z. B. Dimethylacetal, durch Säure katalysierte Kondensation mit einem Alkohol, z. B. Methanol, umgewandelt werden.

Verbindungen der Formel I, worin B Carboxy bedeutet, können durch die wohlbekannte Arndt-Eistert Synthese zu Verbindungen der Formel I, worin B Carboxy ist und die Kette ein Kohlenstoffatom mehr enthält, umgewandelt werden. Es wird insbesondere ein reaktionsfähiges funktionelles Derivat der als Ausgangsstoff verwendeten Carbonsäure, z. B. das Säurechlorid mit Diazomethan, z. B. in Diäthyläther behandelt, wobei man eine Verbindung der Formel Ia, worin C Diazoacetyl bedeutet, erhält. Umwandlung mit z. B. Silberoxid ergibt die genannte Carbonsäure der Formel I, worin die Kette A ein Kohlenstoffatom mehr enthält.

Eine besondere Ausführungsform des Verfahrens 4) betrifft die Herstellung von Verbindungen der Formel I, worin B Carboxy bedeutet. Sie besteht darin, dass man in einer Verbindung der Formel Ia, worin C einen in eine Carboxygruppe überfürhbaren Rest bedeutet, die Gruppe C, gegebenenfalls unter Verlängerung der Kette A innerhalb ihrer Definition, in die Carboxygruppe überführt.

In eine Carboxygruppe überführbare Reste sind z. B. veresterte Carboxygruppen, anhydrisierte Carboxygruppen, inklusive entsprechende Gruppen von asymmetrischen und inneren Anhydriden, amidierte Carboxygruppen, Cyan, Amidinogruppen, inklusive cyclische Amidinogruppen, wie 5-Tetrazolyl, Iminoäthergruppen, inklusive cyclische Iminoäthergruppen, wie z. B. durch Niederalkyl substituierte 2-Oxazolinyl- oder Dihydro-2-oxazolinylgruppen, ferner Methyl, Hydroxymethyl, veräthertes Hydroxymethyl, Niederalkanoyloxymethyl, Trialkoxymethyl, Acetyl, Trihaloacetyl, Halomethyl, Carboxycarbonyl (COCOOH), Formyl (CHO), Di-niederalkoxymethyl, Alkylendioxymethyl, Vinyl, Aethynyl, oder Diazoacetyl. Bei der Umwandlung in die Carboxygruppe kann gleichzeitig die Kette A innerhalb ihrer Definition verlängert werden.

Veresterte Carboxygruppen sind vorzugsweise in der Form ihrer Niederalkylester, z. B. Methyl-, Aethyl-, n- oder iso-(Propyl oder Butyl)-ester. Ferner in der Form von substituierten Niederalkylestern, z. B. ω-Amino-, ω-Mono- oder Dimethylamino-, α-Carboxy- oder α-Carbäthoxy-(äthyl, propyl oder butyl)-ester. Weitere Ester sind Aryl-niederalkylester, z. B. Benzyl-, (Methyl, Methoxy-, Chlor)-subsituierte Benzyl und Pyridylmethylester ; Niederalkanoyloxy-niederalkylester z. B. Pivaloyloxymethylester ; 3-Phthalidyl- und durch Methyl, Methoxy oder Chlor substituierte 3-Phthalidylester, welche von entsprechenden 3-Hydroxy-phthalsäureestern abgeleitet sind, (Hydroxy, Niederalkanoyloxy, Niederalkoxy)-substituierte Niederalkoxy-methylester, z. B. β-(Hydroxy, Acetyloxy, Methoxy)- äthoxymethylester ; Bicycloalkoxycarbonyl-niederalkylester, z. B. solche, die von bicyclischen Monoterpenoidalkoholen, z. B. unsubstituierten oder durch Niederalkyl substituierten Bicyclo [2,2,1] heptyloxycarbonyl-niederalkylester, vorzugsweise Bornyloxycarbonylmethylester ; Halogen substituierte Niederalkylester, z. B. Trichloräthyl- oder Jodäthylester.

Amidierte Carboxygruppen sind vorzugsweise Carboxygruppen in der Form ihrer unsubstituierten Amide ; N-Mono- oder Di-niederalkylamide, z. B. Mono- oder Di-methylamide ; tertiären Amide, welche z. B. von Pyrrolidin, Piperidin oder Morpholin abgeleitet sind ; durch α-Carboniederalkoxy oder Carboxy-substituierte Niederalkylamide, z. B. Mono-N-(carboäthoxymethyl)-amide, und Mono-N-(carboxymethyl)-

amide ; α-Carboniederalkoxy oder Carboxy-substituierte Aryl-niederalkylamide, z. B. (Carboäthoxy oder Carboxy) substituierte Phenyläthylamide ; Amino-niederalkylamide, z. B. β-Aminoäthylamide und β-(Carbobenzyloxy-amino)-äthylamide.

Die Ueberführung in die Carboxygruppe wird nach an sich bekannten Methoden, wie hier oder in den Beispielen beschrieben, z. B. solvolytisch, wie hydrolytisch oder acidolytisch, oder reduktiv (veresterte Carboxygruppen) vorgenommen. So werden z. B. Trichloräthyl oder 2-Jodäthylester durch Reduktion, z. B. mit Zink und einer Carbonsäure in Gegenwart von Wasser in die Carbonsäure überführt. Benzylester oder Nitro-benzylester können durch katalysche Hydrierung, letztere auch mit chemischen Reduktionsmiteln, z. B. Natriumdithionit oder mit Zink und einer Carbonsäure, in die Carboxygruppe umgewandelt werden. Ferner können z. B. tert.-Butylester auch z. B. mit Trifluoressigsäure gespalten werden.

Bei der Reduktion der Gruppe C kann eine Alkenylen- oder Alkynylenkette A in die entsprechende Alkylenkette umgewandelt werden.

Weiter können Verbindungen der Formel Ia, worin C Acetyl bedeutet, oxidativ zu den entsprechenden Verbindungen der Formel I, worin B Carboxy bedeutet, gespalten werden. Zuerst wird der Ausgangsstoff in eine Verbindung Ia, worin C Trihaloacetyl, z. B. Tribrom- oder Trijodacetyl bedeutet, z. B. durch Behandlung mit Natrium-hypobromit umgewandelt und nachfolgend z. B. mit einer wässerigen Base z. B. Natrium-hydroxid, gespalten.

Ausgangsstoffe der Formel Ia, in welchen C Acetyl bedeutet, können ausgehend von Verbindungen Ia, worin C Halomethyl bedeutet, durch Behandlung mit einem Acetessigsäure-niederalkylester, z. B. Acetessigsäure-äthylester, in Gegenwart einer Base, z. B. Natriumhydrid und nachfolgende Hydrolyse mit einer starken Base, z. B. mit wässerigem Natriumhydroxid, hergestellt werden.

Die genannten Verbindungen können auch durch Kondensation einer Verbindung der Formel Ia, worin C Cyan bedeutet, mit z. B. einem Grignard- oder einem anderen Organometall-Reagens, z. B. Methyl-magnesium-bromid, unter Standardbedingungen, hergestellt werden.

Ausgangsstoffe der Formel Ia, worin C Carboxycarbonyl (COCOOH) bedeutet, werden durch thermische Behandlung oder durch Oxidation in die Verbindungen der Formel I, worin B Carboxy bedeutet, überführt. Der Ausgangsstoff wird dabei auf eine erhöhte Temperatur, z. B. ungefähr 200°, in Gegenwart von Glaspulver erhitzt, oder z. B. mit Wasserstoffsuperoxid in Gegenwart eines basischen Mitteln, z. B. Natriumhydroxid, behandelt.

Die Ausgangsstoffe der Formel Ia, worin C COCOOH bedeutet, können z. B. durch Kondensation einer Verbindung der Formel Ia, worin C Halomethyl bedeutet, mit z. B. 2-Aethoxycarbonyl-1,3-dithian, und nachfolgende oxidative Hydrolyse, z. B. mit N-Bromsuccinimid in wäserigem Aceton und dann durch Behandlung mit verdünntem wässerigem Natriumhydroxid, erhalten werden.

Verbindungen der Formel Ia, worin C Formyl, Di-niederalkoxy-methyl oder Alkylendioxymethyl (Formyl geschützt in der Form eines Acetals), z. B. Dimethylacetal bedeutet, werden z. B. mit silbernitrat, Pyridiniumdichromat oder Ozon zu den Verbindungen der Formel I, worin B Carboxy bedeutet, oxidiert.

Verbindungen der Formel Ia, worin C Vinyl bedeutet, werden in die Verbindungen der Formel I, worin B Carboxy ist, zuerst durch Ozonolyse in die Verbindungen der Formel I, worin B Formyl bedeutet, umgewandelt. Diese werden dann zu den Verbindungen der Formel I, worin B Carboxy bedeutet, oxidiert.

Ausgangsstoffe der Formel Ia, worin C Vinyl bedeutet können auch mit Nickelcarbonyl und Kohlenmonoxid unter Hochdruck behandelt werden, wobei man Verbindungen der Formel I, worin B Carboxy bedeutet und die Kette A neben der Carboxygruppe eine Doppelbindung enthält.

Verbindungen der Formel Ia, worin C Aethynyl bedeutet können mit einer starken Base, z. B. Butyllithium behandelt, dann mit Kohlendioxid oder einem Halogenameisensäure-niederalkylester, z. B. Chlorameinsensäureäthylester kondensiert und nachfolgend hydrolysiert werden. Man erhält Verbindungen der Formel I, worin B Carboxy bedeutet und die Kette A in benachbarter Stellung zu der Carboxygruppe eine Dreifachbindung enthält.

Verbindungen der Formel Ia, worin C Halomethyl bedeutet können in die entsprechenden metallorganischen Zwischenprodukte, z. B. Kupfer- oder Magnesiumderivate nach an sich bekannten Methoden umgewandelt werden.

Kondensation z. B. eines erhaltenen organischen Magnesium-(Grignard)-Reagenses, z. B. mit einer Verbindung der Formel Ia, worin C z. B. in $CH_2MgCl$ umgewandelt ist, mit Kohlendioxid, ergibt eine Verbindung der Formel I, worin B Carboxy bedeutet und die Kette um ein Kohlenstoffatom verlängert ist.

Kondensation des genannten Grignard-Reaganses mit z. B. einem Halogenessigsäure-niederalkylester, z. B. Bromessigsäure-äthylester und nachfolgende Hydrolyse ergibt eine Verbindung der Formel I, worin B Carboxy bedeutet und die Kette um 2 Kohlenstoffatome verlängert wird.

Das genannte Grignard-Reagens kann in Gegenwart eines Kupfer-I-halogenids, z. B. Kupfer-I-chlorids, mit einer α,β-ungesättigten Säure oder einem Ester, z. B. mit Propiolsäure oder Acrylsäure, kondensiert werden, wobei man eine Verbindung der Formel I erhält, worin B Carboxy bedeutet und die Kette durch 3 Kohlenstoffatome verlängert wird.

Ferner können Verbindungen der Formel Ia, worin C Halomethyl bedeutet mit z. B. dem 3-Lithio-Derivat der Propiolsäure (hergestellt in situ z. B. mit Lithium-diisopropylamid) kondensiert werden, wobei man eine Verbindung der Formel I erhält, in welcher A ein terminales Alkynylen bedeutet, B für Carboxy steht und die Kettenlänge um 3 Kohlenstoffatome verlängert wird.

Verbindungen der Formel I, worin A Niederalkylen oder eine direkte Bindung bedeutet und B für Hydroxymethyl, als reaktionsfähiges funktionelles Derivat, steht, können mit einem Niederalkanol (oder Thiol), oder einem Phenol (oder Thiophenol), (welche in geeigneter Weise durch B substituiert sind), vorzugsweise in Gegenwart einer starken Base, kondensiert werden. Man erhält Verbindungen der Formel I, worin A Niederalkylen- (thio oder oxy)-phenylen oder Niederalkylen-(thio oder oxy)-niederalkylen bedeutet.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln, und/oder in einer inerten Atmosphäre, unter Kühlung, bei Zimmtertemperatur oder bei erhöhten Temperaturen, vorzugsweise beim Siedepunkt des verwendeten Lösungsmittels, bei normalen oder erhöhtem Druck, durchgeführt. Die bevorzugten Lösungsmittel, Katalysatoren und Reaktionsbedingungen sind in den beigefügten Beispielen offenbart.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangstoff in Form eines Salzes oder optisch reinen Antipoden verwendet wird.

Im Verfahren der vorliegenden Erfindung werden vorteilhafterweise solche Ausgangstofe verwendet, welche zu den im vorstehenden als besonders wertvoll beschriebenen Verbindungen führen.

Die Erfindung betrifft auch die neuen Ausgangsstoffe und Verfahren zu ihrer Herstellung.

Je nach der Wahl von Ausgangsstoffen und Verfahren, können die neuen Verbindungen in Form eines der möglichen Isomeren oder Gemischen von solchen, vorliegenden, so können sie z. B. je nach der Anwesenheit einer Doppelbindung und nach der Anzahl der asymmetrischen Kohlenstoffatome, als reine optische Isomeren, z. B. als Antipoden, oder als Gemische von optischen Isomeren, z. B. als Racemate, Gemische von Diastereomeren, Gemische von Racematen oder Gemische von geometrischen Isomeren sein. Die vorher genannten möglichen Isomeren und ihre Gemische gehören dem Bereich dieser Erfindung an. Manche spezifischen Isomeren können bevorzugt sein.

Erhaltene Gemische von Diastereomeren, Gemische von Racematen oder geometrischen Isomeren können auf des Basis der physikochemischen Unterschiede der Komponenten, in an sich bekannter Weise, in die reinen Isomeren, Diasteromeren, Racematen oder geometrischen Isomeren, z. B. durch Chromatographie und/oder fraktionierte Kristallisatin, getrennt werden.

Erhaltene Racemate können weiter in die optischen Antipoden in an sich bekannter Weise, z. B. durch Umsetzung des saueren Endproduktes mit einer optisch aktiven Base, welche mit der racemischen Säure Salze bildet, aufgetrennt werden. Diese Salze können z. B. durch fraktionierte Kristallisation in die diastereomeren Salze getrennt werden. Aus den letzteren können die optisch aktiven Säure-Antipoden durch Ansäuern freigesetzt werden. Basische racemische Produkte können in analoger Weise, wie durch Trennung ihrer diastereomeren Salze mit einer optisch aktiven Säure und Freisetzung der optisch aktiven Base mit einer Base, getrennt werden. Racemische Produkte der Erfindung können so in ihre optischen Antipoden, z. B. durch fraktionierte Kristallisation von d- oder l-(Tartraten, Mandelaten, Camphersulfonaten, oder von d- oder l-($\alpha$-Methylbenzylamin, Cinchonidin, Cinchonin, Chinin, Chinidin, Ephedrin, Dehydroabietylamin, Brucin oder Strychin)-salzen, gespalten werden. Vorzugsweise wird von den zwei Antipoden der stärker wirksame isoliert.

Schliesslich können die Verbindungen der Erfindung in freier Form oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z. B. durch Behandlung mit einer stärkeren Base, wie mit einem Metallhydroxyd oder Ammoniumhydroxyd oder einem basischen Salz, z. B. einem Alkalimetallhydroxid oder -carbonat, oder einem basischen Salz, z. B. einem Alkalimetallhydroxid oder -carbonat, oder einem Kationenaustauscher, umgewandelt werden. Eine Verbindung der Formel I, in welcher B Carboxy bedeutet, kann auch in die entsprechenden Metall- oder Ammoniumsalze übergeführt werden. Diese oder andere Salze, z. B. die Pikrate, können auch in der Reinigung von den erhaltenen freien Basen verwendet werden. Die Basen werden in ihre Salze übergeführt, die Salze abgetrennt und die Basen aus den Salzen freigesetzt.

Infolge der engen Beziehung zwischen den neuen Verbindungen in freier Form und in Form ihrer Salze sind in vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in Form ihrer Hydrate erhalten werden oder andere, für die Kristallisation verwendeten Lösungsmittel einschliessen.

Die erfindungsgemässen pharmazeutischen Präparate sind solche, die sich für enterale, z. B. orale oder rektale, und parenterale Verabreichung an Säugern, inklusive Menschen, eignen. Die Präparate werden für die Behandlung oder Vorbeugung von Krankheiten, welche auf die Hemmung der Thromboxan-Synthetase, reagieren, verwendet. Diese Präparate enthalten eine wirksame Dosis einer pharmakologisch aktiven Verbindung der Formel I, oder eines pharmazeutisch verwendbaren Salzes davon, allein oder im Kombination mit einem oder mehreren pharmazeutisch annehmbaren Trägermaterialien.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinkappseln, welche den Wirkstoff zusammen mit Verdünnungsmitteln, z. B. Laktose, Dextrose, Rohrzucker, Mannitol, Sorbitol, Cellulose und/oder Glycin, und Schiermitteln, z. B. Kieselerde, Talk, Stearinsäure oder Salze davon, wie Magnesium- oder Calciumstearat, und/oder Polyäthylenglykol, aufweisen ; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausenmischungen oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1 % bis etwa 75 %, insbesondere von etwa 1 % bis etwa 50 %, des Aktivstoffes. Einzeldosen für Säuger mit einem Gewicht von ungefähr 50-70 g können zwischen ungefähr 10-100 mg des aktiven Bestandteils enthalten.

Die folgenden Beispiele dienen zur Illustration der Erfindung und sie sind nicht als Einschränkung ihres Umfangs aufzufassen. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z. B. zwischen ungefähr 15 und 100 mg/Hg, durchgeführt.

## Beispiel 1

Man füllt in einen Glaskessel von 76 Litern 1 640 ml Dimethylformamid und 430 g Kalium-tert.-butoxid. Die Lösung wird unter Stickstoff gerührt und auf – 8° gekühlt. Man gibt innerhalb von 0,75 Stunden eine Lösung von 682 g 3-Methyl-2-(3-pyridyl)-indol in 3 280 ml Dimethylformamid dazu, wobei man die Temperatur unter O° hält. Man rührt das Gemisch 2 Stunden bei – 10° und gibt innerhalb 1 Stunde 1 640 ml einer Lösung von 780 g 8-Bromoctansäure-methylester in Dimethylformamid dazu. Die Reaktionstemperatur wird unter 0° gehalten. Man rührt das Reaktionsgemisch 2 Stunden und lässt es sich über Nacht auf Raumtemperatur erwärmen. Das rostfarbige Gemisch wird dann auf ungefähr 5° gekühlt und mit 19 700 ml Eiswasser behandelt. Die Temperatur steigt auf 25°. Das Gemisch wird 0,5 Stunden gerührt und mit 2 × 8 000 ml Aether extrahiert. Die Extrakte werden über Magnesiumsulfat getrocknet und im Vakuum eingedampft. Man erhält das 1-(7-Methoxycarbonylheptyl)-3-methyl-2-(3-pyridyl)-indol als ein Oel. Man behandelt 1 293 g dieses Oels mit 6 530 ml 1-normaler Natriumhydroxidlösung und erhitzt das Gemisch auf dem Dampfbad 2,5 Stunden auf 90°. Die Lösung wird auf Raumtemperatur gekühlt und mit 3 × 3 000 ml Aether gewaschen. Die wässerige Schicht wird auf 10° gekühlt und mit 3 400 ml 2-normaler Chlorwasserstoffsäure auf den pH-Wert, 3,5 eingestellt. Die erhaltene schwere Suspension wird mit 4 × 4 000 ml Methylenchlorid extrahiert. Die vereinigten Extrakte werden einmal mit 4 000 ml Wasser gewaschen und über Magnesiumsulfat getrocknet. Man filtriert, dampft das Lösungsmittel bei 60° ab, trituriert den Rückstand mit 2 000 ml Aether und Trocknet das Produkt. Man erhält das 1-(7-Carboxyheptyl)-3-methyl-2-(3-pyridyl)-indol. F. 113-115°. Nach Umkristallisation aus Aethanol steigt der Schmelzpunkt auf 114-116°.

Der 3-Methyl-2-(3-pyridyl)-indol-Ausgangsstoff wird im wesentlichen gemäss dem USA-Patent 3,468,894 hergestellt.

Der 8-Bromoctansäure-methylester wird aus Azelainsäure im wesentlichen gemäss dem USA-Patent 3,852,419, oder durch direkte Veresterung der 8-Bromoctansäure, wie folgt hergestellt :

Ein Gemisch von Methanol (4 700 ml), 8-Bromoctansäure (912 g) und Schwefelsäure (912 ml) wird in einem geeigneten Reaktionsgefäss 5 Stunden unter Rückfluss gekocht und über Nacht bei Raumtemperatur gerührt. Das Lösungsmittel wid unter vermindertem Druck (3 mm Hg) abgedampft und der ölige Rückstand in Aether (4 000 ml) gelöst. Die Lösung wird mit Wasser (3 × 2 000 ml), gesättigter wässeriger Natriumhydrogencarbonatlösung (1 000 ml) und gesättigter wässeriger Natriumchloridlösung gewaschen. Die Aetherschicht wird über Magnesiumsulfat getrocknet und filtriert. Nach Abdampfen des Lösungsmittels und Destillation des rohen Oels erhält man den 8-Bromoctansäuremethylester, der bei 73-76°/0,05 mmHg siedet. $n_D^{23}$ = 1,4614.

## Beispiel 2

Zu einer Suspension, welche man durch Verdünnung von 4,8 g einer 50 %igen Natriumhydrid-Suspension in Mineralöl mit 40 ml Dimethylformamid erhält, gibt man tropfenweise, unter Stickstoff, eine Lösung von 13,5 g 3-Methyl-2-(3-pyridyl)-indol in 80 ml Dimethylformamid. Nach der Beendigung der Zugabe wird as grüngelbe Gemisch bei Zimmertemperatur ungefähr 1 Stunde gerührt. Das Reaktionsgemisch wird tropfenweise, unter Kühlung auf 0-5°, mit Bromessigsäure-äthylester (11,2 ml, 0,10 Mol) versetzt und 4 Stunden bei Raumtemperatur gerührt.

Das Reaktionsgemisch wird in 1 000 ml Eiswasser gegossen und mit 3 × 300 ml Aether extrahiert. Die Aetherschicht wird mit 3 × 300 ml 1-normaler Chlorwasserstoffsäure extrahiert. Der sauere Extrakt wird mit konzentriertem Ammoniumhydroxid auf den pH-Wert 9-10 eingestellt und mit 3 × 250 ml Aether extrahiert. Die vereinigten Aetherextrakte werden über Magnesiumsulfat getrocknet, filtriert und unter vermindertem Druck eingedampft. Man erhält das 1-Aethoxycarbonylmethyl-3-methyl-2-(3-pyridyl)-indol als ein Oel.

Das vorher genannte Oel wird in 500 ml 1-normaler Chlorwasserstoffsäure 4 Stunden unter Rückfluss gekocht. Nach Stehenlassen über Nacht wird ein gelbes festes Material abgetrennt und 12 Stunden bei 60-80°/30 mmHg getrocknet. Nach Umkristallisation aus Aethanol erhält man das 1-Carboxymethyl-3-methyl-2-(3-pyridyl)-indol-hydrochlorid. F. 204-207°.

Wünscht man die freie Aminosäure, so kann diese durch Einstellung des pH-Wertes des Hydrolyse-Mediums auf 3,5 erhalten werden.

## Beispiele 3-6

Analog zu den Verfahren der Beispiele 1 und 2 werden auch die Verbindungen der Formel II, worin $R'_1 = CH_3$, $R'_2$ und $R'_3 = H$ und $R_4 = OH$, hergestellt :

| Beispiel | Ester-Ausgangsstoff | $C_mH_{2m}$ | Pyr | F° | Umkristallisiert aus |
|---|---|---|---|---|---|
| 3 | $Br(CH_2)_5COOEt$* | $(CH_2)_5$ | 3-Pyridyl | 113-4 | Acetonitril |
| 4 | $Br(CH_2)_6COOMe$ | $(CH_2)_6$ | 3-Pyridyl | 106-7,5 | Acetonitril |
| 5 | $Br(CH_2)_4COOMe$ | $(CH_2)_4$ | 3-Pyridyl | 123-5 | Aethanol |
| 6 | $Br(CH_2)_5COOEt$ | $(CH_2)_5$ | 4-Pyridyl | 136-8 | Acetonitril |

* Et = Aethyl ; Me = Methyl.

Die als Ausgangsstoffe verwendeten 2-(3- und 4-Pyridyl)-indole werden gemäss dem USA-Patent 3,468,894 hergestellt.

Die als Ausgangstoffe verwendeten Aethyl- oder Methyl-ω-brom-erster sind käuflich oder werden von käuflichen ω-Brom-säuren, wie dies nachfolgend für den 6-Brom-hexansäure-methylester illustriert ist, hergestellt : Eine Lösung von 6-Brom-hexansäure (10 g) in 50 ml Methanol wird mit 1,0 ml konzentrierter Schwefelsäure versetzt und 8 Stunden unter Rückfluss gekocht. Das Methanol wird abgedampft und der Rückstand in Aether gelöst. Die Aetherlösung wird durch Waschen mit Wasser von der Säure befreit, über Natriumsulfat getrocknet und zur Trockene eingedampft. Die Destillation bei 0,8 mmHg ergibt den 6-Brom-hexansäure-methylester, der bei 85-90°/0,8 mmHg siedet.

Der 1-(7-Carboxyheptyl)-3-methyl-2-(2-pyridyl)-indol wird analog zum Beispiel 1 hergestellt. Das als Ausgangsstoff verwendete 3-Methyl-2-(2-pyridyl)-indol ist in J. Chem. Soc. *1955,* 2865 beschrieben.

Die entsprechenden Verbindungen der Formel II, worin $R'_1$ = Wasserstoff, Pyr = 2-, 3- oder 4-Pyridyl und $R'_2$ = Fluor, Wasserstoff oder Methyl, und $R'_3$ = Wasserstoff, werden in analoger Weise, unter Verwendung der in den Beispielen 1 und 2 beschriebenen Verfahren hergestellt. Als Ausgangsstoffe werden die erforderlichen ω-Brom-ester und die nachfolgend genannten bekannten 2-(Pyridyl)-indole eingesetzt : Die 2-(2-, 3- und 4-Pyridyl)-indole sind in Pharm. Bull. Japan 4, 16 (1956) und die 5-(Fluor und Methyl)-2-(3-pyridyl)-indole in Bull. Soc. Chim. France *1969,* 4154 beschrieben.

## Beispiele 7 und 8

Die folgenden Verbindungen der Formel II, worin $R'_1 = CH_3$, $R'_3$ = Wasserstoff, Pyr = 3-Pyridyl, $C_mH_{2m} = (CH_2)_5$ und $R_4 = OH$ werden analog zu den in den vorhergehenden Beispielen beschriebenen Verfahren hergestellt.

| Beispiel | $R'_2$ | F° | Salz |
|---|---|---|---|
| 7 | 5-Cl | 143-145 | - |
| 8 | $5-OCH_3$ | 175-178 | HCl |

Die Verbindung des Beispiels 7 wird wie folgt hergestellt : Zu einer Suspension, welche man durch Verdünnung von 1,39 g einer 50 %igen Natriumhydrid-Suspension in Mineralöl mit 30 ml Dimethylformamid erhält, gibt man unter Rühren in einer Stickstoffatomosphäre bei 0-5°, tropfenweise eine Lösung von 6,59 g 5-Chlor-3-methyl-2-(3-pyridyl)-indol (hergestellt gemäss dem USA- Patent 3,468,894) in 60 ml Dimethylformamid. Nach der Beendigung der Zugabe wird die Suspension 1/2 Stunde bei 0° gerührt und dann unter Kühlen auf 0° mit einer Lösung von 6,06 g 6-Brom-hexansäure-methylester in 10 ml Dimethylformamid tropfenweise versetzt. Man lässt das Reaktionsgemisch sich auf Raumtemperatur erwärmen, rührt es 5 Stunden bei Raumtemperatur und giesst es in 400 ml Eiswaser. Das erhaltene Gemisch wird mit Essigsäureäthylester (3 × 300 ml) extrahiert. Der Extrakt wird mit gesättigter wässeriger Natriumchloridlösung gewaschen, über Magnesiumsulfat getrocknet und zur Trockene eingedampft. Man erhält das 1-(5-Methoxycarbonylpentyl)-5-chlor-3-methyl-2-(3-pyridyl)-indol als ein Oel.

Eine Lösung von 3,2 g der letztgenannten Verbindung in 30 ml 3-normaler Natriumhydroxidlösung wird 17 Stunden unter Rückfluss gekocht. Nach Abkühlen wird das erhaltene Produkt abfiltriert und in 50 ml Wasser gelöst. Nach Ansäuern mit 2-normaler Chlorwasserstoffsäure auf den pH-Wert 4-5, fällt das Produkt aus. Es wird durch Suspendieren in Aether gereinigt. Man erhält das 1-(5-Carboxypentyl)-5-chlor-3-methyl-2-(3-pyridyl)-indol. F. 143-145°.

Aehnlich wird das 1-(5-Carboxypentyl)-5-methoxy-3-methyl-2-(3-pyridyl)-indol als ein Oel hergestellt. Nach Behandlung mit äthanolischer Chlorwasserstoffsäure in Aethanol und Kristallisation durch Zugabe von Aethyläther, erhält man die Verbindung des Beispiels 8, nämlich das 1-(5-Carboxypentyl)-5-methoxy-3-methyl-2-(3-pyridyl)-indolhydrochlorid, welches bei 175-178° schmilzt.

Das 1-(5-Carboxypentyl)-5-hydroxy-3-methyl-2-(3-pyridyl)-indol wird wie folgt hergestellt : Eine Lösung von 1,70 g 1-(5-Carboxypentyl)-5-methoxy-3-methyl-2-(3-pyridyl)-indol in 85 ml 48 %iger Bromwasserstoffsäure wird 30 Minuten unter Rückfluss gekocht. Das Reaktionsgemisch wird zur Trockene eingedampft, mit Wasser verdünnt und der pH-Wert mit verdünnter Natiumhydroxidlösung auf 6 gestellt. Der Niederschlag wird abgetrennt und aus Aceton/Essigsäureäthylester umkristallisiert. Man erhält das 1-(5-Carboxypentyl)-5-hydroxy-3-methyl-2-(3-pyridyl)-indol.

Beispiele 9 und 10

Die folgenden Beispiele der Formel III, worin $C_pH_{2p}$ den Rest $CH_2$—$CH_2$ bedeutet und Pyr für 3-Pyridyl steht, werden im wesentlichen gemäss dem Verfahren des Beispiels 2 hergestellt. Kondensation des 3-Methyl-2-(3-pyridyl)-indol-5-propionsäure-äthylesters mit 6-Bromhexansäure-äthylester bzw. mit 8-Bromoctansäuremethylester ergibt die Ester der Beispiele 9a und 10a. Hydrolyse mit Chlorwasserstoffsäure ergibt die entsprechenden Di-säuren der Beispiele 9 und 10.

| Beispiel | $C_nH_{2n}$ | F° | $R_5$ | $R_6$ | Umkristallisiert aus |
|---|---|---|---|---|---|
| 9a | $(CH_2)_5$ | Oel | $OC_2H_5$ | $OC_2H_5$ | --- |
| 9 | $(CH_2)_5$ | 143-145 | OH | OH | Acetonitril |
| 10a | $(CH_2)_7$ | Oel | $OCH_3$ | $OC_2H_5$ | -- |
| 10 | $(CH_2)_7$ | 128-130 | OH | OH | Acetonitril |

Die 1-Carboxheptyl-3-methyl-2-(4-pyridyl)-indol-5-propionsäure wird in analoger Weise hergestellt.

Die als Ausgangsstoffe verwendeten Indole werdn wie folgt hergestellt : Eine Suspension von p-Hydrazinohydrocinnamonsäure [Manske and Kulka, Can. J. Res. B *25*, 376 (1947), 4,50 g] in 50 ml absolutem Aethanol wird unter Stickstoff bei Zimmertemperatur, unter Rühren, mit 10 ml gesättigter äthanolischer Chlorwasserstofflösung versetzt. Man erhält eine Lösung in ungefähr 5 Minuten, die rotorangefarbige Lösung wird mit 3-Propionylpyridin (3,37 g, 0,025 Mol) versetzt, das Reaktionsgemisch zum Rückfluss erhitzt und 18 Stunden unter Rückfluss gehalten. Die erhaltene Lösung wird in einem Eiswasserbad gekühlt und die erhaltenen Kristalle des 3-Methyl-2-(3-pyridyl)-indol-5-propionsäureäthyl ester-hydrochlorids werden abgetrennt. F. 249-251°. Die freie Base, nämlich der 3-Methyl-2-(3-pyridyl)-indol-5-propionsäureäthylester wird durch Aufschlämmung des Hydrochlorid-Salzes in Wasser, Basischmachen mit 3-normaler Natriumhydroxidlosung und Extraktion mit Aether hergestellt.

In analoger Weise wird das 3-Methyl-2-(4-pyridyl)-indol-5-propionsäureäthylester-Hydrochlorid, F. höher als 275° und die entsprechende freie Base hergestellt.

Erhitzt man 2 Stunden eine Suspension von 7,5 g 3-Methyl-2-(3-pyridyl)-indol-5-propionsäure-äthylester-hydrochlorids in 450 ml 2-normaler Chlorwasserstoffsäure unter Rückfluss, kühlt sie ab und trennt das erhaltene feste Material ab, so erhält man das 3-Methyl-2-(3-pyridyl)-indol-5-propionsäure-hydrochlorid, welches bei 290° schmilzt.

Eine ähnliche Hydrolyse des 3-Methyl-2-(4-pyridyl)-indol-5-propionsäure-äthylester-hydrochlorids ergibt das 3-Methyl-2-(4-pyridyl)-indol-5-propionsäure-hydrochlorid, welches über 305° schmilzt.

Beispiel 11

a) Eine Lösung von 1-(4-Cyanbenzyl)-3-methyl-2-(3-pyridyl)-indol (5,8 g) in 100 ml eines 1 : 1-Gemisches von 20 %iger wässeriger Chlorwasserstoffsäure und Eisessig wird 20 Stunden unter Rückfluss gekocht. Nach Abkühlen wird die Lösung in Eiswasser (100 ml) gegossen und der pH-Wert mit gesättigte wässeriger Natriumhydrogencarbonatlösung auf 4,5-5gestellt. Der erhaltene Niederschlag wird mit Essigsäureäthylester extrahiert, der Extrakt mit Wasser gewaschen und zur Trockene eingedampft. Man erhält das 1-(4-Carboxybenzyl)-3-methyl-2-(3-pyridyl)-indol. F. 273-275°.

Das als Ausgangsstoff verwendete Nitril wird wie folgt hergestellt : Eine Suspension von 2,9 g (0,06 Mol) 50 %igem Natriumhydrid in Mineralöl, in 40 ml Dimethylformamid wird unter Stickstoff bei 0-5°, innerhalb 20 Minuten, mit einer Lösung von 10,4 g (0,05 Mol) 3-Methyl-2-(3-pyridyl)-indol in 60 ml Dimethylformamid tropfenweise versetzt. Das Reaktionsgemisch wird eine halbe Stunde bei 0-5° gerührt, und dann tropfenweise mit 9,8 g (0,05 Mol) p-Cyan-benzylbromid in 50 ml Dimethylformamid versetzt. Das Gemisch wird 1 Stunde bei 0-10°, dann bei Raumtemperatur 30 Minuten gerührt und in Eiswasser (600 ml) gegossen. Das erhaltene feste Material wird gesammelt, getrocknet, mit Petroläther gewaschen und in Aether (500 ml) wieder aufgelöst. Die Aetherlösung wird zuerst mit Wasser, dann mit gesättigter wässeriger Natriumhydrogencarbonatlösung gewaschen, über Magnesiumsulfat getrocknet, mit Aktivkohle behandelt und filtriert. Nach Eindampfen der Aetherlösung zur Trockene erhält man ein gelbes festes Material. Dieses Produkt wird in heisem cyclohexan aufgeschlämmt und abfiltriert. Man erhält das 1-(4-Cyanbenzyl)-3-methyl-2-(3-pyridyl)-indol. F. 127-129°.

b) In analoger Weise erhält man das 1-(4-Carboxybenzyl)-5-chlor-3-methyl-2-(3-pyridyl)-indol-hydrochlorid. F. 217-220°.

Beispiel 12

a) Eine Suspension von 0,49 g Lithiumaluminiumhydrid in 50 ml wasserfreiem Tetrahydrofuran wird in einer Stickstoffatmosphäre bei Raumtemperatur mit einer Lösung von 3,92 g 1-(5-Methoxycarbonylpentyl)-5-chlor-3-methyl-2-(3-pyridyl)-indol in 30 ml wasserfreiem Tetrahydrofuran tropfenweise versetzt. Nach der Beendigung der Zugabe wird die Suspension 1 Stunde bei Zimmertemperatur gerührt und mit 50 ml gesättigter wässeriger Ammoniumchloridlösung versetzt. Das Reaktionsgemisch wird über Nacht bei Raumtemperatur stehen gelassen und die organische Schicht abgetrennt. Die wässerige Schicht wird zwecks Abtrennung von Salzen filtriert und mit Essigsäureäthylester (2 × 50 ml) extrahiert. Die vereinigten organischen Schichten werden mit gesättigter wässeriger Natriumchloridlösung gewaschen, über Magnesiumsulfat getrocknet und im Vakuum eingedampft. Das Rohprodukt wird durch Triturieren mit Hexan/Aether gereinigt und in Aethanol gelöst. Die Lösung wird mit äthanolischer Chlorwasserstoffsäure angesäuert und die Lösung mit wasserfreiem Aether verdünnt, um das kristalline Produkt auszufällen. Das erhaltene 1-(6-Hydroxyhexyl)-5-chlor-3-methyl-2-(3-pyridyl)-indol-hydrochlorid-hemihydrat schmilzt bei 115-118°.

b) In analoger Weise wird das 1-(6-Hydroxyhexyl)-3-methyl-2-(3-pyridyl)-indol, als ein Oel, erhalten. NMR ($CDCl_3$) δ 3,50 (t, 2H), 3,98 (t, 2H).

Beispiel 13

Eine Suspension von 1,52 g 1-(5-Carboxypentyl)-5-chlor-3-methyl-2-(3-pyridyl)-indol in 50 ml Toluol wird bei Raumtemperatur unter Stickstoff mit 0,31 ml Thionylchlorid tropfenweise versetzt. Das erhaltene Gemisch wird 1 Stunde unter Rückfluss gekocht, zusätzlich mit 0,10 g Thionylchlorid versetzt und die Lösung über Nacht bei Raumtemperatur gerührt. Die erhaltene Suspension wird zur Trockene eingedampft. Mane erhält das rohe 1-(5-Chlorcarbonylpentyl)-5-chlor-3-methyl-2-(3-pyridyl)-indol, welches ohne weitere Reinigung direkt verwendet wird.

Eine Suspension von 0,86 g des obigen 1-(5-Chlorcarbonylpentyl)-5-chlor-3-mehyl-2-(3-pyridyl)-indol in 20 ml konzentriertem Ammoniumhydroxid wird über Nacht bei Raumtemperatur gerührt. Filtration der Suspension und Aufschlämmung des erhaltenen festen Materials in Aether ergibt das 1-(5-Carbamoylpentyl)-5-chlor-3-methyl-2-(3-pyridyl)-indol. F. 137-140°.

Beispiel 14

Eine Suspension von 2,9 g (0,06 Mol) 50 %igem Natriumhydrid in Mineralöl, in 40 ml Dimethylformamid, wird unter Stickstoff bei 0-5° mit einer Lösung von 10,4 g 3-Methyl-2-(3-pyridyl)-indol in 60 ml Dimethylformamid innerhalb 20 Minuten tropfenweise versetzt. Das Gemisch wird bei 0 – 5° 30 Minuten gerührt und dann mit 17,6 g (0,06 Mol) 1-Tetrahydropyranyloxy-8-bromoctan in 50 ml Dimethylformamid tropfenweise versetzt. Das Gemisch wird 1 Stunde bei 0 – 10° und 30 Minuten bei Raumtemperatur gerührt in Eiswasser gegossen und mit Aether extrahiert. Der Aetherextrakt wird mit Wasser gewaschen, über Magnesiumsulfat getrocknet und zur Trockene eingedampft. Der Rückstand wird in 100 ml 3-

normaler Chlorwasserstoffsäure gelöst, die erhaltene Lösung 30 Minuten bei Raumtemperatur gehalten, mit Aether gewaschen, mit wässeriger 3-normaler Natriumhydroxidlösung basisch gemacht und mit Methylenchlorid extrahiert. Die Methylenchloridlösung wird zur Trockene eingedampft. Man erhält das 1-(8-Hydroxyoctyl)-2-(3-pyridyl)-3-methylindol.

Beispiel 15

Eine Lösung von 4 g 1-(7-Methoxycarbonylheptyl)-3-methyl-2-(3-pyridyl)-indol in 40 ml n-Butanol wird mit Methylamin gesättigt und ine einem Druckgefäss 3 Tage auf dem Dampfbad erhitz. Das Reaktionsgemisch wird zur Trockene eingedampft und das Produkt aus Essigsaureäthylester umkristallisiert. Man erhält das 1-[7-(N-Methylcarbamoyl)-heptyl]-3-methyl-2-(3-pyridyl)-indol.

Beispiel 16

Herstellung von 10 000 Tabletten mit einem Gehalt von je 10 mg der aktiven Substanz des Beispiels 1 :

Bestandteile :

| | |
|---|---|
| 1-(7-Carboxyheptyl)-3-methyl-2-(3-pyridyl)-indol | 100 g |
| Milchzucker | 1 157 g |
| Maisstärke | 75 g |
| Polyäthylenglykol 6 000 | 75 g |
| Talkpulver | 75 g |
| Magnesiumstearat | 18 g |
| gereinigtes Wasser | q. s. |

Verfahren : Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 17

Herstellung von 10 000 Kapseln mit einem Gehalt von je 25 mg der aktiven Substanz des Beispiels 11a :

Bestandteile :

| | |
|---|---|
| 1-(4-Carboxybenzyl)-3-methyl-2-(3-pyridyl)-indol | 250 g |
| Milchzucker | 1 650 g |
| Talkpulver | 100 g |

Verfahren : Sämtliche Pulver werden mit einem Sieb von 0,6 mm Maschenweite gesiebt. Dann wird der Wirkstoff zuerst mit Talk und nachher mit Milchzucker in einem geeigneten Mischer homogenisiert. Kapseln Nr. 3 werden mit je 200 mg der erhaltenen Mischung in einer Füllmaschine abgefüllt.

In analoger Weise werden Tabletten und Kapseln mit einem Gehalt von je ungefähr 10-100 mg der anderen Verbindungen der Erfindung, z. B. von 1-(5-Carboxypentyl)-5-(chlor, fluor, methoxy oder methyl)-3-methyl-2-(3-pyridyl)-indol, 5-Carboxypentyl)-5,6-dichlor-3-methyl-2-(3-pyridyl)-indol, oder von anderen in den Beispielen genannten Verbindungen, hergestellt.

Beispiel 18

Eine Lösung von 50 mg 1-(5-Carbamoylpentyl)-5-chlor-3-methyl-2-(3-pyridyl)-indol in 1 ml 6-normaler Chlorwasserstoffsäure wird 3 Stunden unter Rückfluss gekocht. Nach Abkühlen fällt das Hydrochloridsalz aus. Die Suspension wird zur Trockene eingedampft und der Rückstand mit gesättigter wässeriger Natriumhydrogencarbonatlösung basischgemacht. Diese Lösung wird mit Aether gewaschen und mit 2-normaler Chlorwasserstoffsäure auf den pH-Wert 6-7 eingestellt. Man erhält die rohe freie Säure, nämlich das 1-(5-Carboxypentyl)-5-chlor-3-methyl-2-(3-pyridyl)-indol. F. 137-141°.

Beispiel 19

Ein Gemisch von 4,17 g 3-Methyl-2-(3-pyridyl)-indol, 0,64 g Tetra-n-butyl-ammoniumbromid und

1,02 g pulverisiertem Kaliumhydroxid in 500 ml Acetonitril wird unter Rühren bei Raumtemperatur, unter Stickstoff, mit 5,06 g p-(2-Bromäthoxy)-benzoesäure [hergestellt gemäss USP 2,790,825 (1957)] versetzt. Die Suspension wird 5 Tage gerührt. Das Kaliumbromid wird abfiltriert, das Filtrat zu einem Oel konzentriert. Dieses wird in Essigsäureäthylester gelöst und mit 3-normaler Chlorwasserstoffsäure extrahiert. Die Säureschicht wird abgetrennt und mit 3-normaler Natriumhydroxidlösung behandelt. Die erhaltene Suspension wird mit Essigsäureäthylester (3 × 100 ml) extrahiert und der organische Extrakt abgetrennt. Dieser wird über Magnesiumsulfat getrocknet und konzentriert. Man erhält das 1-[2-(4-Aethoxycarbonylphenoxy)-äthyl]-2-(3-pyridyl)-3-methylindol als ein Oel.

Beispiel 20

Ein Gemisch von 4,7 g 1-[2-(4-Aethoxycarbonylphenoxy)-äthyl]-2-(3-pyridyl)-3-methylindol in 2-normaler Chlorwasserstoffsäure 220 ml wird 6 Stunden unter Rückfluss gekocht. Nach Abkühlen wird die Lösung mit 3-normaler Natriumhydroxidlösung basischgemacht und mit Essigsäureäthylester extrahiert. Die basische Lösung wird filtriert und mit 5-normaler Chlorwasserstoffsäure auf den pH-Wert 6-7 eingestellt. Das feste Material wird abgetrennt, getrocknet und aus Aceton umkristallisiert. Man erhält das 1-[2-(4-Carboxyphenoxy)-äthyl]-2-(3-pyridyl)-3-methylindol. F. 190-193°.

Beispiel 21

Eine Lösung von 5,9 g p-Mercapto-benzoesäure-äthylester (hergestellt gemäss J. Chem. Soc., *1963,* 1947-1954) in 30 ml Dimethylformamid wird tropfenweise zu einem Brei von 1,55 g 50 %igem Natriumhydrid-Mineralöl in 30 ml Dimethylformamid gegeben. Dieses Gemisch wird unter Stickstoff bei Raumtemperatur 30 Minuten gerührt. Diese Lösung wird tropfenweise zu einer Lösung von 9,78 g 1-(2-Methylsulfonyloxy-äthyl)-2-(3-pyridyl)-3-methylindol in 60 ml Dimethylformamid bei − 10° gegeben. Dieses Gemisch wird bei Raumtemperatur über Nacht gerührt und in 1 000 ml Eiswasser gegossen. Das Gemisch wird mehrmals mit Aether (total ungefähr 1 000 ml) extrahiert.

Der Aetherextrakt wird mit Wasser (3 × 200 ml) gewaschen, über Magnesiumsulfat getrocknet und im Vakuum eingedampft. Man erhält das 1-[2-(4-Aethoxycarbonyl-phenylthio)-äthyl]-2-(3-pyridyl)-3-methylindol als ein Oel. NMR ($CDCl_3$) bestätigt die Struktur.

Der Ausgangsstoff wird wie folgt hergestellt : Man versetzt 11,77 g 1-(2-Aethoxycarbonyl-methyl)-2-(3-pyridyl)-3-methylindol in 400 ml trockenem Tetrahydrofuran bei 0° mit 60 ml einer 1-molaren Lösung von Lithiumaluminiumhydrid in Tetrahydrofuran. Das Reaktionsgemisch wird 1 Stunde bei Raumtemperatur gerührt, dann in einem Eisbad gekühlt und nacheinander mit 2,26 ml Wasser, 2,26 ml einer 15 %igen Natriumhydroxidlösung und mit 6,78 ml Wasser zersetzt. Das Gemisch wird filtriert und im Vakuum konzentriert. Der Rückstand wird in Aether gelöst, mit gesättigter Natriumhydrogencarbonatlösung gewaschen, über Magnesiumsulfat getrocknet und im Vakuum konzentriert. Man erhält das halbfeste 1-(2-Hydroxyäthyl)-2-(3-pyridyl)-3-methylindol, welches im nächsten Schritt direkt verwendet wird.

Man gibt tropfenweise, bei − 10°, Methansulfonylchlorid (2,70 ml) zu einer Lösung von 7,5 g 1-(2-Hydroxyäthyl)-2-(3-pyridyl)-3-methylindol und 10,34 ml Triäthylamin in 150 ml Methylenchlorid. Das Gemisch wird bei Raumtemperatur 30 Minuten gerührt und in 600 ml Eiswasser gegossen. Der erhaltene Brei wird mit Methylenchlorid extrahiert, der Extrakt mit gesättigter Natriumhydrogencarbonatlösung gewaschen, über Magnesiumsulfat getrocknet und im Vakuum eingedampft. Man erhält das 1-(2-Methylsulfonyloxy-äthyl)-2-(3-pyridyl)-3-methylindol, welches in der obigen Reaktion direkt verwendet wird.

Beispiel 22

Ein Gemisch von 6,39 g 1-[2-(4-äthoxycarbonyl-phenylthio)-äthyl]-2-(3-pyridyl)-3-methylindol in 260 ml 2-normaler Chlorwasserstoffsäure wird 6 Stunden unter Rückfluss gekocht. Nach Abkühlen wird der pH-Wert mit gesättigter Natriumhydrogencarbonatlösung (ungefähr 500 ml) auf 6-7 gestellt. Das Gemisch wird mit 200 ml Aether versetzt und 30 Minuten gerührt. Das feste Material wird abgetrennt, zuerst mit Wasser, dann mit Aether gewaschen und in 100 ml absolutem Aethanol gelöst. Die Lösung wird filtriert, und die noch heisse Lösung mit 1,68 ml 6,5-normalem äthanolischem Chlorwasserstoff behandelt. Die Lösung wird gekühlt und mit ungefähr 100 ml Aether verdünnt. Das erhaltene Produkt wird abgetrennt. Man erhält das 1-[2-(4-Carboxyphenylthio)-äthyl]-2-(3-pyridyl)-3-methylindolhydrochlorid. F. 222-224°.

Beispiel 23

Eine Lösung von Lithiumdiisopropylamid (LDA) wird durch Zugabe von n-Butyl-lithium (7,66 mMol, 1,6 Mol in Hexan) zu einer Lösung von Diisopropylamin (7,6 mMol) in Tetrahydrofuran (THF, 12 ml) bei − 20° hergestellt. Die LDA-Lösung wird auf − 78° gekühlt und tropfenweise mit 1-(5-Methoxycarbonylpentyl)-2-(3-pyridyl)-3-methyl-indol (2,48 g) in THF (24 ml) innerhalb 5 Minuten versetzt. Das Gemisch wird bei − 78° 20 Minuten gerührt und dann mit Phenylselenylchlorid (1,5 g) in THF (12 ml) versetzt. Nach

5 Minuten entfernt man das Kühlbad und lässt das Gemisch sich auf 0° erwärmen. Es wird gesättigte wässerige Natriumhydrogencarbonatlösung (60 ml) dazu gegeben und dann mit Aether (3 × 50 ml) extrahiert. Die vereinigten organischen Extrakte werden mit gesättigter wässeriger Natriumhydrogencarbonatlösung, gesättigter wässeriger Natriumchloridlösung gewaschen und über wasserfreiem Magnesiumsulfat getrocknet. Konzentrieren im Vakuum ergibt das rohe 1-(5-Methoxycarbonyl-5-phenylselenylpentyl-2-(3-pyridyl)-3-methylindol als ein gelbes Oel. Das rohe Selenid wird in Dichlormethan gelöst (40 ml) und tropfenweise mit 30 %igem Wasserstoffsuperoxid (1,8 g, 16 mMol) in Wasser (1,8 ml) versetzt. Nach der Zugabe von ungefähr 10 % des Wasserstoffsuperoxids beginnt eine exoterme Reaktion. Bis zur Beendigung der Zugabe steigt die Temperatur auf 30°. Das Gemisch wird weitere 30 Minuten gerührt und dann mit 5 %iger wässeriger Natriumcarbonatlösung (40 ml) versetzt. Die Dichlormethanschicht wird abgetrennt. Die wässerige Phase wird mit Dichlormethan (25 ml) extrahiert. Die vereinigten organischen Phasen werden mit 5 %igem wässerigem Natriumcarbonat, Wasser, gesättigtem wässerigem Natriumchlorid gewaschen und über wasserfreiem Magnesiumsulfat getrocknet. Konzentrieren im Vakuum ergibt das 1-(5-Methoxycarbonyl-pent-4-enyl)-2-(3-pyridyl)-3-methylindol als hellgelbes Oel. Die weitere Reinigung wird durch Kurzweg-Chromatographie (Silicagel) mit Essigsäureäthylester-Hexan (2 : 3) als Eluent durchgeführt. NMR ($COCl_3$) δ 5,53 (d, 1H) ; 6,65 (m, 1H) ; IR (flüssig) 1 720 $cm^{-1}$.

Beispiel 24

Eine Lösung des α,β-ungesättigten Esters, nämlich des 1-(5-Methoxycarbonylpent-4-enyl)-2-(3-pyridyl)-3-methylindols (84 mg) in Methanol (1 ml) wird mit 1-normaler wässeriger Lithiumhydroxidlösung (1 ml) versetzt. Das Gemisch wird über Nacht bei Raumtemperatur gerührt und dann im Vakuum zur Trockene eingedampft. Der Rückstand wird in Wasser (2 ml) gelöst und mit Diäthyläther (5 ml) gewaschen. Die wässerige Phase wird auf den pH-Wert 6,6-7,0 angesäuert und mit Dichlormethan extrahiert. Der organische Extrakt wird mit gesättigter wässeriger Natriumchloridlösung gewaschen, über Magnesiumsulfat getrocknet und im Vakuum eingedampft. Man erhält ein blassgelbes Oel, welches sich nach Triturieren mit Chloroform verfestigt. Man erhält das 1-(5-Carboxypent-4-enyl)-2-(3-pyridyl)-3-methylindol. F. 145-147°.

Beispiel 25

Eine Lösung von Collins-Reagens, welches unter Stickstoff bei 0-5° aus Chromtrioxid (5,6 mg) und Pyridin (8,86 g, 112 mMol) in Dichlormethan (150 ml) hergestellt ist, wird auf einmal mit 1,8 g 1-(6-Hydroxyhexyl)-3-methyl-2-(3-pyridyl)-indol in Dichlormethan (15 ml) versetzt. Das Gemisch wird 25 Minuten gerührt und durch Celite filtriert. Das Filtrat wird dann auf eine Silicagelsäule gebracht und mit einem 1 : 1-Gemisch von Essigsäureäthylester-Dichlormethan (500 ml) eluiert. Konzentrieren im Vakuum ergibt das 1-(5-Formylpentyl)-2-(3-pyridyl)-3-methylindol als blassgelbes Oel. NMR ($CDCl_3$) δ 9,7 (t, 1H) ; IR (flüssig) 2 710, 1 720 $cm^{-1}$.

Beispiel 26

Man gibt tropfenweise unter Stickstoff, Trimethylphosphonoacetat (328 mg) zu einer Lösung von Kalium-tert.butoxid (220 mg) in THF (5 ml) bei 0°. Die Lösung wird 20 Minuten bei 0° gerührt, dann auf − 78° gekühlt und mit einer Lösung von 1-(5-Formylpentyl)-2-(3-pyridyl)-3-methylindol (450 mg) in THF (5 ml) tropfenweise versetzt. Das Gemisch wird 15 Minuten bei − 78° gehalten und die Kühlung eingestellt. Das Gemisch wird über Nacht bei Raumtemperatur gerührt, mit Wasser (25 ml) verdünnt und mit Diäthyläther (3 × 25 ml) extrahiert. Die vereinigten Extrakte werden mit gesättigter wässeriger Natriumhydrogencarbonatlösung und gesättigter wässeriger Natriumchloridlösung gewaschen und über Magnesiumsulfat getrocknet. Nach Eindampfen in Vakuum erhält man das 1-(7-Methoxycarbonylhept-6-enyl)-2-(3-pyridyl)-3-methylindol als blassgelbes Oel. IR (flüssig) 1 735 $cm^{-1}$.

Beispiel 27

Hydrolyse von 50 mg 1-(7-Methoxycarbonylhept-6-enyl)-2-(3-pyridyl)-3-methylindol gemäss dem Verfahren des Beispiels 24 ergibt das 1-(7-Carboxyhept-6-enyl)-2-(3-pyridyl)-3-methylindol. F. 144-146 (umkristallisiert aus Dichlormethan-Hexan).

Beispiel 28

1-(7-Carboxyhept-6-enyl)-2-3-(pyridyl)-3-methylindol (10 mg) wird in 1 ml absolutem Aethanol mit einer katalytischen Menge von 10 %igem Palladium-auf-Kohle bei einem Druck von 1 Atmosphäre hydriert. Nach 3,5 Stunden wird der Katalysator abfiltriert und mit einigen Milliliter Aethanol gewaschen. Die vereinigten organischen Extrakte werden im Vakuum eingedampft. Man erhält ein farbloses Oel, welches dann auskristallisiert. Man erhält das 1-(7-Carboxyheptyl)-3-methyl-2-(3-pyridyl)-indol des Beispiels 1. Das rohe Produkt schmilzt bei 110-113°.

## Beispiel 29

1-(4-Cyanbutyl)-3-methyl-2-(3-pyridyl)-indol (578 mg) wird bei 185° 30 Minuten mit 450 mg pulverisiertem Natriumhydroxid und 5 ml Aethylenglykol erhitzt. Das Gemisch wird in 50 ml Wasser gegossen, mit Aether gewaschen und der pH-Wert mit 2-normaler Chlorwasserstoffsäure auf 6 eingestellt. Das erhaltene Oel kristallisiert aus. Man erhält das 1-(4-Carboxybutyl)-3-methyl-2-(3-pyridyl)-indol des Beispiels 5. (F. 127-129°).

Der Ausgangsstoff wird wie folgt hergestellt : Eine Lösung von 3-Methyl-2-(3-pyridyl)-indol (2,09 g) in 12 ml DMF wird zu einer Suspension von 50 %igem Natriumhydrid-Mineralöl in 6 ml DMF bei 0° gegeben. Das Gemisch wird 30 Minuten bei 0° gerührt und mit einer Lösung von 1,78 g 5-Bromvaleronitril in 4 ml DMF behandelt. Das Gemisch wird bei Raumtemperatur über Nacht gerührt, in 125 ml Wasser gegossen und mit 2 × 50 ml Aether extrahiert. Der Extrakt wird mit 3 × 20 ml Wasser gewaschen und über Magnesiumsulfat getrocknet. Man erhält das 1-(4-Cyanbutyl)-3-methyl-2-(3-pyridyl)-indol als ein Oel.

## Beispiel 30

Ein Gemisch von 578 mg 1-(4-Cyanbutyl)-3-methyl-2-(3-pyridyl)-indol, 173 mg Natriumazid, 142 mg Ammoniumchlorid und 5 mg Lithiumchlorid in 2 ml DMF wird über Nacht bei 120° erhitzt. Nach Abkühlen wird das Gemisch filtriert und das Filtrat mit ungefähr 25 ml Wasser verdünnt. Der pH-Wert wird mit 3-normaler Natriumhydroxidlösung auf 10-11 gestellt, und die Lösung, zwecks Beseitigung des nicht-reagierten Nitrils mit Aether gewaschen. Der pH-Wert des wässerigen Phase wird mit 2-normaler Chlorwasserstoffsäure auf 5-6 gestellt und mit Aether extrahiert. Der Aetherextrakt wird mit Wasser gewaschen, über Magnesiumsulfat getrocknet und im Vakuum eingedampft. Der feste Rückstand wird in Petroläther aufgeschlämmt und abgetrennt. Man erhält das 1-[4-(5-Tetrazolyl)-butyl]-3-methyl-2-(3-pyridyl)-indol. F. 177-179°.

## Beispiel 31

Eine Lösung von 3-Methyl-2-(3-pyridyl)-indol (2,08 g) in 12 ml DMF wird unter Stickstoff, bei 10-15°, zu einer Suspension von 0,528 g 50 %igem Natriumhydrid-Mineralöl in 6 ml DMF gegeben. Nach der Beendigung der Zugabe wird das Gemisch bei Raumtemperatur 30 Minuten gerührt und mit 2,39 g 3-(p-Chlormethyl-phenyl)-2-methyl-acrylsäure-äthylester in 5 ml DMF tropfenweise behandelt. Das erhaltene Gemisch wird über Nacht bei Raumtemperatur gerührt und in 100 ml Wasser gegossen. Das erhaltene Gemisch wird mit Essigsäureäthylester (2 × 50 ml) extrahiert. Die organische Schicht wird mit 100 ml gesättigter wässeriger Natriumchloridlösung gewaschen, über Magnesiumsulfat getrocknet und eingedampft. Man erhält das 1-[p-(2-Aethoxycarbonylpropen-1-yl)-benzyl]-3-methyl-2-(3-pyridyl)-indol.

Die Hydrolyse mit 2-normaler wässeriger Chlorwasserstoffsäure ergibt das 1-[p-(2-Carboxypropen-1-yl)-benzyl]-3-methyl-2-(3-pyridyl)-indol.

Der Ausgangsstoff wird wie folgt hergestellt: Eine Suspension von 10.0 g eines 50 %igen Natriumhydrids in Mineralöl in frisch destilliertem Dimethoxyäthan (DME, 350 ml) wird unter Stickstoff bei 10° gerührt und innerhalb ungefähr 40 Minuten mit Triäthyl-2-phosphonopropionat versetzt. Das Gemisch wird 30 Minuten bei 10° gerührt. Unter Rühren für 1,5 weitere Stunden lässt man die Temperatur auf Raumtemperatur steigen. Diese Lösung wird unter Stickstoff mit einer Kanüle in ein 500 ml Tropftrichter gebracht und tropfenweise zu einer Lösung von Terephthalaldehyd (33,53 g) in trockenem DME (475 ml) innerhalb 1 Stunde bei 22-34° gegeben. Nach der Beendigung der Zugabe wird das Reaktionsgemisch bei Raumtemperatur 2 Stunden mechanisch gerührt, in 100 ml Wasser gegossen und mit 4 × 500 ml Aether extrahiert. Der Aetherextrakt wird mit gesättigter wässeriger Natriumchloridlösung (700 ml gewaschen), über Magnesiumsulfat getrocknet, filtriert und im Vakuum konzentriert. Man erhält ein gelbes Oel, welches nach Stehenlassen teilweise kristallisiert. Das rohe Gemisch wird durch Suspendieren in Petroläther-Essigsäureäthylester (93 : 7) gereinigt. Das Filtrat wird, nach der Beseitigung des nicht-reagierten Dialdehyds, im Vakuum konzentriert. Man erhält ein Gemisch, das durch Hochdruck-Flüssigkeitschromatographie (wobei man Petroläther/Essigsäureäthylester 93 : 7 verwendet) weiter gereinigt wird. Man erhält den reinen 4-Formyl-α-methylcinnamonsäure-äthylester. Eine Lösung dieses Aldehyds (34,80 g) in 820 ml absolutem Aethanol wird mit 12,11 g granuliertem Natriumborhydrid bei Raumtemperatur unter Stickstoff behandelt. Das erhaltene Gemisch wird 3 Stunden bei Raumtemperatur gerührt (bzw. bis sich das Borhydrid auflöst), auf ein Volumen von ungefähr 200 ml konzentriert, mit 400 ml Wasser verdünnt und 3 × 200 ml Aether extrahiert. Der Aetherextrakt wird mit 100 ml Wasser und gesättigter wässeriger Natriumchloridlösung gewaschen, über Magnesiumsulfat getrocknet, filtriert und das Filtrat im Vakuum konzentriert. Man erhält den 3-(p-Hydroxymethylphenyl)-2-methyl-acrylsäure-äthylester. Zu einer Lösung dieses Produkts in 350 ml Methylenchlorid gibt man bei Raumtemperatur 11,53 ml Thionylchlorid, tropfenweise, innerhalb 25 Minuten. Die klare, farblose Lösung wird 2 Stunden gerührt. Die Lösung wird mit 100 ml Wasser, 200 ml gesättigter wässeriger Natriumhydrocarbonatlösung, 100 ml Wasser und 100 ml gesättigter wässeriger Natriumchloridlösung gewaschen. Die organische Schicht wird getrocknet und eingedampft. Man erhält den 3-(p-Chlormethyl-phenyl)-2-methyl-acylsäure-äthylester, welcher ohne weitere Reinigung verwendet wird.

Beispiel 32

1-(5-Formylpentyl)-3-methyl-2-(3-pyridyl)-indol (127 mg) wird in DMF (0,66 ml) gelöst und auf einmal mit Pyridiniumdichromat (298 mg) versetzt. Das Gemisch wird über Nacht bei Raumtemperatur gerührt, dann mit einem Gemisch von Aether und Essigsäureäthylester (25 ml ; 4 : 1) verdünnt und filtriert. Das feste Material wird mit heissem Chlorofom gewaschen und die vereinigten Filtrate werden im Vakuum konzentriert. Man erhält ein dunkelbraunes gummiartiges Material, welches mit Aether : Essigester (4 : 1) aufgeschlämmt und mit 0,1-normaler wasseriger Natriumhydroxidlösung (2 ml) extrahiert wird. Der wässerige Extrakt wird auf den pH-Wert 5,5-6,0 eingestellt und mit Chloroform extrahiert. Der Chloroformextrakt wird getrocknet und im Vakuum konzentriert. Man erhält ein gelbes Oel. Die Dünnschichtchromatographie (Silicagel ; Essigsäureäthylester : Hexan, 1 : 1) zeigt die Gegenwart der gewünschten Säure. Weitere Chromatographie auf Silicagel unter Verwendung von Essigsäureäthylester : Hexan (1 : 1) als Eluent, ergibt das gewünschte 1-(5-Carboxypentyl)-3-methyl-2-(3-pyridyl)-indol des Beispiels 3.

Beispiel 33

Man gibt unter Eisbad-Kühlung Brom (0,344 ml) zu einer Lösung von 692 mg Natriumhydroxid in 4 ml Wasser. Die erhaltene Lösung wird zu 400 mg 1-(5-Oxohexyl)-3-methyl-2-(3-pyridyl)-indol gegeben und das Gemisch 2 Stunden bei Raumtemperatur gerührt. Das Gemisch wird mit Aether gewaschen. Die wässerige Lösung wird filtriert und mit 2-normaler Chlorwasserstoffsäure auf den pH-Wert 5-6 eingestellt. Man trennt ein weisses festes Rohprodukt ab, welches in einem Bereich von 108-120° schmilzt. Die Trennung durch Dünnschichtchromatographie (Silicagel ; Methylenchlorid-Methanol, 9 : 1) ergibt das 1-(4-Carboxybutyl)-3-methyl-2-(3-pyridyl)-indol des Beispiels 5.

Der Ausgangsstoff wird wie folgt hergestellt : 1-(4-Cyanbutyl)-3-methyl-2-(3-pyridyl)-indol (1,5 g) in 15 ml Aether wird zu einer Lösung von 0,0103 Mol Methylmagnesium-bromid in 15 ml Aether gegeben. Das Gemisch wird 3 Stunden unter Rückfluss gekocht. Nach Abkühlen gibt man tropfenweise 10 ml 6-normaler Chlorwasserstoffsäure dazu und erhitzt das Gemisch mehrere Stunden unter Rückfluss. Das Reaktionsgemisch wird mit Aether gewaschen und mit 3-normaler Natriumhydroxidlösung auf den pH-Wert 10-11 eingestellt. Nach Extraktion mit Aether und Eindampfen der Lösungsmittels erhält man das 1-(5-Oxohexyl)-3-methyl-2-(3-pyridyl)-indol. IR 1 720 $cm^{-1}$ ; NMR ($CDCl_3$) δ 2,0.

Beispiel 34

1-(7-Carboxyheptyl)-5-chlor-3-methyl-2-(3-pyridyl)-indolhydrochlorid (421 mg) gelöst in 7 ml Tetrahydrofuran wird erwärmt und mit 202 mg (0,278 ml) Triäthylamin behandelt. Diese Lösung wird tropfenweise zu einer Lösung von 108 mg (0,096 ml) Chlorameisensäureäthylester in 1 ml Tetrahydrofuran gegeben und auf 0-5° gekühlt. Das Reaktionsgemisch wird 1 Stunde bei dieser Temperatur gerührt und zwecks Beseitigung des Triäthylamin-hydrochlorids, filtriert. Das Filtrat wird mit einer Lösung von Hydroxylamin-hydrochlorid (69 mg) und Natriumhydroxid (40 mg) in 10 ml Methanol behandelt. Dieses Gemisch wird 0,5 Stunden gerührt und im Vakuum konzentriert. Der Rückstand wird mit 25 ml Aether-Methanol (10 : 1) behandelt und filtriert. Das Filtrat wird im Vakuum eingedampft. Man erhält ein dickes Oel, welches in Aceton gelöst und mit 6,5-normalem äthanolischem Chlorwasserstoff behandelt. Man erhält das 1-(7-Hydroxycarbamoyl-heptyl)-5-chlor-3-methyl-2-(3-pyridyl)-indol-hydrochlorid. F. 170-173°.

Beispiel 35

Aethanolischer Chlorwasserstoff (7,1-normal ; 0,14 ml) wird zu 236 mg N-Phenyl-N-(5-methoxycarbonylpentyl)-hydrazin in 2 ml absolutem Aethanol gegeben und dann mit 135 mg 3-Propionyl-pyridin versetzt. Das Gemisch wird über Nacht unter Rückfluss gekocht. Dann wird es mit weiterem äthanolischem Chlorwasserstoff (0,62 ml) versetzt und weitere 24 Stunden unter Rückfluss erhitzt. Nach Abkühlen wird das Gemisch filtriert und das Filtrat im Vakuum eingedampft. Der Rückstand wird in 10 ml Wasser gerührt und mit 1-normaler Natriumhydroxidlösung auf den pH-Wert 10-11 gestellt. Dieses Gemisch wird mit Aether extrahiert. Der Extrakt wird mit Wasser gewaschen, über Magnesiumsulfat getrocknet und eingedampft. Man erhält ein Oel, welches das 1-(5-Aethoxycarbonylpentyl)-3-methyl-2-(3-pyridyl)-indol ist.

Dieser Ester wird mit 10 ml 2-normaler Chlorwasserstoffsäure unter Rückfluss hydrolysiert, dann wird der pH-Wert mit gesättigter wässeriger Natriumhydrogencarbonatlösung auf ungefähr 6 eingestellt und mit Aether extrahiert. Die Aufarbeitung des organischen Extrakts ergibt das 1-(5-Carboxypentyl)-3-methyl-2-(3-pyridyl)-indol des Beispiels 3 (Schmelzpunkt des Rohproduktes 111-113°).

Der Ausgangsstoff wird wie folgt hergestellt : Anilin (2,79 g ; 2,73 ml), 6,27 g 6-Bromhexansäuremethylester und 12,24 g (0,09 Mol) Natrium-acetat-trihydrat werden in 15 ml absolutem Aethanol über Nacht bei 80-100° erhitzt. Nach Abkühlen wird das Gemisch in 75 ml Eiswasser gegossen und mit Aether extrahiert. Der organische Extrakt wird mit Wasser gewaschen, über Magnesiumsulfat getrocknet und im Vakuum eingedampft. Man erhält das N-(5-Methoxycarbonylpentyl)-anilin.

Eine Lösung von 1,4 g Natriumnitrit in 5 ml Wasser wird tropfenweise, bei 0-10°, zu einem Gemisch von 4,42 g N-(5-Methoxycarbonylpentyl)-anilin, 2,9 ml konzentrierter Chlorwasserstoffsäure und Eis (das zur Kühlung auf die genannte Temperatur notwendig ist) gegeben. Das Gemisch wird dann eine Stunde bei Raumtemperatur gerührt und mit Aether extrahiert. Der Extrakt wird mit Wasser gewaschen, über Magnesiumsulfat getrocknet und im Vakuum eingedampft. Man erhält das N-Nitroso-N-(5-methoxycarbonylpentyl)-anilin als ein Oel.

Das obige Nitroso-Derivat (3,6 g) in 4 ml Eisessig wird tropfenweise zu 3,94 g Zinkpulver in 6 ml Wasser gegeben. Nach der exothermen Reaktion bis zu 35° wird das Gemisch 2 Stunden bei Raumtemperatur gerührt. Das Zink wird abfiltriert, das Filtrat mit Aether gewaschen mit 40 %iger Natriumhydroxidlösung der pH-Wert auf 10-11 eingestellt und mit Aether extrahiert. Der Extrakt wird über Magnesiumsulfat getrocknet und im Vakuum eingedampft. Man erhält ein rohes Oel. Nach einer Kurzweg-Chromatographie auf Silicagel mit Hexan-Essigsäure (5 : 1), erhält man das N-Phenyl-N-(5-methoxycarbonylpentyl)-hydrazin von ungefähr 80 % Reinheit. Dieses Produkt wird in der oben beschriebenen Fischer-Cyclisierung direkt verwendet.

## Beispiel 36

Man löst 1-[7,7-(Bis-methoxycarbonyl)-heptyl]-3-methyl-2-(3-pyridyl)-indol (273 mg) in Methanol (0,5 ml) und versetzt es mit 1-normaler wässeriger Lithiumhydroxidlösung (1,95 ml). Das Gemisch wird 1 Stunde bei Raumtemperatur gerührt und dann 2,5 Stunden unter Rückfluss gekocht. Die Klare Lösung wird zur Trockene eingedampft, der Rückstand in Wasser gelöst und der pH-Wert auf 6-6,2 eingestellt. Es entsteht ein gelbes, gummiartiges, festes Material, das in Chloroform extrahiert wird. Der Extrakt wird über Magnesiumsulfat getrocknet und eingedampft. Man erhält das rohe 1-[7,7-(Bis-carboxy)-heptyl]-3-methyl-2-(3-pyridyl)-indol. NMR ($CDCl_3$) δ 10,60 (2H).

Eine Probe der rohen Dicarbonsäure (28 mg) wird in p-Xylol (3 ml), welches 0,1-normale Chlorwasserstoffsäure (0,1 ml) enthält, 30 Minuten erhitzt. Man lässt die klare Lösung sich auf Raumtemperatur abkühlen. Es fällt ein gummiartiges Material aus, welches in Natriumhydroxid extrahiert wird. Die wässerige Phase wird abgetrennt, ihr pH-Wert auf 6-6,2 eingestellt und mit Essigsäureäthylester : Aether (8 : 2) extrahiert. Die organische Phase wird über Magnesiumsulfat getrocknet und eingedampft. Man erhält ein farbloses Oel, welches sich nach Stehenlassen verfestigt. Man erhält das 1-(7-Carboxyheptyl)-3-methyl-2-(3-pyridyl)-indol, welches gemäss seinen NMR- und TLC-Werten mit der Verbindung des Beispiels 1 identisch ist.

Der Ausgangsstoff wird wie folgt hergestellt : Man gibt bei 0° Thionylchlorid (0,36 ml) zu 1-(6-Hydroxyhexyl)-3-methyl-2-(3-pyridyl)-indol (1,37 g) und rührt das Gemisch 1 Stunde bei Raumtemperatur. Dann wird das Gemisch mit gesättigter wässeriger Natriumhydrogencarbonatlösung versetzt und mit Dichlormethan extrahiert. Der Extrakt wird mit gesättigter wässeriger Natriumchloridlösung gewaschen und über Magnesiumsulfat getrocknet. Nach Eindampfen im Vakuum erhält man das rohe Chlorid als ein Oel. Reinigung durch Silicagel-Chromatographie (Methylenchlorid/Essigsäureäthylester 19 : 1) ergibt das 1-(6-Chlorhexyl)-3-methyl-2-(3-pyridyl)-indol als hellgelbes Oel. NMR ($CDCl_3$) δ 3,30 (t, 2H), 3,92 (t, 2H).

Das 1-(6-Chlorhexyl)-3-methyl-2-(3-pyridyl)-indol (0,5 g) wird zu einem Gemisch von Dimethylmalonat (792 mg), Kaliumcarbonat (790 mg) und Dimethylformamid (11,6 ml) gegeben und unter Stickstoff 18 Stunden bei 80-90° erhitzt. Das Gemisch wird in Eiswasser (80 ml) gegossen, mit 1-normaler Chlorwasserstoffsäure angesäuert und mit Aether gewaschen. Die wässerige Schicht wird auf den pH-Wert 6 eingestellt und mit Aether extrahiert. Der Extrakt wird über Magnesiumsulfat getrocknet und zu einem gelben Oel eingedampft. Reinigung durch präparative Dünnschichtchromatographie (Chloroform-Essigsäureäthylester 9 : 1) ergibt das 1-[7,7-(Bis-methoxycarbonyl)-heptyl]-3-methyl-2-(3-pyridyl)-indol. NMR ($CDCl_3$), δ 3,32 (t, 1H), 3,78 (s, 6H), 4,03 (t, 2H). IR (flüssig) 1 750 $cm^{-1}$.

## Beispiel 37

1-(6-Chlorhexyl)-3-methyl-2-(3-pyridyl)-indol (165 mg) in trockenem THF (2 ml) wird tropfenweise zu Magnesiumspänen (12 mg) in trockenem THF (2 ml) unter Stickstoff gegeben. Zur Einleitung der Reaktion wird während der Zugabe ein Jodkristall hinzugesetzt. Das Gemisch wird nach der Beendigung der Vermischung 4 Stunden unter Rückfluss gekocht, auf 0° gekühlt. Unter Rühren wird 15 Minuten trockenes Kohlendioxidgas in das Reaktionsgefäss eingeleitet. Das trübe Gemisch wird in 5 ml 1-normaler Natriumhydroxidlösung gegossen und mit Aether extrahiert. Die wässerige Phase wird auf den pH-Wert 6-6,2 eingestellt und mit Essigsäureäthylester extrahier. Die organische Phase wird über Magnesiumsulfat getrocknet und im Vakuum zur Trockne eingedampft. Das Rohprodukt schmilzt bei 106-107°. Das Produkt ist das 1-(6-Carboxyhexyl)-3-methyl-2-(3-pyridyl)-indol, dessen TLC- und NMR-Werte mit der Verbindung des Beispiels 4 identisch sind.

## Beispiel 38

1-(Prop-2-ynyl)-3-methyl-2-(3-pyridyl)-indol (90 mg) wird unter Stickstoff in THF (2 ml) gelöst und die erhaltene Lösung au − 78° gekühlt. Eine Lösung von n-Butyl-lithium (0,024 ml, 1,6 Mol in Hexan) wird

tropfenweise, innerhalb 1 Minute, dazu gegeben. Das orangefarbene Gemisch wird weitere 10 Minuten bei − 78° gerührt, dann mit Chlorameisensäure-methylester (0,031 ml) versetzt. Man lässt das Gemisch sich auf Raumtemperatur zu erwärmen, giesst es in gesättigte wässerige Natriumchloridlösung und extrahiert es mit Aether. Der Extrakt wird mit Wasser gewaschen und über Magnesiumsulfat getrocknet. Eindampfen im Vakuum ergibt ein Oel, welches durch präparative Dünnschichtchromatographie gereinigt wird. Als Solvent verwendet man ein 1 : 1-Gemisch von Essigsäureäthylester : Hexan. Das 1-(3-Methoxycarbonyl-prop-2-ynyl)-3-methyl-2-(3-pyridyl)-indol wird als Oel isoliert. NMR ($CDCl_3$) δ 3,73 (s, 3H), 4,83 (s, 2H) ; IR ($CHCl_3$) 1 715, 2 245 $cm^{-1}$.

Der Ausgangsstoff wird als folgt hergestellt : Natriumhydrid (50 %ige Mineralöl-Dispersion ; 53 mg) wird unter Stickstoff mit Petroläther gewaschen. Das gewaschene Natriumhydrid wird in trockenem DMF (2 ml) suspendiert und tropfenweise mit 3-Methyl-2-(3-pyridyl)-indol (208 mg) in DMF (2 ml) versetzt. Das Gemisch wird weitere 30 Minuten gerührt und dann tropfenweise mit Propargylbromid versetzt. Das Reaktionsgemisch wird weitere 2 Stunden gerührt, in Eiswasser gegossen, mit 1-normaler Chlorwasserstoffsäure angesäuert und mit Aether extrahiert. Die wässerige Phase wird mit Natriumhydrogencarbonat basisch gemacht und mit Aether extrahiert. Der Aetherextrakt wird mit Wasser und gesättigter wässeriger Natriumchloridlösung gewaschen und über Magnesiumsulfat getrocknet. Eindampfen im Vakuum ergibt das 1-(Prop-2-ynyl)-3-methyl-2-(3-pyridyl)-indol ; NMR ($CDCl_3$) δ 2,20 (s, 4H), 4,70 (d, 2H, J = 3 Hz) ; IR (flüssig) 3200, 2 120 $cm^{-1}$. Schmelzpunkt 104-105° nach Reinigung durch Kurzweg-Chromatographie, unter Verwendung von einem 1 : 1-Gemisch von Essigsäureäthylester : Hexan.

Beispiel 39

Die Behandlung von 33 mg 1-(3-Methoxycarbonylprop-2-ynyl-3-methyl-2-(3-pyridyl)-indol in 1 ml Methanol mit 0,3 ml 1-normaler Lithiumhydroxidlösung bei Raumtemperatur ergibt das 1-(3-Carboxyprop-2-ynyl)-3-methyl-2-(3-pyridyl)-indol. IR 1 720 $cm^{-1}$.

Beispiel 40

Analog zu den in den vorhergehenden Beispielen beschriebenen Verfahren werden auch die folgenden Verbindungen der Formel II, wori $R_1' = CH_3$, Pyr = 3-Pyridyl und $R_4$ = OH, hergestellt :

| Verbindung | $R_2'$ | $R_3'$ | $C_mH_{2m}$ | Salz | F. |
|---|---|---|---|---|---|
| 40/1 | 5-Cl | H | $(CH_2)_7$ | HCl | 173-176° |
| 40/2 | 5-$OCH_3$ | H | $(CH_2)_5$ | HBr | 188-189° |
| 40/3 | 5-Cl | 6-Cl | $(CH_2)_5$ | HCl | 178-80° |
| 40/4 | 5-F | H | $(CH_2)_5$ | HCl | 216-219° |
| 40/5 | 5-$CH_3$ | H | $(CH_2)_5$ | HCl | 185-8° |
| 40/6 | 5-$CH_3$ | H | $(CH_2)_7$ | - | 124-125° |
| 40/7 | H | H | $(CH_2)_{10}$ | - | 100-102° |
| 40/8 | 5-O-$CH_2$-O-6 | | $(CH_2)_5$ | | |
| 40/9 | 5-OH | H | $(CH_2)_5$ | - | 168-170° |
| 40/10 | 5-$SCH_3$ | H | $(CH_2)_5$ | - | 135-7° |

Die als Ausgangsstoffe verwendeten N-unsubstituierten Indole sind bekannt. Der neue Ausgangsstoff für die Verbindung 40/10, nämlich das 5-Methylthio-3-methyl-2-(3-pyridyl)-indol schmilzt bei 160-162°.

Die Verbindung 40/9 wird durch Hydrogenolyse des 1-(5-Carboxypentyl)-5-benzyloxy-3-methyl-2-(3-pyridyl)-indols, F. 176-178° hergestellt. Das als Ausgangsstoff verwendete 5-Benzyloxy-3-methyl-2-(3-pyridyl)-indol schmilzt bei 164-166°.

Beispiel 41

Analog zu den in den vorhergehenden Beispielen beschriebenen Methoden werden auch die folgenden Verbindungend der Formel I, worin $R_1 = CH_3$, Ar = 3-Pyridyl und B = COOH hergestellt :

(Siehe Tabelle, Seite 25 f.)

| Beispiel | $R_2$ | $R_3$ | A | B |
|---|---|---|---|---|
| 41/1 | H | H | $C{\equiv}C-(CH_2)_3$ | COOH |
| 41/2 | H | H | $CH_2S(CH_2)_2$ | COOH |
| 41/3 | H | H | $(CH_2)_2O(CH_2)_2$ | COOH |
| 41/4 | H | H | $(CH_2)_2O(CH_2)_3$ | COOH |

Die alkylierenden Ausgangsstoffe für die Verbindungen 41/2, 41/3 und 41/4 werden gemäss J. Org. Chem. *34*, 2955 (1969), US-Patent 3,984,459 bzw. Chem. Abstr. *83*, 166177b hergestellt.

Effekt auf Thromboxan-Synthetase der menschlichen Blutplättchen

Die Methode wird gemäss der vorne angegebenen Beschreibung durchgeführt. Diese in vitro Hemmung des Thromboxan-Synthetase Enzyms ist analog zu der Methode von Sun, Biochem. Biophys. Res. Comm. *74*, 1432 (1977) gezeigt worden.

Ergebnisse :

| Verbindung des Beispiels | $IC_{50}$ (µM) Thromboxan Synthetase |
|---|---|
| 3 | 0,003 |
| 1 | 0,012 |
| 2 | 1,800 |
| 4 | 0,008 |
| 5 | 0,007 |
| 9 | 0,021 |
| 10 | 0,069 |
| 11 | 0,050 |
| 6 | 3,400 |
| 7 | 0,001 |
| 12 | 0,260 |
| 13 | 0,013 |

**Patentansprüche** (für die Vertragsstaaten BE, CH, DE, FR, IT, LI, LU, NL, SE)

1. Verbindungen der allgemeinen Formel I

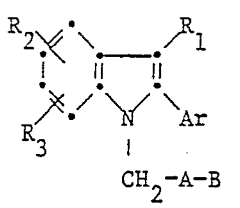

(I)

worin $R_1$ Wasserstoff oder Niederalkyl bedeutet, Ar für unsubstituiertes oder durch Niederalkyl, Carboxy, Niederalkoxycarbonyl oder Carbamoyl substituiertes Pyridyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy, Niederalkoxycarbonyl oder Niederalkyl-(thio, sulfinyl oder sulfonyl) bedeutet, oder $R_2$ und $R_3$ zusammen, an benachbarten Kohlenstoffatomen, für Niederalkylendioxy stehen ; A für Alkylen mit 1 bis 12 Kohlenstoffatomen, Alkenylen mit 2 bis 12 Kohlenstoffatomen, Alkynylen mit 2 bis 12 Kohlenstoffatomen, Niederalkylen-phenylen-niederalkylen, Niederalkylen-phenylen, Phenylen-niederalkylen, Phenylen, eine direkte Bindung, Niederalkylen-(thio oder oxy)-niederalkylen, (Thio oder Oxy)-phenylen, Niederalkylen-(thio oder oxy)-phenylen, Phenylen-(thio oder oxy)-niederalkylen oder Phenylen-niederalkenylen steht, B Carboxy, Niederalkoxycarbonyl, Carbamoyl, Mono- oder Di-niederalkyl-carbamoyl, Hydroxymethyl, Hydroxycarbamoyl, 5-Tetrazolyl oder Formul bedeutet ; ihre N-Oxide ; und ihre Salze, wobei die mit « nieder » bezeichneten Reste höchstens 7 Kohlenstoffatome aufweisen.

2. Verbindungen nach Anspruch 1, worin $R_1$ Wasserstoff oder Niederalkyl bedeutet, Ar für unsubstituiertes oder durch Niederalkyl, Carboxy, Niederalkoxycarbonyl oder Carbamoyl substituiertes Pyridyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy oder Niederalkoxycarbonyl bedeutet, A für Alkylen mit 1 bis 12 Kohlenstoffatomen, Alkenylen mit 2 bis 12 Kohlenstoffatomen, Alkynylen mit 2 bis 12 Kohlenstoffatomen, Niederalkylen-phenylen-niederalkylen, Niederalkylen-phenylen, Phenylen-niederalkylen, Phenylen oder eine direkte Bindung steht, B Carboxy, Niederalkoxycarbonyl, Carbamoyl, Mono- oder Di-niederalkyl-carbamoyl oder Hydroxymethol bedeutet ; ihre N-Oxide ; und ihre Salze, wobei die mit « nieder » bezeichneten Reste höchstens 7 Kohlenstoffatome aufweisen.

3. Verbindungen der Formel II

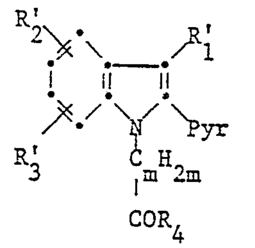

(II)

worin $R'_1$ Wasserstoff oder Niederalkyl bedeutet, $R'_2$ und $R'_3$ unabhängig voneinander Wasserstoff, Niederalkyl, Halogen, Trifluormethyl, Hydroxy, Niederalkylthio oder Niederalkoxy steht, oder $R'_2$ und $R'_3$ zusammen, an benachbarten Kohlenstoffatomen, Methylendioxy bedeuten, Pyr 2-, 3- oder 4-Pyridyl bedeutet, m für eine ganze Zahl von 1 bis 13 steht, $R_4$ Hydroxy, Niederalkoxy oder Amino bedeutet ; und ihre Salze, wobei die mit « nieder » bezeichneten Reste höchstens 7 Kohlenstoffatome aufweisen.

4. Verbindungen der Formel III

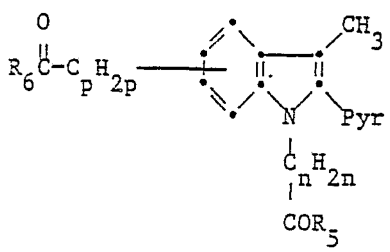

(III)

worin n eine ganze Zahl von 3 bis 10 bedeutet, p für eine ganze Zahl von 0 bis 4 steht, Pyr 2-, 3- oder 4-Pyridyl bedeutet, $R_5$ und $R_6$ unabhängig voneinander für Hydroxy oder Niederalkoxy stehen ; und Salze, wobei die mit « nieder » bezeichneten Reste höchstens 7 Kohlenstoffatome aufweisen.

5. Verbindungen der Formel IV

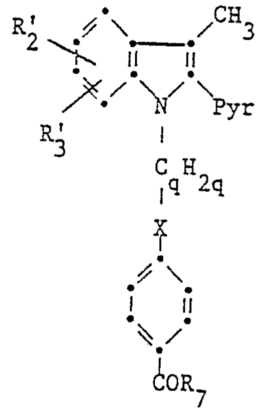

(IV)

worin $R'_2$ und $R'_3$ unabhängig voneinander Wasserstoff, Niederalkyl, Halogen, Niederalkoxy, Niederalkylthio oder Hydroxy bedeuten, oder $R'_2$ und $R'_3$ zusammen, an benachbarten Kohlenstoffatomen für Methylendioxy stehen ; X Sauerstoff, Schwefel oder eine direkte Bindung bedeutet, q für eine ganze Zahl von 1 bis 4 steht, $R_7$ Hydroxy oder Niederalkoxy bedeutet ; Pyr für 2-, 3-, oder 4-Pyridyl steht, und Salze, wobei die mit « nieder » bezeichneten Reste höchstens 7 Kohlenstoffatome aufweisen.

6. 1-(7-Carboxyheptyl)-3-methyl-2-(3-pyridyl)-indol und seine Salze.

7. 1-(5-Carboxypentyl)-3-methyl-2-(3-pyridyl)-indol und seine Salze.

8. 1-(4-Carboxybutyl)-3-methyl-2-(3-pyridyl)-indol und seine Salze.

9. 1-(5-Carboxypentyl)-5-chlor-3-methyl-2-(3-pyridyl)-indol und seine Salze.

10. Pharmazeutische Präparate enthaltend Verbindungen der Ansprüche 1, 3 und 5 zusammen mit einem pharmazeutischen Trägermaterial.

11. Pharmazeutische Präparate enthaltend Verbindungen der Ansprüche 2, 4 und 6 bis 9 zusammen mit einem pharmazeutischen Trägermaterial.

12. Die in den Ansprüchen 1, 3 und 5 genannten Verbindungen zur Anwendung in einem Verfahren zur therapeutischen Behandlung des menschlichen oder tierischen Körpers.

13. Die in den Ansprüchen 2, 4 und 6 bis 9 genannten Verbindungen zur Anwendung in einem Verfahren zur Behandlung des menschlichen oder tierischen Körpers.

14. Die in den Ansprüchen 1, 3 und 5 genannten Verbindungen als Hemmer der Thromboxan-Synthetase.

15. Die in den Ansprüchen 2, 4 und 6 bis 9 genannten Verbindungen als Hemmer der Thromboxan-Synthetase.

16. Verwendung der in den Ansprüchen 1, 3 und 5 genannten Verbindungen zur Herstellung von pharmazeutischen Präparaten.

17. Verwendung der in den Ansprüchen 2, 4 und 6 bis 9 genannten Verbindungen zur Herstellung von pharmazeutischen Präparaten.

18. Verfahren zur Herstellung von den im Anspruch 1 genannten Verbindungen der Formel I, ihren N-Oxiden und Salzen dieser Verbindungen, dadurch gekennzeichnet, dass man

1) eine Verbindung der Formel V

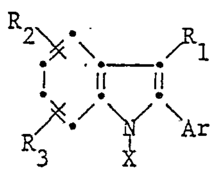

(V)

worin X Wasserstoff, Alkalimetall oder Tri-niederalkylsilyl bedeutet, $R_1$, $R_2$, $R_3$ und Ar die angegebene Bedeutung haben, mit einem reaktionsfähigen funktionellen Derivat einer Verbindung der Formel VI

$$HO—CH_2—A—B \qquad (VI)$$

worin A und B die oben angegeben Bedeutung haben, kondensiert, oder

2) eine Verbindung der Formel VII

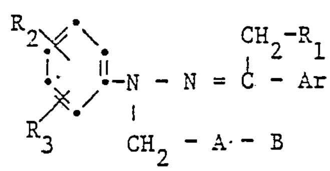

(VII)

worin Ar, $R_1$, $R_2$, $R_3$, A und B die oben angegebene Bedeutung haben, ringschliesst, oder

3) eine Verbindung der Formel VIII

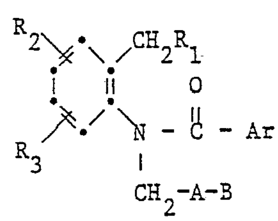

(VIII)

worin Ar, $R_1$, $R_2$, $R_3$, A und B die angegebene Bedeutung haben, cyclisiert, oder

4) in einer Verbindung der Formel Ia

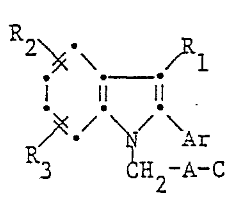

(Ia)

worin A, Ar, $R_1$, $R_2$ und $R_3$ die oben angegebene Bedeutung haben, und c eine von einer Gruppe B verschiedene, in die Gruppe B überführbare Gruppe bedeutet, die Gruppe C in B umwandelt, gegebenenfalls unter Verlängerung der Kette A innerhalb ihrer Definition, oder

5) eine Verbindung der Formel IX

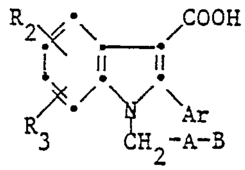

(IX)

worin A, B, Ar, $R_2$ und $R_3$ die oben angegebene Bedeutung haben, decarboxyliert, und, wenn erwünscht oder notwendig, eine störende reaktionsfähige Gruppe in allen diesen Verfahren vorübergehend schützt, und, wenn etwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

19. Die nach dem Verfahren des Anspruchs 18 erhältlichen Verbindungen.

**Patentansprüche** (für den Vertragsstaat AT)

1. Verfahren zur Herstellung von N-substituierten 2-Pyridylindolen der allgemeinen Formel I

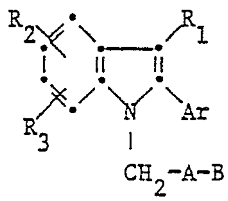

(I)

worin $R_1$ Wasserstoff oder Niederalkyl bedeutet, Ar für unsubstituiertes oder durch Niederalkyl, Carboxy, Niederalkoxycarbonyl oder Carbamoyl substituiertes Pyridyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy, Niederalkoxycarbonyl oder Niederalkyl-(thio, sulfinyl oder sulfonyl) bedeutet, oder $R_2$ und $R_3$ zusammen, an benachbarten Kohlenstoffatomen, für Niederalkylendioxy stehen ; A für Alkylen mit 1 bis 12 Kohlenstoffatomen, Alkenyl mit 2 bis 12 Kohlenstoffatomen, Alkynylen mit 2 bis 12 Kohlenstoffatomen, Niederalkylen-phenylen-niederalkylen, Niederalkylen-phenylen, Phenylen-niederalkylen, Phenylen, eine direkte Bindung, Niederalkylen-(thio oder oxy)-niederalkylen, (Thio oder Oxy)-phenylen, Niederalkylen-(thio oder oxy)-phenylen, Phenylen-(thio oder oxy)-niederalkylen oder Phenylen-niederalkenylen steht, B Carboxy, Niederalkoxycarbonyl, Carbamoyl, Mono- oder Di-niederalkyl-carbamoyl, Hydroxymethyl, Hydroxycarbamoyl, 5-Tetrazolyl oder Formyl bedeutet ; ihren N-Oxiden ; und Salzen dieser Verbindungen, wobei die mit « nieder » bezeichneten Reste höchstens 7 Kohlenstoffatome aufweisen, dadurch gekennzeichnet, dass man

1) eine Verbindung der Formel V

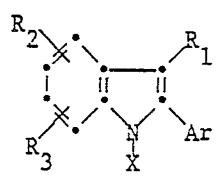

(V)

worin X Wasserstoff, Alkalimetall oder Tri-niederalkylsilyl bedeutet, $R_1$, $R_2$, $R_3$ und Ar die angegebene Bedeutung haben, mit einem reaktionsfähigen funktionellen Derivat einer Verbindung der Formel VI

$$HO{-}CH_2{-}A{-}B \qquad (VI)$$

worin A und B die oben angegebene Bedeutung haben, kondensiert, oder

2) eine Verbindung der Formel VII

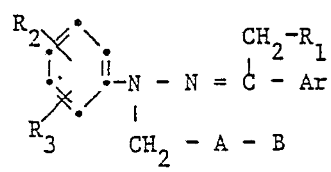

(VII)

worin Ar, $R_1$, $R_2$, $R_3$, A und B die oben angegebene Bedeutung haben, ringschliesst, oder

3) eine Verbindung der Formel VIII

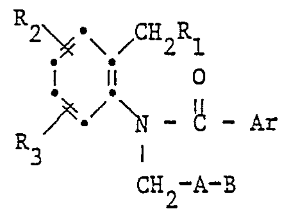

(VIII)

worin Ar, $R_1$, $R_2$, $R_3$, A und B die angegebene Bedeutung haben, cyclisiert, oder

4) in einer Verbindung der Formel Ia

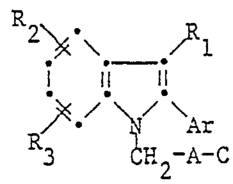

(Ia)

worin A, Ar, $R_1$, $R_2$ und $R_3$ die oben angegebene Bedeutung haben, und C eine von einer Gruppe B verschiedene, in die Gruppe B überführbare Gruppe bedeutet, die Gruppe C in B umwandelt, gegebenenfalls unter Verlängerung der Kette A innerhalb ihrer Definition, oder

5) eine Verbindung der Formel IX

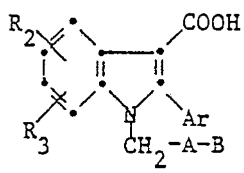

(IX)

worin A, B, Ar, $R_2$ und $R_3$ die oben angegebene Bedeutung haben, decarboxyliert, und, wenn erwünscht oder notwendig, eine störende reaktionsfähige Gruppe in allen diesen Verfahren vorübergehend schützt, und, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

2. Verfahren anch Anspruch 1, zur Herstellung von Verbindungen der im Anspruch 1 gezeigten Formel I, worin $R_1$ Wasserstoff oder Niederalkyl bedeutet, Ar für unsubstituiertes oder durch Niederalkyl, Carboxy, Niederalkoxycarbonyl oder Carbamoyl substituiertes Pyridyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy oder Niederalkoxycarbonyl bedeutet, A für Alkylen mit 1 bis 12 Kohlenstoffatomen, Alkenylen mit 2 bis 12 Kohlenstoffatomen, Alkynylen mit 2 bis 12 Kohlenstoffatomen, Niederalkylen-phenylen-niederalkylen, Niederalkylen-phenylen, Phenylen-niederalkylen, Phenylen oder eine direkte Bindung steht, B Carboxy, Niederalkoxycarbonyl, Carbamoyl, Mono- oder Diniederalkyl-carbamoyl oder Hydroxymethyl bedeutet ; ihren N-Oxiden ; und Salzen ideser Verbindungen, wobei die mit « nieder » bezeichneten Reste höchstens 7 Kohlenstoffatome aufweisen, dadurch gekennzeichnet, dass man eine Verbindung der im Anspruch 1 gezeigten Formel V, worin X Wasserstoff bedeutet, und die anderen Symbole die oben angegebenen Bedeutungen haben, mit einem reaktionsfähigen funktionellen Derivat einer Verbindung der Formel VIa

$$HO—CH_2—A—B' \qquad (VIa)$$

worin A die oben angegebene Bedeutung hat und B' für Carboxy, Niederalkoxycarbonyl, Carbamoyl, Mono- oder Dinieralkyl-carbamoyl, Hydroxymethyl, veräthertes Hydroxymethyl, Halogenmethyl, Trialkoxymethyl oder Cyan steht, kondensiert, und in einer erhaltenen Verbindung der Formel Ib

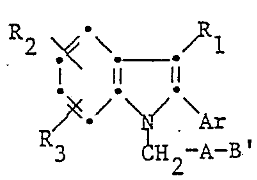

(Ib)

worin sich B′ von B unterscheidet, den Rest B′, gegebenenfalls unter Verlängerung der Kette A innerhalb ihrer Definition, in die Gruppe B umwandelt, und, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

3. Verfahren nach Anspruch 1, dadurch gekennzeichnet, dass man Verbindungen der Formel II

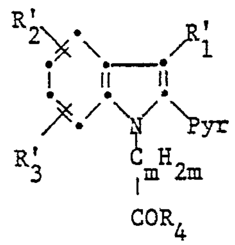

(II)

worin $R'_1$ Wasserstoff oder Niederalkyl bedeutet, $R'_2$ und $R'_3$ unabhängig voneinander Wasserstoff, Niederalkyl, Halogen, Trifluormethyl, Hydroxy, Niederalkylthio oder Niederalkoxy steht, oder $R'_2$ und $R'_3$ zusammen, an benachbarten Kohlenstoffatomen, Methylendioxy bedeuten, Pyr 2-, 3-, oder 4-Pyridyl bedeutet, m für eine ganze Zahl von 1 bis 13 steht, $R_4$ Hydroxy, Niederalkoxy oder Amino bedeutet ; und ihre Salze herstellt, wobei die mit « nieder » bezeichneten Reste höchstens 7 Kohlenstoffatome aufweisen.

4. Verfahren nach Anspruch 2, dadurch gekennzeichnet, dass man Verbindungen der Formel III

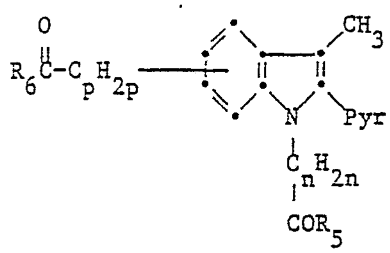

(III)

worin n eine ganze Zahl von 3 bis 10 bedeutet, p für eine ganze Zahl von 0 bis 4 steht, Pyr 2-, 3- oder 4-Pyridyl bedeutet, $R_5$ und $R_6$ unabhängig voneinander für Hydroxy oder Niederalkoxy stehen ; und ihre Salze herstellt, wobei die mit « nieder » bezeichneten Reste höchstens 7 Kohlenstoffatome aufweisen.

5. Verfahren nach Anspruch 1, dadurch gekennzeichnet, dass man Verbindungen der Formel IV

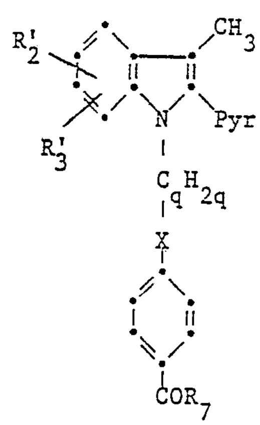

(IV)

worin $R'_2$ und $R'_3$ unabhängig voneinander Wasserstoff, Niederalkyl, Halogen, Niederalkoxy, Niederalkylthio oder Hydroxy bedeuten, oder $R'_2$ und $R'_3$ zusammen, an benachbarten Kohlenstoffatomen für Methylendioxy stehen ; X Sauerstoff, Schwefel oder eine direkte Bindung bedeutet, q für eine ganze Zahl von 1 bis 4 steht, $R_7$ Hydroxy oder Niederalkoxy bedeutet ; Pyr für 2-, 3- oder 4-Pyridyl steht, und ihre Salze herstellt, wobei die mit « nieder » bezeichneten Reste höchstens 7 Kohlenstoffatome aufweisen.

6. Verfahren nach Anspruch 1, dadurch gekennzeichnet, dass man 1-(7-Carboxyheptyl)-3-methyl-2-(3-pyridyl)-indol und seine Salze herstellt.

7. Verfahren nach Anspruch 2, dadurch gekennzeichnet, dass man 1-(7-Carboxyheptyl)-3-methyl-2-(3-pyridyl)-indol und seine Salze herstellt.

8. Verfahren nach Anspruch 1, dadurch gekennzeichnet, dass man 1-(5-Carboxypentyl)-3-methyl-2-(3-pyridyl)-indol und seine Salze herstellt.

9. Verfahren nach Anspruch 2, dadurch gekennzeichnet, dass man 1-(5-Carboxypentyl)-3-methyl-2-(3-pyridyl)-indol und seine Salze herstellt.

10. Verfahren nach Anspruch 1, dadurch gekennzeichnet, dass man 1-(4-Carboxybutyl)-3-methyl-2-(3-pyridyl)-indol und seine Salze herstellt.

11. Verfahren nach Anspruch 2, dadurch gekennzeichnet, dass man 1-(4-Carboxybutyl)-3-methyl-2-(3-pyridyl)-indol und seine Salze herstellt.

12. Verfahren nach Anspruch 1, dadurch gekennzeichnet, dass man 1-(5-Carboxypentyl)-5-chlor-3-methyl-2-(3-pyridyl)-indol und seine Salze herstellt.

13. Verfahren nach Anspruch 2, dadurch gekennzeichnet, dass man 1-(5-Carboxypentyl)-5-chlor-3-methyl-2-(3-pyridyl)-indol und seine Salze herstellt.

14. Verfahren zur Herstellung eines pharmazeutischen Präparates, gekennzeichnet durch die Verarbeitung eines erfindungsgemässen Wirkstoffes nach einem der Ansprüche 1, 3, 5, 6, 8, 10 und 12 mit einem pharmazeutischen Trägermaterial.

15. Verfahren zur Herstellung eines pharmazeutischen Präparates, gekennzeichnet durch die Verarbeitung eines erfindungsgemässen Wirkstoffes nach einem der Ansprüche 2, 4, 7, 9, 11 und 13 mit einem pharmazeutischen Trägermaterial.

**Claims** (for the Contracting States : BE, CH, DE, FR, IT, LI, LU, NL, SE)

1. A compound of the general formula I

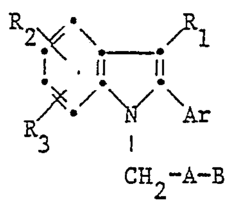

(I)

wherein $R_1$ represents hydrogen or lower alkyl ; Ar represents pyridyl unsubstituted or substituted by lower alkyl, carboxy, lower alkoxycarbonyl or carbamoyl ; $R_2$ and $R_3$ independently represent hydrogen, lower alkyl, halogen, trifluoromethyl, hydroxy, lower alkoxy, carboxy lower alkyl, lower alkoxycarbonyl lower alkyl, carboxy, lower alkoxycarbonyl, or lower alkyl-(thio, sulfinyl or sulfonyl), or $R_2$ and $R_3$ together on adjacent carbon atoms represent lower alkylenedioxy ; A represents alkylene of 1 to 12 carbon atoms, alkenylene of 2 to 12 carbon atoms, alkynylene of 2 to 12 carbon atoms, lower alkylenephenylene lower alkylene, lower alkylenephenylene, phenylene lower alkylene, phenylene, a direct bond, lower alkylene-(thio or oxy)-lower alkylene, (thio or oxy)-phenylene, lower alkylene-(thio or oxy)-phenylene, phenylene-(thio or oxy)-lower alkylene or phenylene lower alkenylene ; B represents carboxy, lower alkoxycarbonyl, carbamoyl, mono- or dilower alkylcarbamoyl, hydroxymethyl, hydroxycarbamoyl, 5-tetrazolyl or formyl ; or an N-oxide or a salt thereof, with the radicals qualified by the term « lower » containing not more than 7 carbon atoms.

2. A compound of the formula I shown in claim 1, wherein $R_1$ represents hydrogen or lower alkyl ; Ar represents pyridyl unsubstituted or substituted by lower alkyl, carboxy, lower alkoxycarbonyl or carbamoyl ; $R_2$ and $R_3$ independently represent hydrogen, lower alkyl, halogen, trifluoromethyl, hydroxy, lower alkoxy, carboxy lower alkyl, lower alkoxycarbonyl lower alkyl, carboxy or lower alkoxycarbonyl, A represents alkylene of 1 to 12 carbon atoms, alkenylene of 2 to 12 carbon atoms, alkynylene of 2 to 12 carbon atoms, lower alkylenephenylene lower alkylene, lower alkylenephenylene, phenylene lower alkylene, phenylene, a direct bond, B represents carboxy, lower alkoxycarbonyl, carbamoyl, mono- or di-lower alkylcarbamoyl or hydroxymethyl ; or an N-oxide or a salt thereof, with the radicals qualified by the term « lower » containing not more than 7 carbon atoms.

3. A compound of the formula II

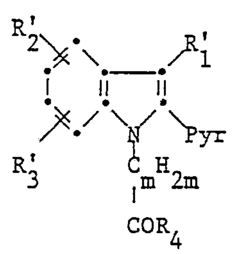

(II)

wherein $R_1'$ reprensents hydrogen or lower alkyl ; $R_2'$ and $R_3'$ independently represent hydrogen, lower alkyl, halogen, trifluoromethyl, hydroxy, lower alkylthio or lower alkoxy ; or $R_2'$ and $R_3'$ together on adjacent carbon atoms represent methylenedioxy ; Pyr represents 2-, 3- or 4- pyridyl ; m represents an

integer from 1 to 13 ; $R_4$ represents hydroxy, lower alkoxy or amino ; or a salt thereof, with the radicals qualified by the term « lower » containing not more than 7 carbon atoms.

4. A compound of the formula III

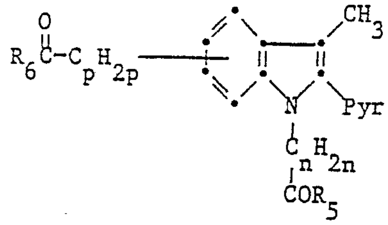

(III)

wherein n represents an integer from 3 to 10 ; p represents an integer from 0 to 4 ; Pyr represents 2-, 3- or 4-pyridyl ; $R_5$ and $R_6$ independently represent hydroxy or lower alkoxy ; or a salt thereof, with the radicals qualified by the term « lower » containing not more than 7 carbon atoms.

5. A compound of the formula IV

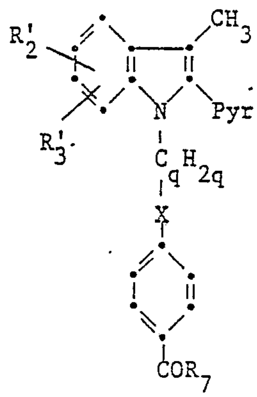

(IV)

wherein $R_2'$ and $R_3'$ independently represent hydrogen, lower alkyl, halogen, lower alkoxy, lower alkylthio or hydroxy ; or $R_2'$ and $R_3'$ together on adjacent carbon atoms represent methylenedioxy ; X represents oxygen, sulfur or a direct bond ; q represents an integer from 1 to 4 ; $R_7$ represents hydroxy or lower alkoxy ; and Pyr represents 2-, 3- or 4- pyridyl ; or a salt thereof, with the radicals qualified by the term « lower » containing not more than 7 carbon atoms.

6. 1-(7-Carboxyheptyl)-3-methyl-2-(3-pyridyl)-indole, or a salt thereof.

7. 1-(5-Carboxypentyl)-3-methyl-2-(3-pyridyl)-indole, or a salt thereof.

8. 1-(4-Carboxybutyl)-3-methyl-2-(3-pyridyl)-indole, or a salt thereof.

9. 1-(5-Carboxypentyl)-5-chloro-3-methyl-2-(3-pyridyl)-indole, or a salt thereof.

10. A pharmaceutical composition containing a compound as claimed in any one of claims 1, 3 and 5, together with a pharmaceutical carrier.

11. A pharmaceutical composition containing a compound as claimed in any one of claims 2, 4 and 6 to 9, together with a pharmaceutical carrier.

12. A compound as claimed in any one of claims 1, 3 and 5 for use in a therapeutic method of treating the human or animal body.

13. A compound as claimed in any one of claims 2, 4 and 6 to 9 for use in a method of treating the human or animal body.

14. A compound as claimed in any one of claims 1, 3 and 5 for inhibiting the synthesis of thromboxane.

15. A compound as claimed in any one of claims 2, 4 and 6 to 9 for inhibiting the synthesis of thromboxane.

16. Use of a compound as claimed in any one of claims 1, 3 and 5 for the preparation of a pharmaceutical composition.

17. Use of a compound as claimed in any one of claims 2, 4 and 6 to 9 for the preparation of a pharmaceutical composition.

18. A process for the preparation of a compound of the formula I as claimed in claim 1, of an N-oxide or a salt thereof, which process comprises

1) condensing a compound of the formula V

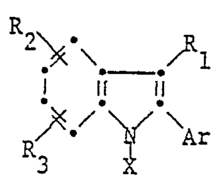

(V)

wherein X is hydrogen, alkaline metal or tri-lower alkyl silyl, $R_1$, $R_2$, $R_3$ and Ar have the given meaning with a reactive functional derivative of a compound of the formula VI

$HOCH_2—A—B$ (VI)

wherein A and B have the meaning given above, or

2) ring-closing a compound of formula VII

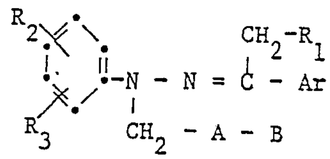

(VII)

wherein Ar, $R_1$, $R_2$, $R_3$, A and B have the meaning given above, or

3) cyclizing a compound of the formula VIII

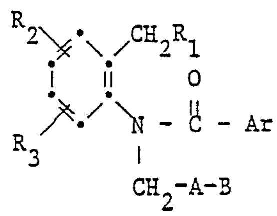

(VIII)

wherein Ar, $R_1$, $R_2$, $R_3$, A and B have the given meaning, or

4) in a compound of the formula Ia

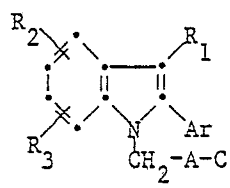

(Ia)

wherein A, Ar, $R_1$, $R_2$ and $R_3$ have the meaning given above and C is a group differing from B and convertible into B, converting said group C into B, optionally by extending the chain A within its definition, or

5) decarboxylating a compound of the formula IX

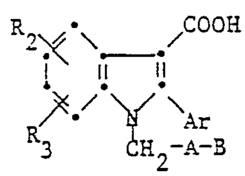

(IX)

in which A, B, Ar, $R_2$ and $R_3$ have the meaning given above and, if desired or necessary, temporarily protecting in each of these processes an interfering reactive group, and, if desired, converting any resulting compound of the formula I into another compound of the invention, and/or, if desired, converting a resulting free compound into a salt or a resulting salt into the free compound or into another salt, and/or, if required, resolving a mixture of isomers or racemates obtained into the single isomers or racemates, and/or, if required, resolving a racemate obtained into the optical antipodes.

19. A compound obtainable according to a process as claimed in claim 18.

**Claims** (for the Contracting State AT)

1. A process for the preparation of an N-substituted-2-pyridylindole of the general formula 1

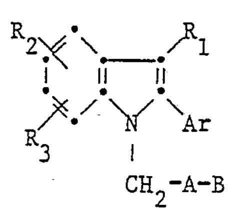

(I)

wherein $R_1$ represents hydrogen or lower alkyl ; Ar represents pyridyl unsubstituted or substituted by lower alkyl, carboxy, lower alkoxycarbonyl or carbamoyl ; $R_2$ and $R_3$ independently represent hydrogen, lower alkyl, halogen, trifluoromethyl, hydroxy, lower alkoxy, carboxy lower alkyl, lower alkoxycarbonyl lower alkyl, carboxy, lower alkoxycarbonyl, or lower alkyl-(thio, sulfinyl or sulfonyl), or $R_2$ and $R_3$ together on adjacent carbon atoms represent lower alkylenedioxy ; A represents alkylene of 1 to 12 carbon atoms, alkenylene of 2 to 12 carbon atoms, alkynylene of 2 to 12 carbon atoms, lower alkylenephenylene lower alkylene, lower alkylenephenylene, phenylene lower alkylene, phenylene, a direct bond, lower alkylene-(thio or oxy)-lower alkylene, (thio or oxy)-phenylene, lower alkylene-(thio or oxy)-phenylene, phenylene-(thio or oxy)-lower alkylene or phenylene lower alkenylene ; B represents carboxy, lower alkoxycarbonyl, carbamoyl, mono- or di-lower alkylcarbamoyl, hydroxymethyl, hydroxycarbamoyl, 5-tetrazolyl or formyl ; or an N-oxide or a salt thereof, with the radicals qualified by the term « lower » containing not more than 7 carbon atoms, which process comprises

1) condensing a compound of the formula V

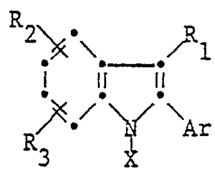

(V)

wherein X is hydrogen, alkaline metal or tri-lower alkyl silyl, $R_1$, $R_2$, $R_3$ and Ar have the given meaning, with a reactive functional derivative of a compound of the formula VI

$$HOCH_2—A—B \quad (VI)$$

wherein A and B have the meaning given above, or

2) ring-closing a compound of formula VII

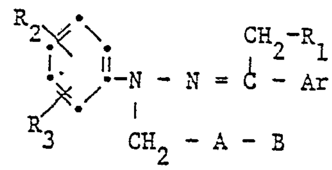

(VII)

wherein Ar, $R_1$, $R_2$, $R_3$, A and B have the meaning given above, or

3) cyclizing a compound of the formula VIII

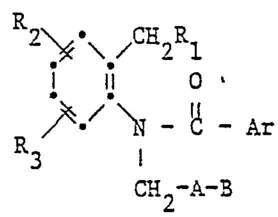

(VIII)

wherein Ar, $R_1$, $R_2$, $R_3$, A and B have the given meaning, or

4) in a compound of the formula Ia

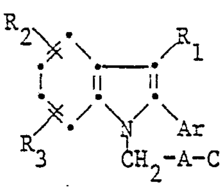

(Ia)

wherein A, Ar, $R_1$, $R_2$ and $R_3$ have the meaning given above and C is a group differing from B and convertible into B, converting said group C into B, optionally by extending the chain A within its definition, or

5) decarboxylating a compound of the formula IX

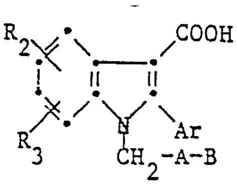

(IX)

in which A, B, Ar, $R_2$ and $R_3$ have the meaning given above, and, if desired or necessary, temporarily protecting in each of these processes an interfering reactive group, and, if desired, converting any resulting compound of the formula I into another compound of the invention, and/or, if desired, converting a resulting free compound into a salt or a resulting salt into the free compound or into another salt, and/or, if required, resolving a mixture of isomers or racemates obtained into the single isomers or racemates, and/or, if required, resolving a racemate obtained into the optical antipodes.

2. A process according to claim 1 for the preparation of a compound shown in claim 1, wherein $R_1$ represents hydrogen or lower alkyl ; Ar represents pyridyl unsubstituted or substituted by lower alkyl, carboxy, lower alkoxycarbonyl or carbamoyl ; $R_2$ and $R_3$ independently represent hydrogen, lower alkyl, halogen, trifluoromethyl, hydroxy, lower alkoxy, carboxy lower alkyl, lower alkoxycarbonyl lower alkyl, carboxy or lower alkoxycarbonyl, A represents alkylene of 1 to 12 carbon atoms, alkenylene of 2 to 12 carbon atoms, alkynylene of 2 to 12 carbon atoms, lower alkylenephenylene lower alkylene, lower alkylenephenylene, phenylene lower alkylene, phenylene, a direct bond, B represents carboxy, lower alkoxycarbonyl, carbamoyl, mono- or di-lower alkylcarbamoyl or hydroxymethyl ; or an N-oxide or a salt thereof, whith the radicals qualified by the term « lower » containing not more than 7 carbon atoms, which process comprises condensing a compound of formula V shown in claim 1, wherein X represents hydrogen, and the other symbols have the meaning given above, with a reactive functional derivative of a compound of the formula VIa

$$HO—CH_2—A—B' \qquad (VIa)$$

wherein A has the meaning given above, and B′ represents carboxy, lower alkoxycarbonyl, carbamoyl, mono- or di-lower alkylcarbamoyl, hydroxymethyl, etherified hydroxymethyl, halomethyl, trialkoxymethy or cyano, and in a resulting compound of the formula Ib

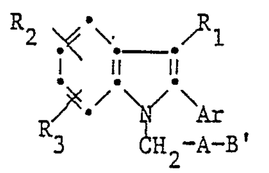

(Ib)

wherein B′ differs from B, converting said radical B′ into the group B, optionally by extending the chain A within its definition, and, if desired, converting any resulting compound of the formula I into another compound of the invention, and/or, if desired, converting a resulting free compound into a salt or a resulting salt into the free compound or into another salt, and/or, if required, resolving a mixture of isomers or racemates obtained into the single isomers or racemates, and/or, if required, resolving a racemate obtained into the optical antipodes.

3. A process according to claim 1, which comprises preparing a compound of the formula II

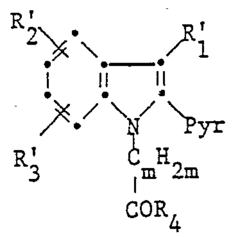

(II)

wherein $R_1'$ represents hydrogen or lower alkyl ; $R_2'$ and $R_3'$ independently represent hydrogen, lower alkyl, halogen, trifluoromethyl, hydroxy, lower alkylthio or lower alkoxy ; or $R_2'$ and $R_3'$ together on adjacent carbon atoms represent methylenedioxy ; Pyr represents 2-, 3- or 4- pyridyl ; m represents an integer from 1 to 13 ; $R_4$ represents hydroxy, lower alkoxy or amino ; or a salt thereof, with the radicals qualified by the term « lower » containing not more than 7 carbon atoms.

4. A process according to claim 2, which comprises preparing a compound of the formula III

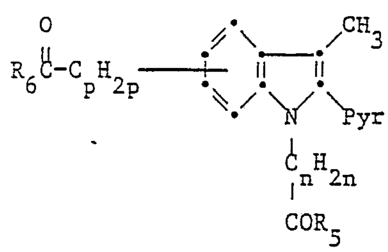

(III)

wherein n represents an integer from 3 to 10 ; p represents an integer from 0 to 4 ; Pyr represents 2-, 3- or 4-pyridyl ; $R_5$ and $R_6$ independently represent hydroxy or lower alkoxy ; or a salt thereof, with the radicals qualified by the term « lower » containing not more than 7 carbon atoms.

5. A process according to claim 1, which comprises preparing a compound of the formula IV

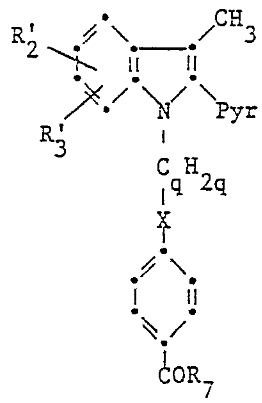

(IV)

wherein $R_2'$ and $R_3'$ independently represent hydrogen, lower alkyl, halogen, lower alkoxy, lower alkylthio or hydroxy ; or $R_2'$ and $R_3'$ together on djacent carbon atoms represent methylenedioxy ; X represents oxygen, sulfur or a direct bond ; q represents an integer from 1 to 4 ; $R_7$ represents hydroxy or lower alkoxy ; and Pyr represents 2-, 3- or 4-pyridyl ; or a salt thereof, with the radicals qualified by the term « lower » containing not more than 7 carbon atoms.

6. A process according to claim 1, which comprises preparing 1-(7-carboxyheptyl)-3-methyl-2-(3-pyridyl)-indole, or a salt thereof.

7. A process according to claim 2, which comprises preparing 1-(7-carboxyheptyl)-3-methyl-2-(3-pyridyl)-indole, or a salt thereof.

8. A process according to claim 1, which comprises preparing 1-(5-carboxypentyl)-3-methyl-2-(3-pyridyl)-indole, or a salt thereof.

9. A process according to claim 2, which comprises preparing 1-(5-carboxypentyl)-3-methyl-2-(3-pyridyl)-indole, or a salt thereof.

10. A process according to claim 1, which comprises preparing 1-(4-carboxybutyl)-3-methyl-2-(3-pyridyl)-indole, or a salt thereof.

11. A process according to claim 2, which comprises preparing 1-(4-carboxybutyl)-3-methyl-2-(3-pyridyl)-indole, or a salt thereof.

12. A process according to claim 1, which comprises preparing 1-(5-carboxypentyl)-5-chloro-3-methyl-2-(3-pyridyl)-indole, or a salt thereof.

13. A process according to claim 2, which comprises preparing 1-(5-carboxypentyl)-5-chloro-3-methyl-2-(3-pyridyl)-indole, or a salt thereof.

14. A process for the preparation of a pharmaceutical composition, which process comprises mixing a compound of the invention as claimed in any one of claims 1, 3, 5, 6, 8, 10 and 12 with a pharmaceutical carrier.

15. A process for the preparation of a pharmaceutical composition, which process comprises mixing a compound of the invention as claimed in any one of claims 2, 4, 7, 9, 11 and 13 with a pharmaceutical carrier.

**Revendications** (pour les Etats contractants : BE, CH, DE, FR, IT, LI, LU, NL, SE)

1. Composés de formule générale I

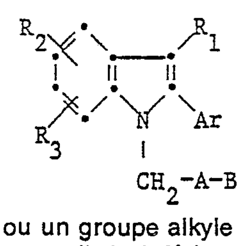

(I)

dans laquelle $R_1$ représente l'hydrogène ou un groupe alkyle inférieur, Ar représente un groupe pyridyle non substitué ou substitué par des groupes alkyle inférieurs, carboxy, (alcoxy inférieur)-carbonyle ou carbamoyle, $R_2$ et $R_3$ représentent, indépendamment l'un de l'autre, l'hydrogène, des groupes alkyle inférieurs, des halogènes, des groupes trifluorométhyle, hydroxy, alcoxy inférieur, carboxyalkyle inférieurs, (alcoxy inférieur)-carbonylalkyle inférieurs, carboxy,(alcoxy inférieur)-carbonyle ou (alkyle inférieur)-(thio, sulfinyle ou sulfonyle), ou bien $R_2$ et $R_3$ forment ensemble, sur des atomes de carbone voisins, un groupe alkylène-dioxy inférieur ; A représente un groupe alkylène en C1-C12, alcénylène en C2-C12, alcynylène en C2-C12, (alkylène inférieur)-phénylène-alkylène inférieur, (alkylène inférieur)-phénylène, phénylènealkylène inférieur, phénylène, une liaison directe, un groupe (alkylène inférieur)-(thio ou oxy)-alkylène inférieur, (thio ou oxy)-phénylène, (alkylène inférieur)-(thio ou oxy)-phénylène, phénylène-(thio ou oxy)-alkylène inférieur ou phénylène-alcénylène inférieur, B représente un groupe carboxy, (alcoxy inférieur)-carbonyle, carbamoyle, mono- ou di-(alkyle inférieur)-carbamoyle, hydroxyméthyle, hydroxycarbamoyle, 5-tétrazolyle ou formyle ; leurs N-oxydes ; et leurs sels, les groupes qualifiés d'« inférieurs » contenant au maximum 7 atomes de carbone.

2. Composés selon la revendication 1, dans lesquels $R_1$ représente l'hydrogène ou un groupe alkyle inférieur, Ar représente un groupe pyridyle non substitué ou substitué par des groupes alkyle inférieurs, carboxy, (alcoxy inférieur)-carbonyle ou carbamoyle, $R_2$ et $R_3$ représentent indépendamment l'un de l'autre, l'hydrogène, des groupes alkyle inférieurs, des halogènes, des groupes trifluorométhyle, hydroxy, alcoxy inférieurs ; carboxyalkyle inférieurs, (alcoxy inférieur)-carbonyl-alkyle inférieur, carboxy ou (alcoxy inférieur)-carbonyle, A représente un groupe alkylène en C1-C12, alcénylène en C2-C12, alcynylène en C2-C12, (alkylène inférieur)-phénylène-alkylène inférieur, (alkylène inférieur)-phénylène, phénylènealkylène inférieur, phénylène ou une liaison directe, B représente un groupe carboxy, (alcoxy inférieur)-carbonyle, carbamoyle, mono- ou di-(alkyle inférieur)-carbamoyle ou hydroxyméthyle ; leurs N-oxydes ; et leurs sels, les groupes qualifiés d'« inférieurs » contenant au maximum 7 atomes de carbone.

3. Composés de formule II

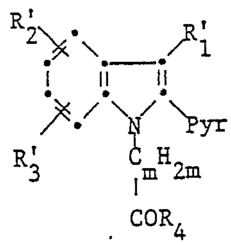

(II)

dans laquelle $R'_1$ représente l'hydrogène ou un groupe alkyle inférieur, $R'_2$ et $R'_3$ représentent, indépendamment l'un de l'autre, l'hydrogène, des groupes alkyle inférieurs, des halogènes, des groupes trifluorométhyle, hydroxy, alkylthio inférieurs ou alcoxy inférieurs, ou bien $R'_2$ et $R'_3$ forment ensemble, sur des atomes de carbone voisins, un groupe méthylène-dioxy, Pyr représente un groupe 2-, 3- ou 4-pyridyle, m est un nombre entier de 1 à 13, $R_4$ représente un groupe hydroxy, alcoxy inférieur ou amino ; et leurs sels, les groupes qualifiés d'« inférieurs » contenant au maximum 7 atomes de carbone.

4. Composés de formule III

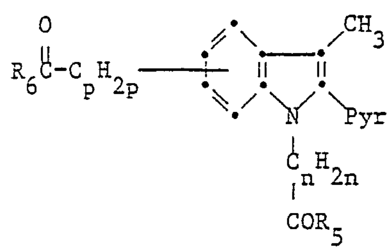

(III)

dans laquelle n est un nombre entier de 3 à 10, p est un nombre entier de 0 à 4, Pyr représente un groupe 2-, 3- ou 4-pyridyle, $R_5$ et $R_6$ représentent, indépendamment l'un de l'autre, des groupes hydroxy ou alcoxy inférieurs ; et leurs sels, les groupes qualifiés d'« inférieurs » contenant au maximum 7 atomes de carbone.

5. Composés de formule IV

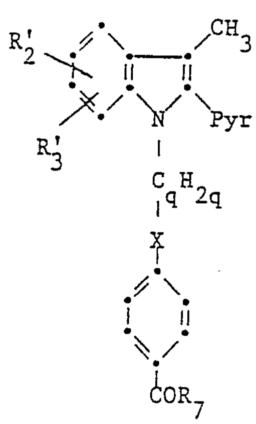

(IV)

dans laquelle $R'_2$ et $R'_3$ représentent, indépendamment l'un de l'autre, l'hydrogène, des groupes alkyle inférieurs, des halogènes, des groupes alcoxy inférieurs, alkylthio ou hydroxy, ou bien $R'_2$ et $R'_3$ forment ensemble, sur des atomes de carbone voisins, un groupe méthylènedioxy ; X représente l'oxygène, le soufre ou une liaison directe, q est un nombre entier de 1 à 4, $R_7$ représente un groupe hydroxy ou alcoxy inférieur ; Pyr représente un groupe 2-, 3- ou 4-pyridyle, et leurs sels, les groupes qualifiés d'« inférieurs » contenant au maximum 7 atomes de carbone.

6. Le 1-(7-carboxyheptyl)-3-méthyl-2-(3-pyridyl)-indole et ses sels.

7. Le 1-(5-carboxypentyl)-3-méthyl-2-(3-pyridyl)-indole et ses sels.

8. Le 1-(4-carboxybutyl)-3-méthyl-2-(3-pyridyl)-indole et ses sels.

9. Le 1-(5-carboxypentyl)-5-chloro-3-méthyl-2-(3-pyridyl)-indole et ses sels.

10. Compositions pharmaceutiques contenant des composés des revendications 1, 3 et 5 avec un véhicule pharmaceutique.

11. Compositions pharmaceutiques contenant des composés des revendications 2, 4 et 6 à 9 avec un véhicule pharmaceutique.

12. Les composés mentionnés dans les revendications 1, 3 et 5, pour l'utilisation dans un procédé pour le traitement thérapeutique de l'organisme humain ou animal.

13. Les composés mentionnés dans les revendications 2, 4 et 6 à 9, pour l'utilisation dans un procédé pour le traitement de l'organisme humain ou animal.

14. Les composés mentionnés dans les revendications 1, 3 et 5 en tant qu'inhibiteurs de la thromboxanne-synthétase.

15. Les composés mentionnés dans les revendications 2, 4 et 6 à 9, en tant qu'inhibiteurs de la thromboxanne-synthétase.

16. L'utilisation des composés mentionnés dans les revendications 1, 3 et 5 dans la préparation de compositions pharmaceutiques.

17. L'utilisation des composés mentionnés dans les revendications 2, 4 et 6 à 9 dans la préparation de compositions pharmaceutiques.

18. Procédé de préparation des composés de formule I mentionnés dans la revendication 1, de leurs N-oxydes et des sels de ces composés, caractérisé en ce que

1) on condense un composé de formule V

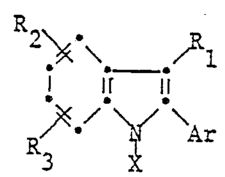

(V)

dans laquelle X représente l'hydrogène, un métal alcalin ou un groupe tri-(alkyle inférieur)-silyle, et $R_1$, $R_2$, $R_3$ et Ar ont les significations indiquées ci-dessus, avec un dérivé fonctionnel réactif d'un composé de formule VI

$$HO—CH_2—A—B \qquad (VI)$$

dans laquelle A et B ont les significations indiquées ci-dessus, ou bien

2) on cyclise un composé de formule VII

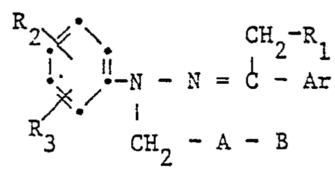

(VII)

dans laquelle Ar, $R_1$, $R_2$, $R_3$, A et B ont les significations indiquées ci-dessus, ou bien

3) on cyclise un composé de formule VIII

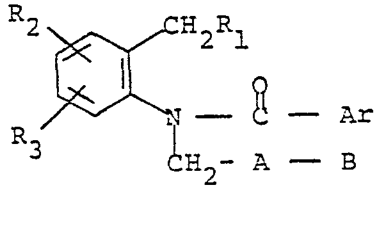

(VIII)

dans laquelle Ar, $R_1$, $R_2$, $R_3$, A et B ont les significations indiquées ci-dessus, ou bien

4) dans un composé de formule Ia

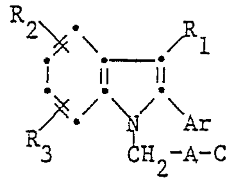

(Ia)

dans laquelle A, Ar, $R_1$, $R_2$ et $R_3$ ont les significations indiquées ci-dessus, et le groupe C est un groupe différent du groupe B mais convertible en ce dernier, on convertit le groupe C en le groupe B, éventuellement en allongeant la chaîne A dans le cadre de sa définition, ou bien

5) on soumet un composé de formule IX

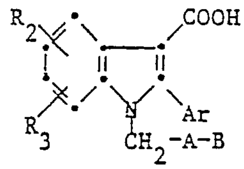

(IX)

dans laquelle A, B, Ar, $R_2$ et $R_3$ ont les significations indiquées ci-dessus, à décarboxylation et, si on le désire ou si c'est nécessaire, on protège transitoirement un groupe réactif gênant dans toutes ces opérations et, si on le désire, on convertit un composé obtenu répondant à la formule I en un autre composé de l'invention, et/ou, si on le désire, on convertit un composé obtenu à l'état libre en un sel ou un sel obtenu en le composé libre ou en un autre sel, et/ou, si on le désire, on sépare un mélange d'isomères ou de racémates obtenu en les isomères ou racémates individuels et/ou, si on le désire, on résout des racémates obtenus en les antipodes optiques.

19. Les composés susceptibles d'être obtenus par le procédé de la revendication 18.

**Revendications** (pour l'Etat contractant AT)

1. Procédé de préparation de 2-pyridylindoles substitués à l'azote et répondant à la formule générale I

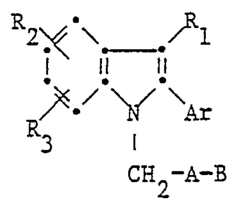

(I)

dans laquelle $R_1$ représente l'hydrogène ou un groupe alkyle inférieur, Ar représente un groupe pyridyle non substitué ou substitué par des groupes alkyle inférieurs, carboxy, (alcoxy inférieur)-carbonyle ou carbamoyle, $R_2$ et $R_3$ représentent, indépendamment l'un de l'autre, l'hydrogène, des groupes alkyle inférieurs, des halogènes, des groupes trifluorométhyle, hydroxy, alcoxy inférieur, carboxyalkyle inférieurs, (alcoxy inférieur)-carbonyl-alkyle inférieurs, carboxy, (alcoxy inférieur)-carbonyle ou (alkyle inférieur)-(thio, sulfinyle ou sulfonyle), ou bien $R_2$ et $R_3$ forment ensemble, sur des atomes de carbone voisins, un groupe alkylène-dioxy inférieur ; A représente un groupe alkylène en C1-C12, alcénylène en C2-C12, alcynylène en C2-C12, (alkylène inférieur)-phénylène-alkylène inférieur, (alkylène inférieur)-phénylène, phénylène-alkylène inférieur, phénylène, une liaison directe, un groupe (alkylène inférieur)-(thio ou oxy)-alkylène inférieur, (thio ou oxy)-phénylène, (alkylène inférieur)-(thio ou oxy)-phénylène, phénylène-(thio ou oxy)-alkylène inférieur ou phénylène-alcénylène inférieur, B représente un groupe carboxy, (alcoxy inférieur)-carbonyle, carbamoyle, mono- ou di-(alkyle inférieur)-carbamoyle, hydroxyméthyle, hydroxycarbamoyle, 5-tétrazolyle ou formyle ; de leurs N-oxydes ; et des sels de ces composés, les groupes qualifiés d'« inférieurs » contenant au maximum 7 atomes de carbone, caractérisé en ce que

1) on condense un composé de formule V

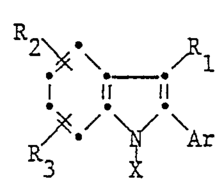

(V)

dans laquelle X représente l'hydrogène, un métal alcalin ou un groupe tri-(alkyle inférieur)-silyle, et $R_1$, $R_2$, $R_3$ et Ar ont les significations indiquées ci-dessus, avec un dérivé fonctionnel réactif d'un composé de formule VI

$$HO—CH_2—A—B \qquad (VI)$$

dans laquelle A et B ont les significations indiquées ci-dessus, ou bien

2) on cyclise un composé de formule VII

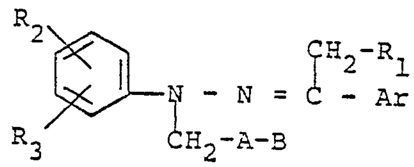

(VII)

dans laquelle Ar, $R_1$, $R_2$, $R_3$, A et B ont les significations indiquées ci-dessus, ou bien

3) on cyclise un composé de formule VIII

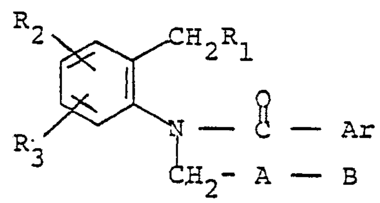

(VIII)

dans laquelle Ar, $R_1$, $R_2$, $R_3$, A et B ont les significations indiquées ci-dessus, ou bien

4) dans un composé de formule Ia

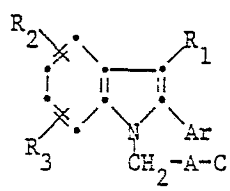

(Ia)

dans laquelle A, Ar, $R_1$, $R_2$ et $R_3$ ont les significations indiquées ci-dessus, et le groupe C est un groupe différent du groupe B mais convertible en ce dernier, on convertit le groupe C en le groupe B, éventuellement en allongeant la chaîne A dans le cadre de sa définition, ou bien

5) on soumet un composé de formule IX

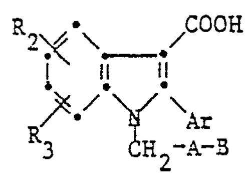

(IX)

dans laquelle A, B, Ar, $R_2$ et $R_3$ ont les significations indiquées ci-dessus, à décarboxylation et, si on le désire ou si c'est nécessaire, on protège transitoirement un groupe réactif gênant dans toutes ces opérations et, si on le désire, on convertit un composé obtenu répondant à la formule I en un autre composé de l'invention, et/ou, si on le désire, on convertit un composé obtenu à l'état libre en un sel ou un sel obtenu en le composé libre ou en un autre sel, et/ou, si on le désire, on sépare un mélange d'isomères ou de racémates obtenu en les isomères ou racémates individuels et/ou, si on le désire, on résout des racémates obtenus en les antipodes optiques.

2. Procédé selon la revendication 1, pour la préparation de composés de formule I de la revendication 1 dans laquelle $R_1$ représente l'hydrogène ou un groupe alkyle inférieur, Ar représente un groupe pyridyle non substitué ou substitué par des groupes alkyle inférieurs, carboxy, (alcoxy inférieur)-carbonyle ou carbamoyle, $R_2$ et $R_3$ représentent, indépendamment l'un de l'autre l'hydrogène, des groupes alkyle inférieurs, des halogènes, des groupes trifluorométhyle, hydroxy, alcoxy inférieurs, carboxyalkyle inférieurs, (alcoxy inférieur)-carbonylalkyle inférieurs, carboxy ou (alcoxy inférieur)-carbonyle, A représente un groupe alkylène en C1-C12, (alcénylène en C2-C12), alcynylène en C2-C12, (alkylène inférieur)-phénylène-alkylène inférieur, (alkylène inférieur)-phénylène, phénylène-alkylène inférieur, phénylène ou une liaison directe, B représente un groupe carboxy, (alcoxy inférieur)-carbonyle,

carbamoyle, mono- ou di-(alkyle inférieur)- carbamoyle ou hydroxyméthyle ; de leurs N-oxydes ; et des sels de ces composés, les groupes qualifiés d'« inférieurs » contenant au maximum 7 atomes de carbone, caractérisé en ce que l'on condense un composé de formule V de la revendication 1 dans laquelle X représente l'hydrogène, et les autres symboles ont les significations indiquées ci-dessus, avec un dérivé fonctionnel réactif d'un composé de formule VIa

$$HO—CH_2—A—B' \qquad (VIa)$$

dans laquelle A a les significations indiquées ci-dessus et B' représente un groupe carboxy, (alcoxy inférieur)-carbonyle, carbamoyle, mono- ou di-(alkyle inférieur)-carbamoyle, hydroxyméthyle, hydroxyméthyle éthérifié, halogénométhyle, trialcoxyméthyle ou cyano, et dans un composé obtenu répondant à la formule Ib

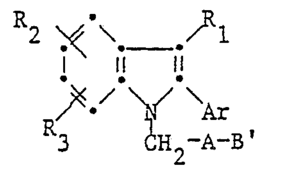

(Ib)

dans laquelle B' est différent de B, on convertit le reste B' en le groupe B, éventuellement en allongeant la chaîne A dans le cadre de sa définition et, si on le désire, on convertit un composé obtenu répondant à la formule I en un autre composé de l'invention et/ou, si on le désire, on convertit un composé obtenu à l'état libre en un sel ou un sel obtenu en le composé libre ou en un autre sel et/ou, si on le désire, on sépare un mélange d'isomères ou de racémates obtenu en les isomères ou racémates individuels et/ou, si on le désire, on résout les racémates obtenus en les antipodes optiques.

3. Procédé selon la revendication 1, caractérisé en ce que l'on prépare des composés de formule II

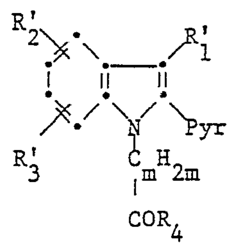

(II)

dans laquelle $R'_1$ représente l'hydrogène ou un groupe alkyle inférieur, $R'_2$ et $R'_3$ représentent, indépendamment l'un de l'autre, l'hydrogène, des groupes alkyle inférieurs, des halogènes, des groupes trifluorométhyle, hydroxy, alkylthio inférieurs ou alcoxy inférieurs, ou bien $R'_2$ et $R'_3$ forment ensemble, sur des atomes de carbone voisins, un groupe méthylène-dioxy, Pyr représente un groupe 2-, 3- ou 4-pyridyle, m est un nombre entier de 1 à 13, $R_4$ représente un groupe hydroxy, alcoxy inférieur ou amino ; et leurs sels, les groupes qualifiés d'« inférieurs » contenant au maximum 7 atomes de carbone.

4. Procédé selon la revendication 2, caractérisé en ce que l'on prépare des composés de formule III

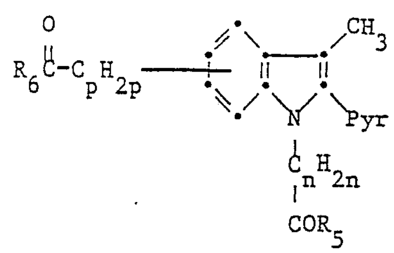

(III)

dans laquelle n est un nombre entier de 3 à 10, p un nombre entier de 0 à 4, Pyr représente un groupe 2-, 3- ou 4-pyridyle, $R_5$ et $R_6$ représentent, indépendamment l'un de l'autre, des groupes hydroxy ou alcoxy inférieurs ; et leurs sels, les groupes qualifiés d'« inférieurs » contenant au maximum 7 atomes de carbone.

5. Procédé selon la revendication 1 caractérisé en ce que l'on prépare des composés de formule IV

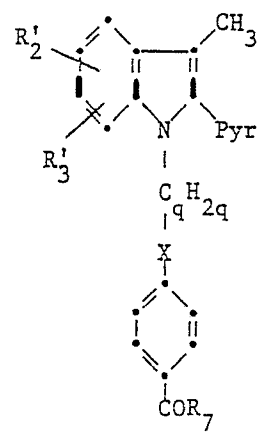

(IV)

dans laquelle $R'_2$ et $R'_3$ représentent indépendamment l'un de l'autre, l'hydrogène, des groupes alkyle inférieurs, des halogènes, des groupes alcoxy inférieurs, alkylthio inférieurs ou hydroxy, ou bien $R'_2$ et $R'_3$ forment ensemble sur des atomes de carbone voisins, un groupe méthylènedioxy ; X représente l'oxygène, le soufre ou une liaison directe, q est un nombre entier de 1 à 4, $R_7$ représente un groupe hydroxy ou alcoxy inférieur ; Pyr représente un groupe 2-, 3- ou 4-pyridyle, et leurs sels, les groupes qualifiés d'« inférieurs » contenant au maximum 7 atomes de carbone.

6. Procédé selon la revendication 1 caractérisé en ce que l'on prépare le 1-(7-carboxyheptyl)-3-méthyl-2-(3-pyridyl)-indole et ses sels.

7. Procédé selon la revendication 2, caractérisé en ce que l'on prépare le 1-(7-carboxyheptyl)-3-méthyl-2-(3-pyridyl)-indole et ses sels.

8. Procédé selon la revendication 1, caractérisé en ce que l'on prépare le 1-(5-carboxypentyl)-3-méthyl-2-(3-pyridyl)-indole et ses sels.

9. Procédé selon la revendication 2, caractérisé en ce que l'on prépare le 1-(5-carboxypentyl)-3-méthyl-2-(3-pyridyl)-indole et ses sels.

10. Procédé selon la revendication 1, caractérisé en ce que l'on prépare le 1-(4-carboxybutyl)-3-méthyl-2-(3-pyridyl)-indole et ses sels.

11. Procédé selon la revendication 2, caractérisé en ce que l'on prépare le 1-(4-carboxybutyl)-3-méthyl-2-(3-pyridyl)-indole et ses sels.

12. Procédé selon la revendication 1, caractérisé en ce que l'on prépare le 1-(5-carboxypentyl)-5-chloro-3-méthyl-2-(3-pyridyl)-indole et ses sels.

13. Procédé selon la revendication 2, caractérisé en ce que l'on prépare le 1-(5-carboxypentyl)-5-chloro-3-méthyl-2-(3-pyridyl)-indole et ses sels.

14. Procédé de préparation d'une composition pharmaceutique, caractérisé en ce que l'on combine une substance active selon l'invention de l'une des revendications 1, 3, 5, 6, 8, 10 et 12 avec un véhicule pharmaceutique.

15. Procédé de préparation d'une composition pharmaceutique, caractérisé en ce que l'on combine une substance active selon l'invention de l'une des revendications 2, 4, 7, 9, 11 et 13 avec un véhicule pharmaceutique.